# EUROPEAN PATENT APPLICATION

(11) **EP 4 818 175 A1**
(43) Date of publication of application: **07.10.2026**
(21) Application number: 25382332.2
(22) Date of filing: 02.04.2025
(51) Int. Cl.: C07D 498/14, A61P 31/12, A61K 31/5383

(54) **NOVEL ANTIVIRAL COMPOUNDS**

(71) Applicant: Consejo Superior de Investigaciones Científicas (CSIC), 28006 Madrid (ES); Universidad Autónoma De Madrid (UAM), 28049 Madrid (ES)
(72) Inventor: MENÉNDEZ ARIAS, Luis, 28049 Madrid (ES); ANDREU SATUÉ, Sabina, 28049 Madrid (ES); LÓPEZ GUERRERO, José Antonio, 28049 Madrid (ES); ZHAN, Peng, Jinan, 250012 (CN); LIU, Xinyong, Jinan, 250012 (CN); TANG, Kai, Jinan, 250012 (CN)
(74) Representative: Balder IP Law, S.L.

(57) **Abstract**

The present invention relates to new tricyclic organic compounds of formula (I): 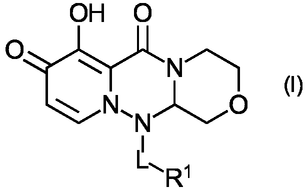 having antiviral activity, stereoisomers and pharmaceutically acceptable salts thereof, their use as a medicament, especially an antiviral medicament, and a pharmaceutical composition comprising the same.

## Description

### TECHNICAL FIELD

The present invention relates to new tricyclic organic compounds, having antiviral activity, stereoisomers and pharmaceutically acceptable salts thereof, their use as a medicament, especially an antiviral medicament, and a pharmaceutical composition comprising the same.

### STATE OF THE ART

Herpes simplex virus (HSV), known as herpes, is a common infection that can cause painful blisters or ulcers. There are two types of herpes simplex virus, type 1 (HSV-1) and type 2 (HSV-2). Both HSV-1 and HSV-2 can cause oral herpes or genital herpes. HSV-1 mostly spreads by oral contact and causes infections in or around the mouth (oral herpes or cold sores). HSV-2 is the primary cause of genital herpes. In addition, both types of viruses can cause keratitis, encephalitis, and severe neonatal disease. According to WHO's latest estimates obtained in 2016, 67% of the world population under the age of 50 have been infected with HSV-1 [James et al., 2020, Bull World Health Organ, 98(5):315-329], while 187 million people aged 15-49 years old had at least one episode of HSV-related painful, recurrent genital ulcer disease [Looker et al., 2020, BMJ Glob Health, 5(3):e001875].

Acyclovir, valacyclovir (an esterified version of acyclovir) and famciclovir (a prodrug of penciclovir) are commonly prescribed to combat HSV-1, although penciclovir, ganciclovir, cidofovir and foscarnet are also used for the treatment of skin lesions caused by HSV-1 and HSV-2. However, the limited efficacy of these antiviral medications is a major concern, since they only shorten the recovery period of infection by one or two days. In addition, the high prevalence of resistance to antiviral drugs, particularly acyclovir, has also been documented in immunocompromised individuals [Poole and James, 2018, Clin Ther., 40(8):1282-1298].

Novel anti-herpetic compounds acting on different targets have been developed to try to overcome resistance while providing effective therapies. Among them, pritelivir and amenamevir are approved inhibitors of the helicase-primase complex that inhibit the replication of both HSV-1 and HSV-2, including acyclovir-resistant viruses [Piret and Boivin, 2021, Enzymes, 50:79-132]. These drugs act by blocking the unwinding, priming and replication of the DNA template. In addition, n-docosanol (also known as behenyl alcohol) is an approved antiviral agent that interacts with cell surface phospholipids, inhibiting the fusion of the HSV envelope with the plasma membrane [Pope et al., 1998, Antiviral Res., 40(1-2):85-94]. Additional efforts in the development of antiviral agents against herpesviruses culminated with the approval of drugs such as maribavir and letermovir, specific for the treatment of human cytomegalovirus (HCMV). Maribavir is an inhibitor of the pUL97 serine/threonine protein kinase, an enzyme involved in viral encapsidation and nuclear egress of viral particles from infected cells. Letermovir targets the pUL56 subunit in the HCMV terminase complex pUL51/pUL56/pUL89.

The terminase complex is required to package the viral double-stranded DNA (dsDNA) genome into a preformed protein capsid. It contains an endonuclease activity (located in HCMV pUL89) that cleaves the viral genome units generated in tandem during the DNA replication process. In HSV-1, the terminase complex is composed of pUL15 (an ATPase/endonuclease) and two regulator/fixer proteins, pUL28 and pUL33 [Yang et al., 2020, Protein Cell., 11(5), 339-351].

α-Hydroxytropolones are natural products with broad biological activity due to their inhibitory activity against dinuclear metalloenzymes. Among them, β-thujaplicinol was found to inhibit HIV and hepatitis B virus RNases H, and human RNase H1 in enzymatic assays [Ponzar et al., 2022, J Biol Chem., 298(4):101790]*.* In cell culture, β-thujaplicinol and other α-hydroxytropolones show potent antiviral activity against HSV-1, HSV-2 and acyclovir-resistant mutants [Gazquez Casals et al., 2023, RSC Adv., 13(13):8743-8752]. In addition, raltegravir, dolutegravir and other HIV-1 integrase inhibitors are also effective against pUL15C and show anti-HSV activity in antiviral assays [Pennington et al., 2018, J Virol., 92(20):e00994-18].

Equivalent nucleases such as pUL89-C in HCMV or ORF29 in human herpesvirus 8 (HHV8; also known as Kaposi's sarcoma-associated herpesvirus) are also susceptible to approved HIV-1 integrase inhibitors [Miller et al., 2018, Antimicrob Agents Chemother., 62(10):e00233-18]*.* In recent years, numerous studies have explored pUL89-C inhibition by using different metal-chelating chemotypes and approaches previously followed in the discovery of HIV-1 RNase H and integrase inhibitors (for a review, see [Jagtap et al., 2023, J Med Chem., 66(20):13874-13887]).

Among such inhibitors, hydroxypyridinones have shown an outstanding capacity to coordinate metal ions. For example, a 2-carboxylic acid-modified 3-hydroxy-4-pyridone pharmacophore is present in baloxavir marboxil [WO2016/175224; O'Hanlon and Shaw, 2019, Curr Opin Virol., 35:14-18], an approved antiviral medication for treatment of influenza.

In view of the existing prevalence of viral infections, especially HSV infections, and the drawbacks and limitations of existing antiviral compounds, especially resistance, there is the need to develop further antiviral compounds with improved efficacy.

### SUMMARY OF THE INVENTION

A new family tricyclic organic compounds has been developed, which has shown to exhibit good antiviral efficacy, in particular against HSV-1.

Accordingly, a first aspect of the present invention relates to a compound of formula (I): 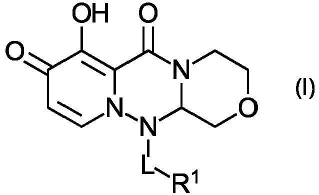 wherein
L is a linker, which is selected from -CH₂- and -(C=O)-;
   R¹ is selected from hydrogen, linear or branched C₁-C₆ alkyl, naphthyl, or 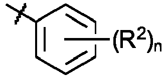 wherein
n is an integer selected from 0, 1 or 2,
if n is 2, the R² groups may be the same or different one from another,
   R² is selected from halogen, C₁-C₃ haloalkyl, C₂-C₁₀ heteroaryl, benzodioxol or 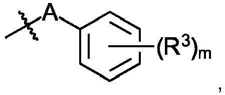 wherein
      A is a bond or is selected from -CH₂-, -(C=O) -, O or S,
      m is an integer selected from 0, 1 or 2,
      if m is 2, the R³ groups may be the same or different one from another, R³ is selected from halogen, -CN, -NH₂, -CHO, -COOH, -SO₂CH₃, -SO₂NH₂, - CONHR⁴ or -OR⁵,
   wherein
      R⁴ is hydrogen, C₁-C₆ alkyl or C₅-C₆ cycloalkyl,
      R⁵ is hydrogen, C₁-C₆ alkyl, C₁-C₃ haloalkyl,
   and stereoisomers thereof and pharmaceutically acceptable salts thereof.

According to a further aspect, the present invention relates to a compound of formula (I) as defined above, for use as a medicament.

According to another aspect, the present invention relates to a compound of formula (I) as defined above, for use as a medicament for the treatment of a viral infection.

Another aspect of the present invention refers to a pharmaceutical composition comprising a compound of formula (I) as defined above, and at least one pharmaceutically acceptable excipient.

### BRIEF DESCRIPTION OF THE FIGURES

To complete the description and in order to provide for a better understanding of the invention, a set of drawings is provided. Said drawings form an integral part of the description and illustrate embodiments of the invention, which should not be interpreted as restricting the scope of the invention, but just as examples of how the invention can be carried out. The drawings comprise the following figures:
Figure 1 is a graph showing the antiviral activity of Compound 19 according to the invention against the Lovelace strain of HSV-2. Tissue culture infective doses per mL (TCID₅₀/mL) were determined using an end-point dilution assay (TCID₅₀/mL, n=3).
Figure 2 is a dose-normalized isobologram for the non-constant ratio combinations. Data points on the hypotenuse indicate an additive effect, those on the lower left indicate synergism, and those on the upper right indicate antagonism. D, dose; Dx, dose of a single drug that exhibits a given percent inhibition.
Figure 3 is a graph corresponding to plasma concentration-time profiles of **Compound 19** in rats following intravenous and oral administration (at 2 and 10 mg·Kg⁻¹, respectively).
Figure 4 is a graph showing HSV-1 titers in lung, brain, olfactory bulb, kidney and spleen of BALB/c mice.

### DESCRIPTION OF THE INVENTION

Within the framework of the present invention, all the terms used herein including technical or scientific terms have the same meaning as commonly understood by one skilled in the art, to which the present invention pertains, unless defined otherwise. Such terms as those defined in a generally used dictionary are to be interpreted to have the meanings equal to the contextual meanings in the relevant art and are not to be interpreted to have ideal or excessively formal meanings, unless clearly defined in the present application.

In the present invention, "C_{x-y}" means having carbon atoms in a range of x to y.

In the present invention, "alkyl" means a linear (or straight-chain) saturated hydrocarbon group, and includes methyl, ethyl, propyl, butyl, pentyl, etc.

In the present invention, "haloalkyl" means a functional group in which at least one hydrogen of the alkyl defined above is substituted with a halogen atom of F, Cl, Br or I, e.g. one, two or three hydrogen of the alkyl are substituted with a halogen atom. When two or more hydrogen atoms are substituted, the halogen atoms may be the same or different, preferably are the same.

In the present invention, "cycloalkyl" means a saturated hydrocarbon ring having a specified number of carbon atoms, and the saturated hydrocarbon ring collectively refers to monocyclic and polycyclic structures, and a ring structure in which at least two rings share at least one carbon atom (for example, spiro ring, bridged ring, etc.). Cycloalkyl includes cyclohexyl, cycloheptanyl, cyclooctanyl, tetrahydronaphthalenyl, etc, but is not limited thereto.

In the present invention, "heteroaryl" means a monocyclic or polycyclic hetero ring in which at least one carbon atom of the aryl defined above is substituted with nitrogen (N), oxygen (O) or sulphur (S). Heteroaryl includes pyridinyl, thiophenyl, triazolyl, tetrazolyl, benzodioxolyl, benzothiazolyl, benzothiophenyl, quinolinyl, indolyl, isoindolyl, benzofuranyl, benzopyrrolyl, furanyl, pyrrolyl, thiazolyl, isothiazolyl, imidazolyl, pyrazolyl, oxazolyl, isoxazolyl, pyrazinyl, pyridazinyl, pyrimidinyl, isoquinolinyl, carbazolyl, benzoxazolyl, benzodioxazolyl, benzodioxinyl, benzimidazolyl, dihydrobenzothiophenyl, dihydrobenzofuranyl, purinyl, indolizinyl, chromanyl, chromenyl, dihydrobenzodioxinyl, etc., but is not limited thereto.

In the present invention, "stereoisomers" include a diastereomer and an optical isomer, in which the optical isomer includes not only an enantiomer, but also a mixture of the enantiomer and even a racemate.

In the present invention, "pharmaceutically acceptable salts" mean the salts conventionally used in a pharmaceutical industry. For example, there are inorganic ion salts prepared from calcium, potassium, sodium, magnesium, etc.; inorganic acid salts prepared from hydrochloric acid, nitric acid, phosphoric acid, bromic acid, iodic acid, perchloric acid, sulphuric acid, hydroiodic acid, etc.; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid, etc.; sulphonic acid salts prepared from methanesulphonic acid, ethanesulphonic acid, benzenesulphonic acid, p-toluenesulphonic acid, naphthalenesulphonic acid, etc.; amino acid salts prepared from glycine, arginine, lysine, etc.; amine salts prepared from trimethylamine, triethylamine, ammonia, pyridine, picoline, etc.; and the like, but types of salts meant in the present invention are not limited to those listed salts. preferable salts may include hydrochloride, phosphate, sulphate, trifluoroacetate, citrate, bromate, maleate or tartrate.

In the present invention, the term "excipient" refers to pharmaceutically acceptable adjuvants, diluents, carriers, etc., which may be incorporated in a pharmaceutical composition comprising the compound(s) of the invention.

According to a first aspect, the present invention refers to a compound of formula (I): 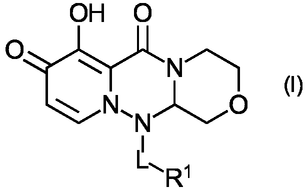 wherein
L is a linker, which is selected from -CH₂- and -(C=O)-;
   R¹ is selected from hydrogen, linear or branched C₁-C₆ alkyl, naphthyl, or 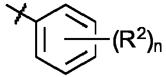 wherein
   n is an integer selected from 0, 1 or 2,
   if n is 2, the R² groups may be the same or different one from another,
      R² is selected from halogen, C₁-C₃ haloalkyl, C₂-C₁₀ heteroaryl, benzodioxol or 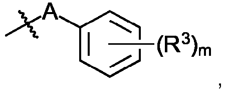 wherein
         A is a bond or is selected from -CH₂-, -(C=O) -, O or S,
         m is an integer selected from 0, 1 or 2,
         if m is 2, the R³ groups may be the same or different one from another,
      R³ is selected from halogen, -CN, -NH₂, -CHO, -COOH, -SO₂CH₃, -SO₂NH₂, - CONHR⁴ or -OR⁵,
         wherein
         R⁴ is hydrogen, C₁-C₆ alkyl or C₅-C₆ cycloalkyl,
         R⁵ is hydrogen, C₁-C₆ alkyl, C₁-C₃ haloalkyl,
and stereoisomers thereof and pharmaceutically acceptable salts thereof.

Pharmaceutically acceptable salts of the compounds according to the invention include, for example, salts with suitable anions, such as carboxylates, sulphonates, sulphates, chlorides, bromides, iodides, phosphates, tartrates, methane sulphonates, hydroxyethane sulphonates, glycinates, maleates, propionates, fumarates, toluene sulphonates, benzene sulphonates, trifluoroacetates, naphthalenedisulphonates-1,5, salicylates, benzoates, lactates, salts of malic acid, salts of 3-hydroxy-2- naphthoic acid-2, citrates and acetates.

Pharmaceutically acceptable salts of the compounds according to the invention further include, for example, salts with suitable pharmaceutically acceptable bases, such as, for example, salts with alkaline or alkaline-earth hydroxides, such as NaOH, KOH, Ca(OH)₂, Mg(OH)₂ etc. , amine compounds such as ethylamine, diethylamine, triethylamine, ethyldiisopropylamine, ethanolamine, diethanolamine, triethanolamine, methylglucamine, dicyclohexylamine, dimethylaminoethanol, procaine, dibenzylarrine, N- methylmorpholine, arginine, lysine, ethylenediamine, N-methylpiperidin, 2-amino-2-methyl-propanol-(1), 2-amino-2-methyl-propandiol-(1,3), 2-amino-2-hydroxyl-methyl-propandiol-(1,3) (TRIS) etc..

Depending on their structure, the compounds according to the invention may exist in stereoisomeric forms (enantiomers, diastereomers) in the presence of asymmetric carbon atoms. The invention therefore includes the use of the enantiomers or diastereomers and the respective mixtures thereof. The pure-enantiomer forms may optionally be obtained by conventional processes of optical resolution, such as by fractional crystallisation of diastereomers thereof by reaction with optically active compounds. Since the compounds according to the invention may occur in tautomeric forms, the present invention covers the use of all tautomeric forms. The compounds according to the present invention may be present as mixtures of various possible isomeric forms, in particular of stereoisomers such as, for example, E- and Z-, syn and anti, as well as optical isomers. The E-isomers and also the Z-isomers as well as the optical isomers and any mixtures of these isomers are claimed.

Further, the novel compounds of the present invention can be present in an amorphous, crystalline or partially crystalline form or they may also be present as hydrates.

In the following, particular embodiments of the compounds of formula (I) will be defined. It is the intention that within the frame of the present invention, any of the particular, individual embodiments, may be combined one with another, even though not expressly mentioned in combination.

In above formula (I), according to a first particular embodiment, linker L is -CH₂-.

According to a further embodiment, R¹ is selected from hydrogen, methyl, naphthyl, or

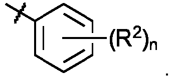

According to a particular embodiment, R¹ is

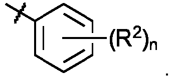

According to a particular embodiment, n is 1; in a further particular embodiment, when n is 1, R¹ is

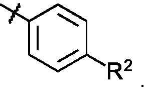

In a further particular embodiment, in the compound of formula (I), when R¹ is 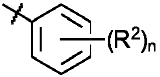 , R² is selected from fluorine, iodine, chlorine, trifluoromethyl, pyridine, pyrimidine, pyrazole, furane, indole, benzodioxol, or

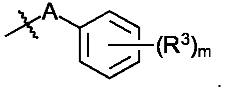

According to a further embodiment, m and n are independently 1 or 2, preferably n and m are both 1.

A further embodiment refers to a compound of formula (I) wherein R² is

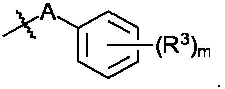

According to another embodiment, in formula (I) R¹ is 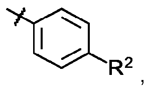 R² is 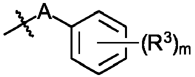 , and R³ is selected from chlorine, fluorine, -CN, -NH₂, -CHO, -COOH, -SO₂CH₃, -SO₂NH₂, - CONH₂ -CONHCH₃, -CONH(cyclopentyl), -OH or -OCH₃. As noted above, in particular embodiments of these compounds, m is 0 or 1. In another embodiment, A is a bond.

In another embodiment, in the compound of formula (I) R¹ is

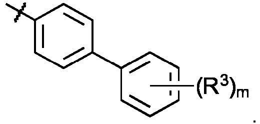

Again, in particular embodiments of these compounds, m is 0 or 1, and the meaning of R³ is particularly selected from chlorine, fluorine, -CN, -NH₂, -CHO, -COOH, -SO₂CH₃, -SO₂NH₂, - CONH₂ -CONHCH₃, -CONH(cyclopentyl), -OH or -OCH₃.

Further particular embodiments refer to a compound of formula (I). wherein L is -CH₂- and R¹ is 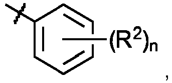 wherein R² is as defined above in general and particular embodiments.

That is, even further particular embodiments refer to a compound of formula (II): 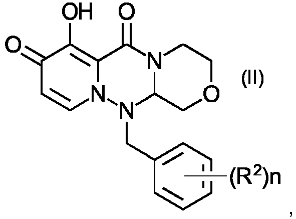 wherein R² is as defined above in general and particular embodiments of formula (I). In particular, n may be 0; if n is 1, R² is located in *para* position; if n is 2, at least one of R², which may be the same or different, is located in *para* position.

According to further embodiments, the compound of the invention is a compound of formula (III): 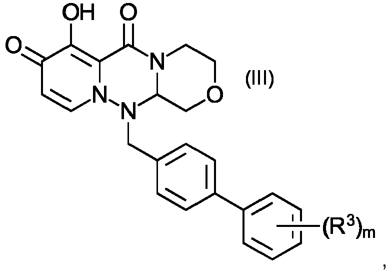 wherein R³ is as defined above in general and particular embodiments of formula (I). Preferably, m is 1, and even more preferably, R³ is located in *para* position,

In particular, the compound of formula (I) is selected from one of the following:

**Table 1**

| **Compound N°** | **Structure** | **Compound N°** | **Structure** |
|---|---|---|---|
| **1** | 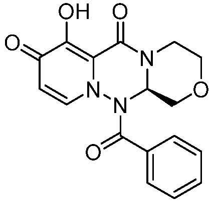 | **2** | 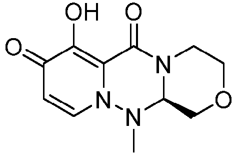 |
| **3** | 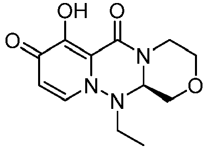 | **4** | 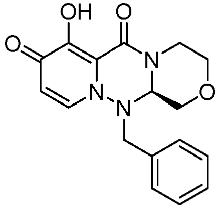 |
| **5** | 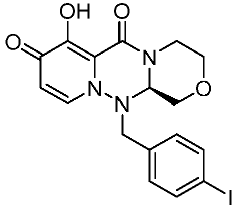 | **6** | 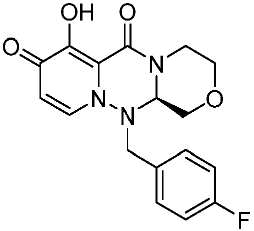 |
| **7** | 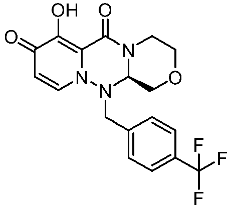 | **8** | 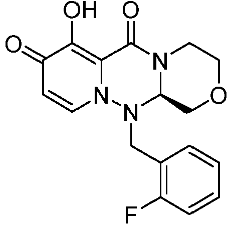 |
| **9** | 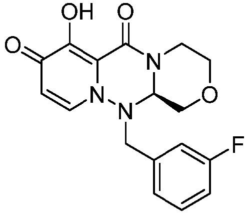 | **10** | 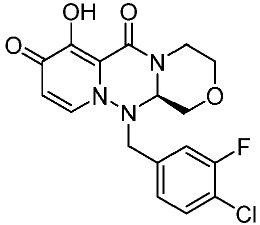 |
| **11** | 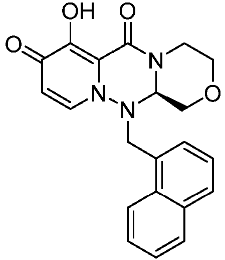 | **12** | 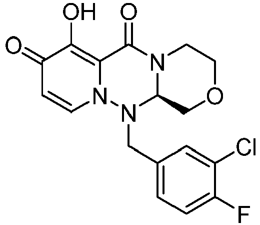 |
| **13** | 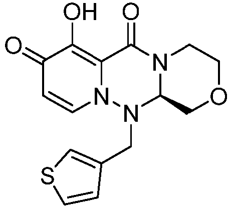 | **14** | 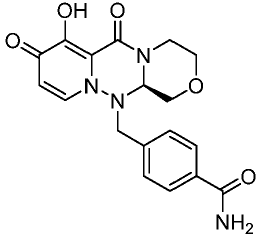 |
| **15** | 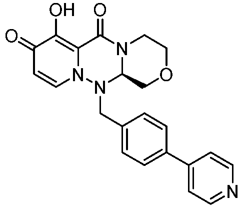 | **16** | 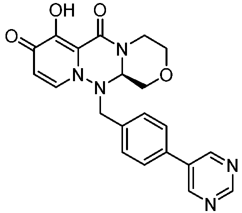 |
| **17** | 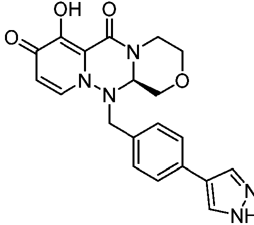 | **18** | 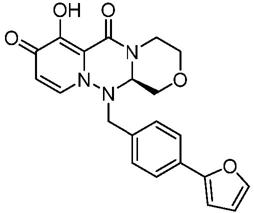 |
| **19** | 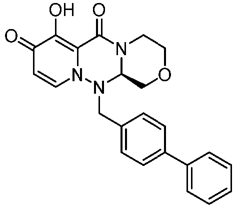 | **20** | 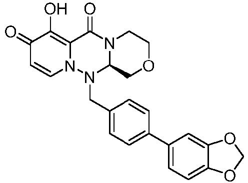 |
| **21** | 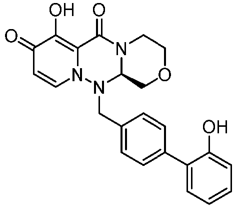 | **22** | 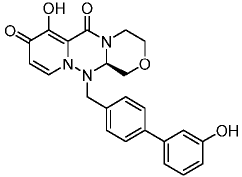 |
| **23** | 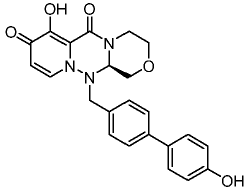 | **24** | 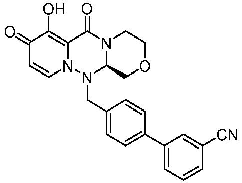 |
| **25** | 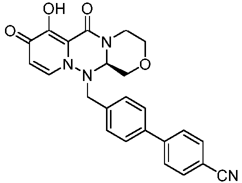 | **26** | 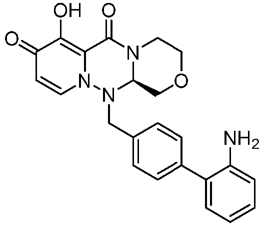 |
| **27** | 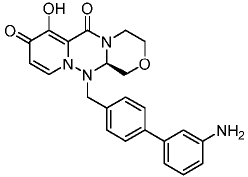 | **28** | 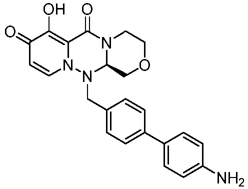 |
| **29** | 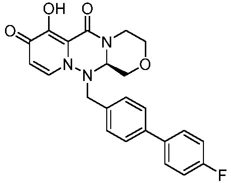 | **30** | 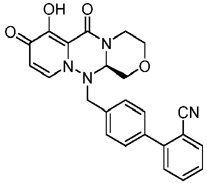 |
| **31** | 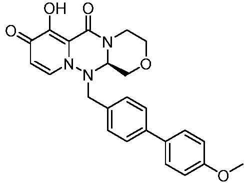 | **32** | 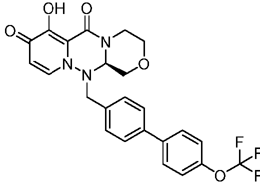 |
| **33** | 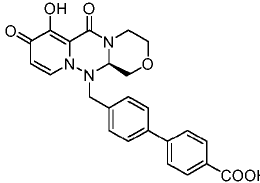 | **34** | 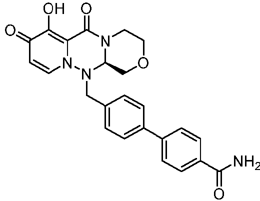 |
| **35** | 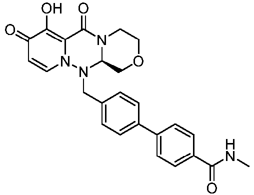 | **36** | 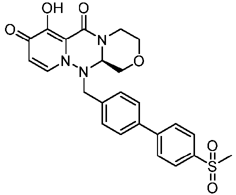 |
| **37** | 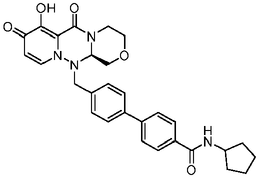 | **38** | 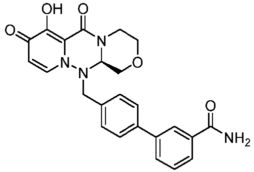 |
| **39** | 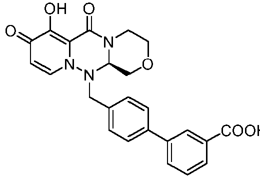 | **40** | 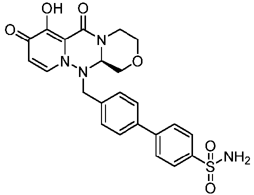 |
| **41** | 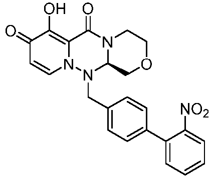 | **42** | 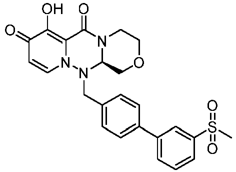 |
| **43** | 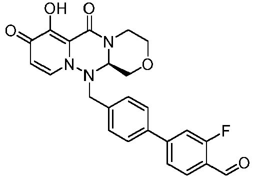 | **44** | 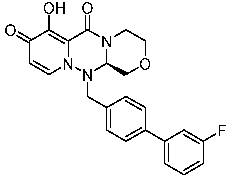 |
| **45** | 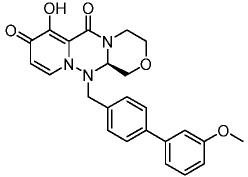 | **46** | 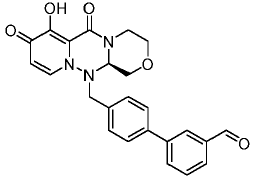 |
| **47** | 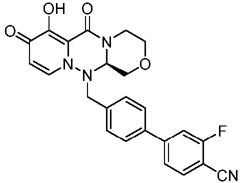 | **48** | 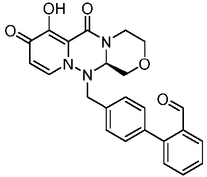 |
| **49** | 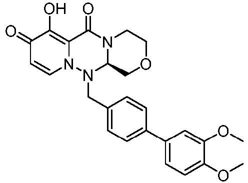 | **50** | 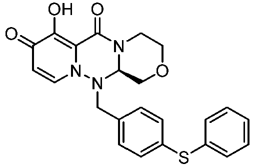 |
| **51** | 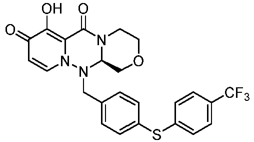 | **52** | 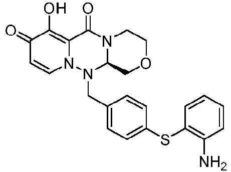 |
| **53** | 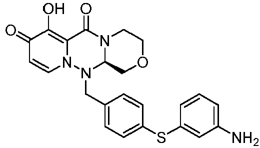 | **54** | 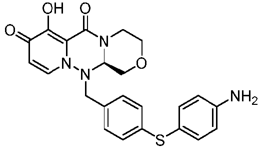 |
| **55** | 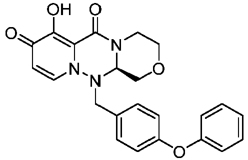 | **56** | 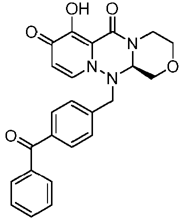 |
| **57** | 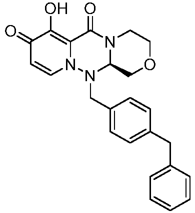 | **58** | 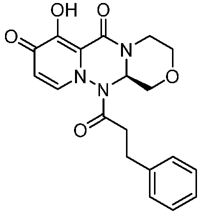 |
| **59** | 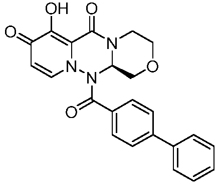 | **60** | 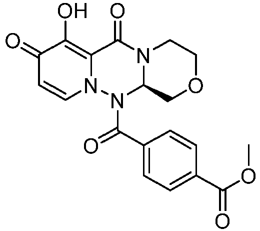 |
| **61** | 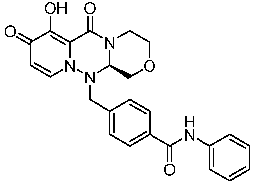 | **62** | 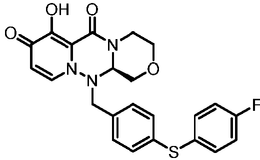 |
| **63** | 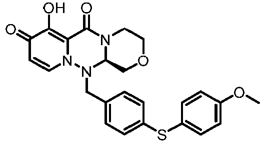 | **64** | 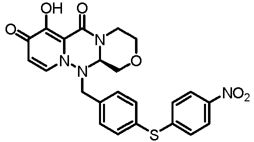 |
| **65** | 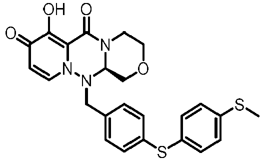 | **66** | 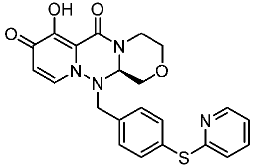 |
| **67** | 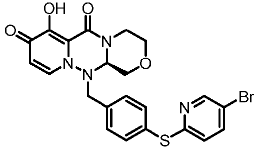 | **68** | 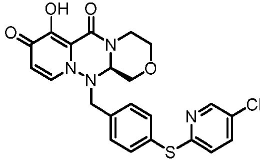 |

According to particular embodiments, the compound of formula (I) is one of:

**Table 2**

| **Compound N°** | **Structure** | **Compound N°** | **Structure** |
|---|---|---|---|
| **7** | 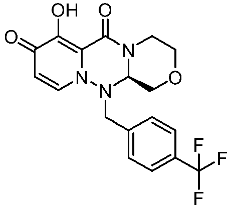 | **10** | 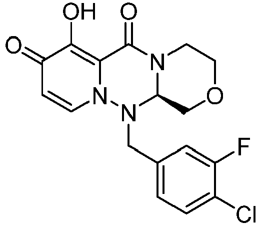 |
| **18** | 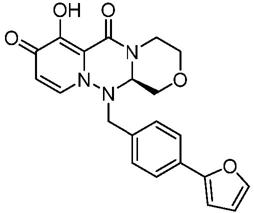 | **19** | 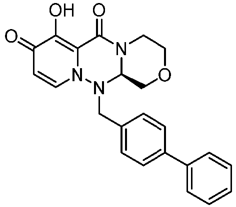 |
| **29** | 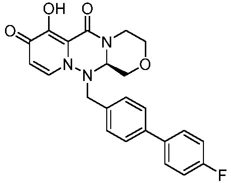 | **32** | 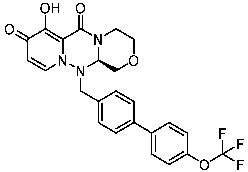 |
| **64** | 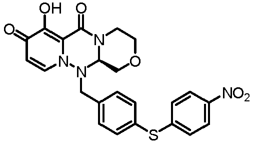 | / | / |

Even more preferably, the compound of formula (I) is:

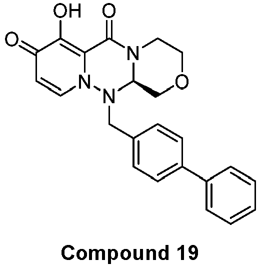

A further aspect of the invention is a compound of formula (I) as defined above, for use as a medicament.

According to a particular embodiment, the medicament is for the treatment or prophylaxis of a viral infection.

According to a further particular embodiment, the viral infection is caused by Herpes Simplex Virus, particularly to Herpes Simplex Virus 1 or 2, especially to Herpes Simplex Virus 1.

A further aspect of the invention refers to a pharmaceutical composition or medicament comprising one or more of the compounds of formula (I) as defined above, as well as optionally one or more pharmacologically acceptable carriers and/or auxiliary substances and/or solvents. A further object of the present invention relates to a pharmaceutical composition and medicament comprising one or more of the compounds according to the invention as defined above as well as optionally one or more further pharmaceutically effective compounds. The said pharmaceutical compositions contain, for example up to 99 weight-% or up to 90 weight-% or up to 80 weight-% or up to 70 weight-% of the compounds of the invention, the remainder being each formed by pharmacologically acceptable carriers and/or auxiliaries and/or solvents and/or optionally further pharmaceutically active compounds.

Therein, the pharmaceutically acceptable carriers, auxiliary substances or solvents are common pharmaceutical carriers, auxiliary substances or solvents, including various organic or inorganic carrier and/or auxiliary materials as they are customarily used for pharmaceutical purposes, in particular for solid medicament formulations. Examples include excipients, such as saccharose, starch, mannitol, sorbitol, lactose, glucose, cellulose, talcum, calcium phosphate, calcium carbonate; binding agents, such as cellulose, methylcellulose, hydroxypropylcellulose, polypropyl pyrrolidone, gelatine, gum arabic, polyethylene glycol, saccharose, starch; disintegrating agents, such as starch, hydrolysed starch, carboxymethylcellulose, calcium salt of carboxymethylcellulose, hydroxypropyl starch, sodium glycol starch, sodium bicarbonate, calcium phosphate, calcium citrate; lubricants, such as magnesium stearate, talcum, sodium laurylsulphate; flavouring agents, such as citric acid, menthol, glycin, orange powder; preserving agents, such as sodium benzoate, sodium bisulphite, paraben (for example methylparaben, ethylparaben, propylparaben, butylparaben); stabilizers, such as citric acid, sodium citrate, acetic acid and multicarboxylic acids from the titriplex series, such as, for example, diethylenetriaminepentaacetic acid (DTPA); suspending agents, such as methylcellulose, polyvinyl pyrrolidone, aluminium stearate; dispersing agents; diluting agents, such as water, organic solvents; waxes, fats and oils, such as beeswax, cocoa butter; polyethylene glycol; white petrolatum; etc.

Liquid medicament formulations, such as solutions, suspensions and gels usually contain liquid carrier, such as water and/or pharmaceutically acceptable organic solvents. Furthermore, such liquid formulations can also contain pH-adjusting agents, emulsifiers or dispersing agents, buffering agents, thickening agents, preserving agents, wetting agents, gelatinizing agents (for example methylcellulose), dyes and/or flavouring agents, for example as defined above.

The above-mentioned pharmaceutical compositions are suitable, for example, for intravenous, intraperitoneal, intramuscular, intravaginal, intraoral, percutaneous, subcutaneous, mucocutaneous, oral, rectal, transdermal, topical, intradermal, intragastric or intradermal application.

The pharmaceutical compositions are provided, for example, in the form of pills, tablets, entericcoated tablets, film tablets, layer tablets, sustained release formulations for oral, subcutaneous or cutaneous administration (in particular as a plaster), depot formulations, dragees, suppositories, gels, salves, syrup, granulates, suppositories, emulsions, dispersions, microcapsules, microformulations, nanoformulations, liposomal formulations, capsules, entericcoated capsules, powders, inhalation powders, microcrystalline formulations, inhalation sprays, drops, nose drops, nose sprays, aerosols, ampoules, solutions, juices, suspensions, infusion solutions or injection solutions etc.

A further aspect of the present invention relates to medicaments or combined preparations containing one or more of the compounds as defined above and at least one further pharmaceutically active compound, such as in particular a compound for the prophylaxis or treatment of a viral infection, especially a viral infection caused by Herpes Simplex Virus. According to a particular embodiment, the one further pharmaceutically active compound is acyclovir.

The compounds according to the invention of general formula (I) may basically be obtained by the processes described below, starting from (R)-7-(benzyloxy)-3,4,12,12a-tetrahydro-1*H-*[1,4]oxazino[3,4-c]pyrido[2,1-*f*][1,2,4]triazine-6,8-dione (**Intermediate 1a**); the process is further shown in detail in the Examples section. Accordingly, a further aspect of the invention is a process to synthesize the compounds of formula (I), by a process according to any one of the following reaction schemes 1 to 4:

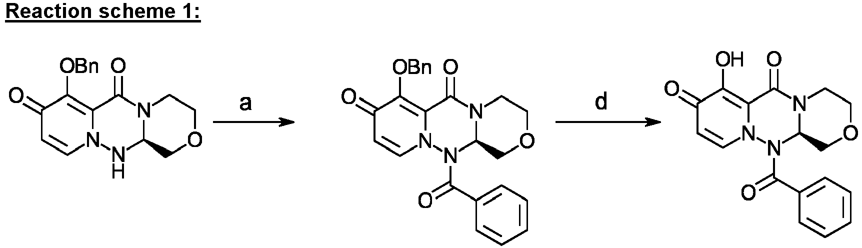

Reaction scheme 1 is applicable e.g. to the preparation of Compounds 1, 58 and 59.
a = benzoyl chloride, 3-phenylpropanoyl chloride
d = lithium chloride.

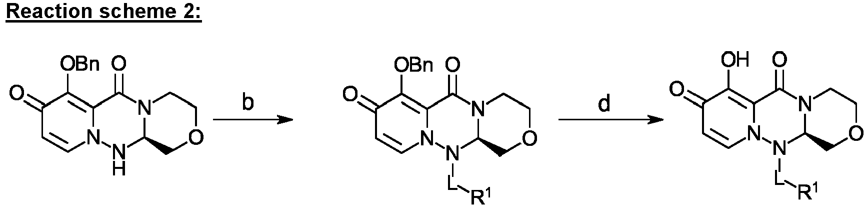

Reaction scheme 2 is applicable e.g. for the preparation of Compounds 2-14, 55-57 and 60-61.
b = different substituted benzyl bromide or benzyl chloride derivatives
d = lithium chloride

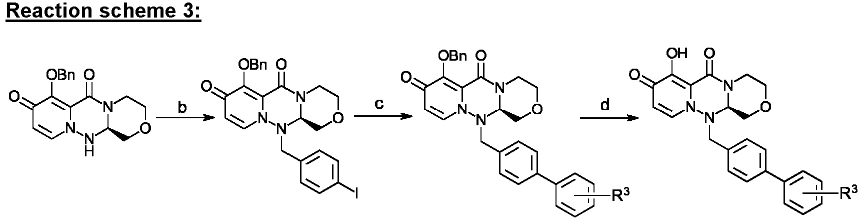

Reaction scheme 3 is applicable e.g. for the preparation of Compounds 15-49.
b = 4-iodobenzyl bromide
c = different substituted boric acid or boric acid pincinol esters
d = lithium chloride

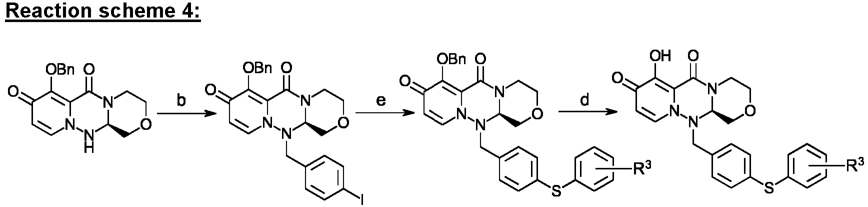

Reaction scheme 4 is applicable e.g. for the preparation of Compounds 50-54 and 62-68.
b = 4-iodobenzyl bromide
e = different substituted thiophenols
d = lithium chloride

The invention is obviously not limited to the specific embodiment(s) described herein, but also encompasses any variations that may be considered by any person skilled in the art, within the general scope of the invention as defined in the claims.

### EXAMPLES

Hereinafter, the present invention will be described in more detail through the following examples and experimental examples. However, the following examples and the like are provided only for the purpose of illustrating the present invention, and thus the scope of the present invention is not limited thereto.

### Synthesis of Compounds according to the invention and their characterization

All reagents and solvents employed were gained from standard suppliers and were directly used without further purification.

Liquid chromatography-mass spectrometry (LC-MS) and thin-layer chromatography (TLC) were used to monitor the reactions.

Purification of intermediates and final products was carried out by silica gel column chromatography (200-300 mesh).

The compounds' melting points were determined by WRS-1A micromelting apparatus and these data were uncorrected.

All the ¹H and ¹³C NMR spectra were recorded with Bruker AVANCE III 400 M spectrometer. Chemical shifts were reported in ppm relative to tetramethylsilane (TMS).

### General procedure for the synthesis of Compounds according to the invention

The Compounds according to the invention may be prepared following the general scheme shown hereinafter:

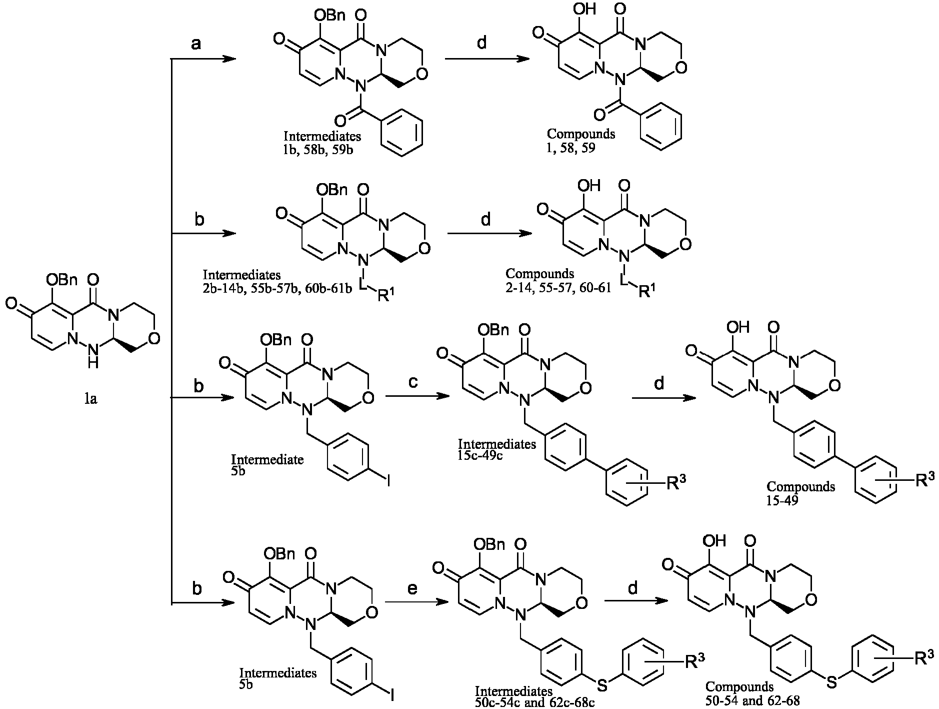

### EXAMPLE 1: Synthesis of Intermediate 1b.

(*R*)-7-(benzyloxy)-3,4,12,12a-tetrahydro-1*H*-[1,4]oxazino[3,4-c]pyrido[2,1-*f*][1,2,4]triazine-6,8-dione **(Intermediate 1a)** (0.5 g, 1.53 mmol, 1 eq) and triethylamine (TEA, 232 mg, 2.30 mmol, 1.5 eq) were added to dichloromethane (DCM, 25 mL), stirred for 10 minutes, then benzoyl chloride ( 325 mg, 1.84 mmol, 1.2 eq) was added dropwise in an ice bath, and the reaction was carried out at room temperature. After 3 hours, the TLC detection reaction was completed. The solvent was concentrated under reduced pressure, added to water, and extracted in ethyl acetate. The organic phase was combined and dried with anhydrous sodium sulphate. The solvent was concentrated after suction filtration. Finally, the concentrated residue was purified by flash column chromatography (DCM/MeOH = 20:1) to give the corresponding **Intermediate 1b.** Brown solid, yield 77.5%. ESI-MS: m/z 432.88 [M+1]⁺. C₂₄H₂₁N₃O₅ (431.15).

### EXAMPLE 2: Synthesis of Intermediate 2b.

**Intermediate 2b** was prepared by stirring a mixture of **Intermediate 1a** (0.5 g, 1.53 mmol, 1 eq.), iodomethane (1.836 mmol, 1.2 eq.), and Cs₂CO₃ (0.99 g, 3.06 mmol, 2 eq.) in DMF (25 mL) for 12 h at room temperature. The reaction was monitored by TLC until it was complete, then concentrated under reduced pressure. The residue was added to a saturated sodium chloride solution, extracted with ethyl acetate (3×50 mL), and combined with the organic phase, which was dried with anhydrous sodium sulphate, and then filtered and concentrated. Finally, the concentrated residue was purified by flash column chromatography (DCM/MeOH = 20:1) to obtain **Intermediate 2b:**

### (R)-7-(benzyloxy)-12-methyl-3,4,12,12a-tetrahydro-1H-[1,4]oxazino[3,4-c]pyrido[2,1-f][1,2,4]triazine-6,8-dione (Intermediate 2b).

ESI-MS: m/z 342.15 [M+1]⁺. C₁₈H₁₉IN₃O₄ (342.47).

### EXAMPLE 3: Synthesis of Intermediates 3b to 14b.

### (R)-7-(benzyloxy)-12-ethyl-3,4,12,12a-tetrahydro-1H-[1,4]oxazino[3,4-c]pyrido[2,1-f][1,2,4]triazine-6,8-dione (Intermediate 3b).

**Intermediate 3b** was synthesized following the procedure used for the preparation of **Intermediate 2b,** with ethane iodide substituted for iodomethane. ESI-MS: m/z 356.16 [M+1]⁺. C₁₉H₂₁IN₃O₄ (356.08).

### (R)-12-benzyl-7-(benzyloxy)-3,4,12,12a-tetrahydro-1H-[1,4]oxazino[3,4-c]pyrido[2,1-f][1,2,4]triazine-6,8-dione (Intermediate 4b).

**Intermediate 4b** was synthesized following the procedure used for the preparation of **Intermediate 2b,** with benzyl bromide substituted for iodomethane. ESI-MS: m/z 417.17 [M+1]⁺. C₂₄H₂₃N₃O₄ (417.47).

### (R)-7-(benzyloxy)-12-(4-iodobenzyl)-3,4,12,12a-tetrahydro-1H-[1,4]oxazino[3,4-c]pyrido[2,1-f][1,2,4]triazine-6,8-dione (Intermediate 5b).

**Intermediate 5b** was synthesized following the procedure used for the preparation of **Intermediate 2b,** with 4-iodobenzyl bromide substituted for iodomethane.

¹H NMR (400 MHz, DMSO-*d*₆) δ 7.67 (d, *J* = 8.2 Hz, 2H), 7.53 (d, *J* = 6.7 Hz, 2H), 7.40 - 7.28 (m, 3H), 7.06 (s, 1H), 700 (d, *J* = 8.2 Hz, 2H), 5.92 (d, *J* = 7.7 Hz, 1H), 5.14 (s, 2H), 4.99 (s, 1H), 4.35 - 4.14 (m, 2H), 3.99 - 3.76 (m, 3H), 3.60 (s, 2H), 3.19 (d, *J* = 10.2 Hz, 1H). ESI-MS: m/z 543.80 [M+1]⁺. C₂₄H₂₂IN₃O₄ (543.07).

### (R)-7-(benzyloxy)-12-(4-fluorobenzyl)-3,4,12,12a-tetrahydro-1H-[1,4]oxazino[3,4-c]pyrido[2,1-f][1,2,4]triazine-6,8-dione (Intermediate 6b).

**Intermediate 6b** was synthesized following the procedure used for the preparation of **Intermediate 2b,** with 4-fluorobenzyl bromide substituted for iodomethane. ESI-MS: m/z 435.16 [M+1]⁺. C₂₄H₂₂FN₃O₄ (435.10).

### (R)-7-(benzyloxy)-12-(4-(trifluoromethyl)benzyl)-3,4,12,12a-tetrahydro-1H-[1,4]oxazino[3,4-c]pyrido[2,1-f][1,2,4]triazine-6,8-dione (Intermediate 7b).

**Intermediate 7b** was synthesized following the procedure used for the preparation of **Intermediate 2b,** with 4-trifluoromethylbenzyl bromide substituted for iodomethane. ESI-MS: m/z 485.16 [M+1]⁺. C₂₅H₂₂FN₃O₄ (485.64).

### (R)-7-(benzyloxy)-12-(2-fluorobenzyl)-3,4,12,12a-tetrahydro-1H-[1,4]oxazino[3,4-c]pyrido[2,1-f][1,2,4]triazine-6,8-dione (Intermediate 8b).

**Intermediate 8b** was synthesized following the procedure used for the preparation of **Intermediate 2b,** with 2-fluorobenzyl bromide substituted for iodomethane. ESI-MS: m/z 435.16 [M+1]⁺. C₂₄H₂₂FN₃O₄ (435.87).

### (R)-7-(benzyloxy)-12-(3-fluorobenzyl)-3,4,12,12a-tetrahydro-1H-[1,4]oxazino[3,4-c]pyrido[2,1-f][1,2,4]triazine-6,8-dione (Intermediate 9b).

**Intermediate 9b** was synthesized following the procedure used for the preparation of **Intermediate 2b,** with 3-fluorobenzyl bromide substituted for iodomethane. ESI-MS: m/z 435.16 [M+1]⁺. C₂₄H₂₂FN₃O₄ (435.55).

### (R)-7-(benzyloxy)-12-(4-chloro-3-fluorobenzyl)-3,4,12,12a-tetrahydro-1H-[1,4]oxazino[3,4-c]pyrido[2,1-f][1,2,4]triazine-6,8-dione (Intermediate 10b).

**Intermediate 10b** was synthesized following the procedure used for the preparation of **Intermediate 2b,** with 4-chloro-3-fluorobenzyl bromide substituted for iodomethane. ESI-MS: m/z 469.12 [M+1]⁺. C₂₄H₂₁FClN₃O₄ (469.90).

### (R)-7-(benzyloxy)-12-(naphthalen-1-ylmethyl)-3,4,12,12a-tetrahydro-1H-[1,4]oxazino[3,4-c]pyrido[2,1-f][1,2,4]triazine-6,8-dione (Intermediate 11b).

**Intermediate 11b** was synthesized following the procedure used for the preparation of **Intermediate 2b,** with 1-chloromethyl naphthalene substituted for iodomethane. ESI-MS: m/z 467.18 [M+1]⁺. C₂₈H₂₅N₃O₄ (469.53).

### (R)-7-(benzyloxy)-12-(3-chloro-4-fluorobenzyl)-3,4,12,12a-tetrahydro-1H-[1,4]oxazino[3,4-c]pyrido[2,1-f][1,2,4]triazine-6,8-dione (Intermediate 12b).

**Intermediate 12b** was synthesized following the procedure used for the preparation of **Intermediate 2b,** with 2-chloro-4-(chloromethyl)-1-fluorobenzene substituted for iodomethane. ESI-MS: m/z 469.12 [M+1]⁺. C₂₈H₂₅N₃O₄ (469.70).

### (R)-7-(benzyloxy)-12-(thiophen-2-ylmethyl)-3,4,12,12a-tetrahydro-1H-[1,4]oxazino[3,4-c]pyrido[2,1-f][1,2,4]triazine-6,8-dione (Intermediate 13b)

**Intermediate 13b** was synthesized following the procedure used for the preparation of **Intermediate 2b,** with 3-(bromomethyl)thiophene substituted for iodomethane. ESI-MS: m/z 424.22 [M+1]+. C₂₂H₂₁N₃O₄S(423.48).

### (R)-4-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)benzamide (Intermediate 14b)

**Intermediate 14b** was synthesized following the procedure used for the preparation of **Intermediate 2b,** with 4-(chloromethyl)benzamide substituted for iodomethane. ESI-MS: m/z 461.17 [M+1]+_{.} C₂₅H₂₄N₄O₅ (460.49).

### EXAMPLE 4: Synthesis of Intermediate 15c.

A mixture of **Intermediate 5b** (0.2 g, 0.36 mmol, 1 eq.), pyridine-4-boronic acid (0.72 mmol, 2 eq.), CsCO₃ (0.18 g, 0.55 mmol, 1.5 eq.), and PdCl₂(dppf) DCM adduct (0.032 g, 0.039 mmol, 0.1 eq.) was weighed into the 50 ml bottle, and a mixed solution of 12 mL dioxane and 2 mL water (dioxane: water = 6:1) was added, and the reaction was heated at 95 °C under nitrogen protection for 11 h. After the reaction was completed, the mixture was filtered with diatomite, and the filtrate was transferred to the separating funnel, extracted with ethyl acetate (3×20 mL), combined with the organic phase, added anhydrous sodium sulphate to the organic phase for drying, filtration, concentration, finally the residue was purified by column chromatography (DCM: MeOH = 20:1) to obtain the **Intermediate 15c:**

### (R)-7-(benzyloxy)-12-(4-(pyridin-4-yl)benzyl)-3,4,12,12a-tetrahydro-1H-[1,4]oxazino[3,4-c]pyrido[2,1-f][1,2,4]triazine-6,8-dione (Intermediate 15c).

¹H NMR (400 MHz, DMSO-*d*₆) δ 8.64 (d, *J* = 5.1 Hz, 2H), 7.76 (dd, *J* = 23.6, 6.5 Hz, 4H), 7.54 (d, *J* = 7.4 Hz, 2H), 7.43 - 7.27 (m, 5H), 7.12 (s, 1H), 5.93 (d, *J* = 7.7 Hz, 1H), 5.15 (s, 2H), 5.04 (s, 1H), 4.28 (d, *J* = 83.0 Hz, 3H), 3.90 (d, *J* = 56.1 Hz, 4H), 3.30 - 3.14 (m, 1H). ESI-MS: m/z 495.57 [M+1]⁺. C₂₉H₂₆N₄O₄ (494.20).

### EXAMPLE 5: Synthesis of Intermediates 16c to 49c.

### (R)-7-(benzyloxy)-12-(4-(pyrimidin-5-yl)benzyl)-3,4,12,12a-tetrahydro-1H-[1,4]oxazino[3,4-c]pyrido[2,1-f][1,2,4]triazine-6,8-dione (Intermediate 16c).

**Intermediate 16c** was synthesized following the procedure used for the preparation of **Intermediate 15c,** with 5-pyrimidinylboronic acid substituted for pyridine-4-boronic acid. ESI-MS: m/z 496.20 [M+1]⁺. C₂₈H₂₆N₅O₄ (496.55).

### (R)-12-(4-(1H-pyrazol-4-yl)benzyl)-7-(benzyloxy)-3,4,12,12a-tetrahydro-1H-[1,4]oxazino[3,4-c]pyrido[2,1-f][1,2,4]triazine-6,8-dione (Intermediate 17c).

**Intermediate 17c** was synthesized following the procedure used for the preparation of **Intermediate 15c,** with 1H-pyrazole-4-boronic acid substituted for pyridine-4-boronic acid. ESI-MS: m/z 484.20 [M+1]⁺. C₂₇H₂₆N₅O₄ (484.54).

### (R)-7-(benzyloxy)-12-(4-(furan-2-yl)benzyl)-3,4,12,12a-tetrahydro-1H-[1,4]oxazino[3,4-c]pyrido[2,1-f][1,2,4]triazine-6,8-dione (Intermediate 18c).

**Intermediate 18c** was synthesized following the procedure used for the preparation of **Intermediate 15c,** with 2-furanboronic acid substituted for pyridine-4-boronic acid. ESI-MS: m/z 483.18 [M+1]⁺. C₂₈H₂₅N₃O₅ (483.26).

### (R)-12-([1,1'-biphenyl]-4-yimethyl)-7-(benzyloxy)-3,4,12,12a-tetrahydro-1H-[1,4]oxazino[3,4-c]pyrido[2,1-f][1,2,4]triazine-6,8-dione (Intermediate 19c).

**Intermediate 19c** was synthesized following the procedure used for the preparation of **Intermediate 15c,** with phenylboronic acid substituted for pyridine-4-boronic acid. ESI-MS: m/z 483.18 [M+1]⁺. C₃₀H₂₇N₃O₄ (483.26).

### (R)-12-(4-(benzo[d][1,3]dioxol-5-yl)benzyl)-7-(benzyloxy)-3,4,12,12a-tetrahydro-1H-[1,4]oxazino[3,4-c]pyrido[2,1-f][1,2,4]triazine-6,8-dione (Intermediate 20c).

**Intermediate 20c** was synthesized following the procedure used for the preparation of **Intermediate 15c,** with 2-(benzo[*d*][1,3]dioxol-5-yl)-4,4,5,5-tetramethyl-1,3,2-dioxaborolane substituted for pyridine-4-boronic acid. ESI-MS: m/z 538.20 [M+1]⁺. C₃₁H₂₈N₃O₆ (538.66).

### (R)-7-(benzyloxy)-12-((2'-hydroxy-[1,1-biphenyl]-4-yl)methyl)-3,4,12,12a-tetrahydro-1H-[1,4]oxazino[3,4-c]pyrido[2,1-f][1,2,4]triazine-6,8-dione (Intermediate 21c).

¹H NMR (400 MHz, DMSO-*d*₆) δ 9.55 (s, 1H), 7.52 (dd, *J* = 12.8, 7.7 Hz, 4H), 7.41 - 7.28 (m, 3H), 7.27 - 7.10 (m, 5H), 6.94 (d, *J* = 8.0 Hz, 1H), 6.87 (t, *J* = 7.4 Hz, 1H), 5.94 (d, *J* = 7.7 Hz, 1H), 5.15 (s, 2H), 500 (s, 1H), 4.50 - 3.73 (m, 7H), 3.28 - 3.18 (m, 1H). ESI-MS: m/z 510.45 [M+1]⁺. C₃₀H₂₇N₃O₅ (509.20)

### (R)-7-(benzyloxy)-12-((3'-hydroxy-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 22c).

**Intermediate 22c** was synthesized following the procedure used for the preparation of **Intermediate 15c,** with 3-hydroxyphenylboronic acid substituted for pyridine-4-boronic acid. ESI-MS: m/z 510.20 [M+1]⁺. C₃₀H₂₈N₃O₅ (510.57).

### (R)-7-(benzyloxy)-12-((4'-hydroxy-[1,1-biphenyl]-4-yl)methyl)-3,4,12,12a-tetrahydro-1H-[1,4]oxazino[3,4-c]pyrido[2,1-f][1,2,4]triazine-6,8-dione (Intermediate 23c).

**Intermediate 23c** was synthesized following the procedure used for the preparation of **Intermediate 15c,** with 4-hydroxyphenylboronic acid substituted for pyridine-4-boronic acid. ESI-MS: m/z 510.20 [M+1]⁺. C₃₀H₂₈N₃O₅ (510.83).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carbonitrile (Intermediate 24c).

**Intermediate 24c** was synthesized following the procedure used for the preparation of **Intermediate 15c,** with 3-cyanophenylboronic acid substituted for pyridine-4-boronic acid. ESI-MS: m/z 519.20 [M+1]⁺. C₃₁H₂₇N₄O₄ (518.96).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carbonitrile (Intermediate 25c).

**Intermediate 25c** was synthesized following the procedure used for the preparation of **Intermediate 15c,** with 4-cyanophenylboronic acid substituted for pyridine-4-boronic acid. ESI-MS: m/z 519.20 [M+1]⁺. C₃₁H₂₇N₄O₄ (519.77).

### (R)-12-((2'-amino-[1,1'-biphenyl]-4-yl)methyl)-7-(benzyloxy)-3,4,12,12a-tetrahydro-1H-[1,4]oxazino[3,4-c]pyrido[2,1-f][1,2,4]triazine-6,8-dione (Intermediate 26c).

**Intermediate 26c** was synthesized following the procedure used for the preparation of **Intermediate 15c,** with 2-aminophenylboronic acid substituted for pyridine-4-boronic acid. ESI-MS: m/z 508.21 [M+1]⁺. C₃₀H₂₈N₄O₄ (508.64).

### (R)-12-((3'-amino-[1,1'-biphenyl]-4-yl)methyl)-7-(benzyloxy)-3,4,12,12a-tetrahydro-1H-[1,4]oxazino[3,4-c]pyrido[2,1-f][1,2,4]triazine-6,8-dione (Intermediate 27c).

**Intermediate 27c** was synthesized following the procedure used for the preparation of **Intermediate 15c,** with 3-aminophenylboronic acid substituted for pyridine-4-boronic acid. ESI-MS: m/z 508.21 [M+1]⁺. C₃₀H₂₈N₄O₄ (510.02).

### (R)-12-((4'-amino-[1,1'-biphenyl]-4-yl)methyl)-7-(benzyloxy)-3,4,12,12a-tetrahydro-1H-[1,4]oxazino[3,4-c]pyrido[2,1-f][1,2,4]triazine-6,8-dione (Intermediate 28c).

**Intermediate 28c** was synthesized following the procedure used for the preparation of **Intermediate 15c,** with 4-aminophenylboronic acid substituted for pyridine-4-boronic acid. ESI-MS: m/z 508.21 [M+1]⁺. C₃₀H₂₈N₄O₄ (509.10).

### (R)-7-(benzyloxy)-12-((4-fluoro-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 29c).

**Intermediate 29c** was synthesized following the procedure used for the preparation of **Intermediate 15c,** with 4-fluorobenzeneboronic acid substituted for pyridine-4-boronic acid. ESI-MS: m/z 511.19 [M+1]⁺. C₃₀H₂₆FN₃O₄ (511.55).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-2-carbonitrile (Intermediate 30c).

**Intermediate 24c** is synthesized following the procedure used for the preparation of **Intermediate 13c,** with 2-cyanophenylboronic acid substituted for pyridine-4-boronic acid. ESI-MS: m/z 519.20 [M+1]⁺. C₃₁H₂₇N₄O₄ (519.58).

### (R)-7-(benzyloxy)-12-((4'-methoxy-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 31c).

**Intermediate 31c** was synthesized following the procedure used for the preparation of **Intermediate 15c,** with 4-methoxyphenylboronic acid substituted for pyridine-4-boronic acid. ESI-MS: m/z 524.22 [M+1]⁺. C₃₁H₃₀N₃O₅ (525.01).

### (R)-7-(benzyloxy)-12-((4'-(trifluoromethoxy)-[1,1-biphenyl]-4-yl)methyl)-3,4,12,12a-tetrahydro-1H-[1,4]oxazino[3,4-c]pyrido[2,1-f][1,2,4]triazine-6,8-dione (Intermediate 32c).

**Intermediate 32c** was synthesized following the procedure used for the preparation of **Intermediate 15c,** with 4-trifluoromethoxyphenylboronic acid substituted for pyridine-4-boronic acid. ESI-MS: m/z 578.19 [M+1]⁺. C₃₁H₂₇F₃N₃O₅ (578.96).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carboxylic acid (Intermediate 33c).

**Intermediate 33c** was synthesized following the procedure used for the preparation of **Intermediate 15c,** with 4-carboxyphenylboronic acid substituted for pyridine-4-boronic acid. ESI-MS: m/z 538.20 [M+1]⁺. C₃₁H₂₈N₃O₆ (538.84).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carboxamide (Intermediate 34c).

Intermediate **34c** was synthesized following the procedure used for the preparation of **Intermediate 15c,** with (4-carbamoylphenyl)boronic acid substituted for pyridine-4-boronic acid. ESI-MS: m/z 537.21 [M+1]⁺. C₃₂H₂₉N₄O₅ (537.60).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-N-methyl-[1,1'-biphenyl]-4-carboxamide (Intermediate 35c).

**Intermediate 35c** was synthesized following the procedure used for the preparation of **Intermediate 15c,** with (4-(methylcarbamoyl)phenyl)boronic acid substituted for pyridine-4-boronic acid. ESI-MS: m/z 551.23 [M+1]⁺. C₃₂H₃₁N₄O₅ (551.62).

### (R)-7-(benzyloxy)-12-((4'-(methylsulphonyl)-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 36c).

**Intermediate 36c** was synthesized following the procedure used for the preparation of **Intermediate 15c,** with (4-(methylcarbamoyl)phenyl)boronic acid substituted for pyridine-4-boronic acid. ESI-MS: m/z 551.23 [M+1]⁺. C₃₂H₃₁N₄O₅ (551.62).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-N-cyclopentyl-[1,1'-biphenyl]-4-carboxamide (Intermediate 37c).

**Intermediate 37c** was synthesized following the procedure used for the preparation of **Intermediate 15c,** with (4-(cyclopentylcarbamoyl)phenyl)boronic acid substituted for pyridine-4-boronic acid. ESI-MS: m/z 605.28 [M+1]⁺. C₃₆H₃₇N₄O₅ (605.72).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carboxamide (Intermediate 38c).

**Intermediate 38c** was synthesized following the procedure used for the preparation of **Intermediate 15c,** with (3'-carbamoyl-[1,1'-biphenyl]-4-yl)boronic acid substituted for pyridine-4-boronic acid. ESI-MS: m/z 536.21 [M+1]⁺. C₃₁H₂₈N₄O₅ (535.98).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carboxylic acid (Intermediate 39c).

**Intermediate 39c** was synthesized following the procedure used for the preparation of **Intermediate 15c,** with (3'-sulphamoyl-[1,1'-biphenyl]-4-yl)boronic acid substituted for pyridine-4-boronic acid. ESI-MS: m/z 537.57 [M+1]⁺. C₃₁H₂₇N₃O₆ (537.19).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-sulphonamide (Intermediate 40c).

**Intermediate 40c** was synthesized following the procedure used for the preparation of **Intermediate 15c,** with (3'-sulphamoyl-[1,1'-biphenyl]-4-yl)boronic acid substituted for pyridine-4-boronic acid. ESI-MS: m/z 572.64 [M+1]⁺. C₃₀H₂₈N₄O₆S (572.17).

### (R)-7-(benzyloxy)-12-((2'-nitro-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 41c).

**Intermediate 41c** was synthesized following the procedure used for the preparation of **Intermediate 15c,** with (2'-nitro-[1,1'-biphenyl]-4-yl)boronic acid substituted for pyridine-4-boronic acid. ESI-MS: m/z 538.56 [M+1]⁺. C₃₀H₂₆N₄O₆ (538.19).

### (R)-7-(benzyloxy)-12-((3'-(methylsulphonyl)-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 42c).

**Intermediate 42c** was synthesized following the procedure used for the preparation of **Intermediate 15c,** with (3'-(methylsulphonyl)-[1,1'-biphenyl]-4-yl)boronic acid substituted for pyridine-4-boronic acid. ESI-MS: m/z 571.18 [M+1]⁺. C₃₁H₂₉N₃O₆S (571.65).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-3-fluoro-[1,1'-biphenyl]-4-carbaldehyde (Intermediate 43c).

**Intermediate 43c** was synthesized following the procedure used for the preparation of **Intermediate 15c,** with (3'-fluoro-4'-formyl-[1,1'-biphenyl]-4-yl)boronic acid substituted for pyridine-4-boronic acid. ESI-MS: m/z 539.19 [M+1]⁺. C₃₁H₂₆FN₃O₅ (539.56).

### (R)-7-(benzyloxy)-12-((3'-fluoro-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 44c).

**Intermediate 44c** was synthesized following the procedure used for the preparation of **Intermediate 15c,** with (3'-fluoro-[1,1'-biphenyl]-4-yl)boronic acid substituted for pyridine-4-boronic acid. ESI-MS: m/z 511.19 [M+1]⁺. C₃₀H₂₆FN₃O₄ (511.55).

### (R)-7-(benzyloxy)-12-((3'-methoxy-[1,1'-biphenyl]-4-yl)methyl)-3,4,12,12a-tetrahydro-1H-[1,4]oxazino[3,4-c]pyrido[2,1-f][1,2,4]triazine-6,8-dione (Intermediate 45c).

**Intermediate 45c** was synthesized following the procedure used for the preparation of **Intermediate 15c,** with (3'-methoxy-[1,1'-biphenyl]-4-yl)boronic acid substituted for pyridine-4-boronic acid. ESI-MS: m/z 523.21 [M+1]⁺. C₃₁H₂₉N₃O₅ (523.59).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carbaldehyde (Intermediate 46c).

**Intermediate 46c** was synthesized following the procedure used for the preparation of **Intermediate 15c,** with (3'-formyl-[1,1'-biphenyl]-4-yl)boronic acid substituted for pyridine-4-boronic acid. ESI-MS: m/z 521.20 [M+1]⁺. C₃₁H₂₇N₃O₅ (521.57).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-3-fluoro-[1,1'-biphenyl]-4-carbonitrile (Intermediate 47c).

**Intermediate 47c** was synthesized following the procedure used for the preparation of **Intermediate 15c,** with (4'-cyano-3'-fluoro-[1,1'-biphenyl]-4-yl)boronic acid substituted for pyridine-4-boronic acid. ESI-MS: m/z 536.56 [M+1]⁺. C₃₁H₂₅FN₄O₄ (536.19).

### (R)-4'-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-2-carbaldehyde (Intermediate 48c).

**Intermediate 48c** was synthesized following the procedure used for the preparation of **Intermediate 15c,** with (2'-formyl-[1,1'-biphenyl]-4-yl)boronic acid substituted for pyridine-4-boronic acid. ESI-MS: m/z 521.57 [M+1]⁺. C₃₁H₂₇N₃O₅ (521.20).

### (R)-7-(benzyloxy)-12-((3',4'-dimethoxy-[1,1'-biphenyl]-4-yl)methyl)-3,4,12,12a-tetrahydro-1H-[1.4]oxazino[3,4-c]pyrido[2,1-f][1,2,4]triazine-6,8-dione (Intermediate 49c).

**Intermediate 49c** was synthesized following the procedure used for the preparation of **Intermediate 15c,** with (3',4'-dimethoxy-[1,1'-biphenyl]-4-yl)boronic acid substituted for pyridine-4-boronic acid. ESI-MS: m/z 553.62 [M+1]⁺. C₃₂H₃₁N₃O₆ (553.22).

### EXAMPLE 6: Synthesis of Compounds 1 to 14.

The **Intermediates 1b-14b** were weighed and placed in 25 mL nightshade bottles respectively and dissolved with 10 mL *N*,*N*-dimethylformamide (DMF). Then lithium chloride (10 eq.) was added at room temperature, and the reaction was heated to 95 °C for 12 h. After the reaction was over, the solvent DMF was removed under pressure, 10 mL of water was added to the concentrated residue, and the solid was then precipitated, and filtered. The filter cake was dissolved by methanol chromatography and purified by HPLC to obtain the corresponding target **Compounds 1-14.**

### (R)-12-benzoyl-7-hydroxy-3,4,12,12a-tetrahydro-1H-[1,4]oxazino[3,4-c]pyrido[2,1-f][1,2,4]triazine-6,8-dione (Compound 1).

Yellow solid, m.p.186-188 °C, yield 73.8%.

¹H NMR (400 MHz, DMSO-*d*₆) δ 7.83 - 7.73 (m, 3H, 3 × Ph-H), 7.62 (t, *J* = 7.8 Hz, 2H, 2 × Ph-H), 7.45 - 7.30 (m, 1H, CH=CH), 6.21 (d, *J* = 7.7 Hz, 1H, CH=CH), 5.47 (d, *J* = 10.1 Hz, 1H, CH), 4.32 (t, *J* = 14.4 Hz, 3H, 3 × CH), 3.93 (t, *J* = 10.7 Hz, 1H, CH), 3.68 (d, *J* = 11.5 Hz, 1H, CH), 3.24 (d, *J* = 12.3 Hz, 1H, CH). ¹³C NMR (100 MHz, DMSO-*d*₆) δ 173.58, 172.18, 160.73, 152.23, 140.12, 133.96, 130.81, 129.70, 129.46, 129.05, 128.74, 127.35, 117.98, 112.78, 70.29, 67.20, 65.70. HRMS (ESI): m/z calcd for C₁₇H₁₅N₃O₅ [M+H]⁺ 342.1090; found 342.1090.

### (R)-7-hydroxy-12-methyl-3,4,12,12a-tetrahydro-1H-[1,4]oxazino[3,4-c]pyrido[2,1-f][1,2,4]triazine-6,8-dione (Compound 2).

Yellow solid, m.p.130-132 °C, yield 20.3%.

¹H NMR (400 MHz, DMSO-*d*₆) δ 7.70 (d, *J* = 7.5 Hz, 1H, CH=CH), 6.12 (d, *J* = 7.3 Hz, 1H, CH=CH), 5.03 (dd, *J* = 10.4, 4.0 Hz, 1H, CH), 4.17 (d, *J* = 13.3 Hz, 1H, CH), 4.01 - 3.85 (m, 2H, 2 × CH), 3.62 - 3.45 (m, 2H, 2 × CH), 3.18 (dt, *J* = 13.0, 6.0 Hz, 1H, CH), 2.79 (s, 3H, NCH₃). ¹³C NMR (100 MHz, DMSO-*d*₆) δ 171.41, 162.47, 153.22, 137.67, 116.21, 113.27, 71.21, 66.70, 65.85, 42.79, 42.58. HRMS (ESI): m/z calcd for C₁₁H₁₃N₃O₄ [M+H]⁺ 252.0984; found 252.0983.

### (R)-12-ethyl-7-hydroxy-3,4,12,12a-tetrahydro-1H-[1,4]oxazino[3,4-c]pyrido[2,1-f][1,2,4]triazine-6,8-dione (Compound 3).

Yellow solid, m.p.135-136 °C, yield 25.4%.

¹H NMR (400 MHz, DMSO-*d*₆) δ 7.35 (s, 1H, CH=CH), 5.69 (s, 1H, CH=CH,), 4.75 (s, 1H, CH), 4.26 (s, 1H, CH), 3.90 - 3.68 (m, 2H, 2 × CH), 3.06 (s, 3H, 3 × CH), 2.95 (s, 1H, CH), 2.79 (s, 1H, CH), 0.94 (t, *J* = 7.1 Hz, 3H, CH₃). ¹³C NMR (100 MHz, DMSO-*d*₆) δ 171.36, 161.50, 136.72, 114.06, 71.58, 66.08, 35.13, 21.57, 13.03. HRMS (ESI): m/z calcd for C₁₂H₁₅N₃O₄ [M+H]⁺ 266.1141; found 266.1141.

### (R)-12-benzyl-7-hydroxy-3,4,12,12a-tetrahydro-1H-[1,4]oxazino[3,4-c]pyrido[2,1-f][1,2,4]triazine-6,8-dione (Compound 4).

Red brown solid, m.p. 223-225°C, yield 28.4%.

¹H NMR (400 MHz, DMSO-*d*₆) δ 7.35 - 7.30 (m, 3H, 3 × Ph-H), 7.29 - 7.23 (m, 2H, 2 × Ph-H), 7.03 (d, *J* = 7.6 Hz, 1H, CH=CH), 5.76 (d, *J* = 7.5 Hz, 1H, CH=CH), 5.12 - 4.98 (m, 1H, CH), 4.28 (s, 3H, 3 × CH), 3.98 - 3.75 (m, 3H, 3 × CH), 3.68 - 3.64 (m, 1H, CH), 3.18 (d, *J* = 12.5 Hz, 1H, CH). ¹³C NMR (100 MHz, DMSO-*d*₆) δ 171.47, 153.34, 138.36, 135.08, 130.36, 129.00, 128.89, 116.21, 111.65, 65.98. HRMS (ESI): m/z calcd for C₁₇H₁₇N₃O₄ [M+H]⁺ 328.1297; found 328.1298.

### (R)-7-hydroxy-12-(4-iodobenzyl)-3,4,12,12a-tetrahydro-1H-[1,4]oxazino[3,4-c]pyrido[2,1-f][1,2,4]triazine-6,8-dione (Compound 5).

Yellow solid, m.p. 240-242°C, yield 52.3%.

¹H NMR (400 MHz, DMSO-*d*₆) δ 7.69 (d, *J* = 8.0 Hz, 2H, 2 × Ph-H), 7.08 (d, *J* = 8.1 Hz, 3H, 2 × Ph-H, CH=CH), 5.77 (s, 1H, CH=CH), 5.02 (s, 1H, CH), 4.25 (s, 3H, 3 × CH), 3.98 - 3.75 (m, 2H, CH₂), 3.71 - 3.49 (m, 2H, 2 × CH), 3.17 (s, 1H, CH). ¹³C NMR (100 MHz, DMSO-*d*₆) δ 171.73, 153.26, 138.55, 137.80, 134.73, 132.59, 95.25, 65.97. HRMS (ESI): m/z calcd for C₁₇H₁₆IN₃O₄ [M+H]⁺ 454.0263; found 454.0260.

### (R)-12-(4-fluorobenzyl)-7-hydroxy-3,4,12,12a-tetrahydro-1H-[1,4]oxazino[3,4-c]pyrido[2,1-f][1,2,4]triazine-6,8-dione (Compound 6).

Light orange solid, m.p. 253-256 °C, yield 49.4%.

¹H NMR (400 MHz, DMSO-*d*₆) δ 11.72 (s, 1H, OH), 7.30 (dd, *J* = 8.4, 5.7 Hz, 2H, 2 × Ph-H), 7.15 (t, *J* = 8.8 Hz, 2H, 2 × Ph-H), 7.02 (d, *J* = 7.4 Hz, 1H, CH=CH), 5.81 - 5.75 (m, 1H, CH=CH), 5.05 (d, *J* = 9.7 Hz, 1H, CH), 4.29 (d, *J* = 9.1 Hz, 3H, 3 × CH), 3.87 (dd, *J* = 34.9, 9.8 Hz, 2H, CH₂), 3.66 (t, *J* = 10.5 Hz, 1H, CH), 3.51 (t, *J* = 11.7 Hz, 1H, CH), 3.24 (td, *J* = 12.5, 3.8 Hz, 1H, CH). ¹³C NMR (100 MHz, DMSO-*d*₆) δ 171.48, 163.76, 161.33, 153.36, 138.41, 132.62, 132.54, 131.43, 131.40, 116.20, 115.93, 115.72, 111.62, 65.97, 55.39. ¹³C NMR (100 MHz, DMSO-*d*₆) δ 171.48, 163.76, 161.33, 153.36, 138.41, 132.62, 132.54, 131.43, 131.40, 116.20, 115.93, 115.72, 111.62, 65.97, 55.39. HRMS (ESI): m/z calcd for C₁₇H₁₆FN₃O₄ [M+H]⁺ 346.1203; found 346.1201.

### (R)-7-hydroxy-12-(4-(trifluoromethyl)benzyl)-3,4,12,12a-tetrahydro-1H-[1,4]oxazino[3,4-c]pyrido[2,1-f][1,2,4]triazine-6,8-dione (Compound 7).

Light orange solid, m.p. 239-242 °C, yield 52.1%.

¹H NMR (400 MHz, DMSO-*d*₆) δ 11.68 (s, 1H, OH), 7.71 (d, *J* = 7.9 Hz, 2H, 2 × Ph-H), 7.54 (d, *J* = 7.9 Hz, 2H, 2 × Ph-H), 7.11 (d, *J* = 7.6 Hz, 1H, CH=CH), 5.79 (d, *J* = 7.6 Hz, 1H, CH=CH), 5.07 (d, *J* = 9.7 Hz, 1H, CH), 4.49 - 4.21 (m, 3H, 3 × CH), 3.89 (dd, *J* = 44.6, 10.0 Hz, 2H, CH₂), 3.65 (t, *J* = 10.6 Hz, 1H, CH), 3.58 - 3.45 (m, 1H, CH), 3.25 (t, *J* = 12.6 Hz, 1H, CH). ¹³C NMR (100 MHz, DMSO-*d*₆) δ 171.53, 153.41, 140.20, 138.30, 131.03, 129.73, 129.41, 129.10, 128.79, 128.69, 125.98, 125.88, 125.84, 125.80, 125.76, 123.28, 116.22, 111.77, 65.92. HRMS (ESI): m/z calcd for C₁₈H₁₆F₃N₃O₄ [M+H]⁺ 396.1171; found 396.1166.

### (R)-12-(2-fluorobenzyl)-7-hydroxy-3,4,12,12a-tetrahydro-1H-[1,4]oxazino[3,4-c]pyrido[2,1-f][1,2,4]triazine-6,8-dione (Compound 8).

Yellow solid, m.p. 121-124°C, yield 45.8%.

¹H NMR (400 MHz, DMSO-*d*₆) δ 7.41 (q, *J* = 7.2 Hz, 1H, Ph-H), 7.28 (t, *J* = 7.8 Hz, 1H, Ph-H), 7.21 - 7.09 (m, 2H, 2 × Ph-H), 6.98 (s, 1H, CH=CH), 5.77 (s, 1H, CH=CH), 5.12 (s, 1H, CH), 4.35 (s, 3H, 3 × CH), 3.99 - 3.76 (m, 2H, CH₂), 3.67 (s, 1H, CH), 3.51 (s, 1H, CH), 3.22 (t, *J* = 11.9 Hz, 1H, CH). ¹³C NMR (100 MHz, DMSO-*d*₆) δ 171.53, 162.82, 160.36, 153.28, 138.19, 133.20, 133.17, 131.66, 131.58, 125.08, 125.04, 121.96, 121.81, 116.22, 116.00, 115.79, 111.76, 65.93. HRMS (ESI): m/z calcd for C₁₇H₁₆FN₃O₄ [M+H]⁺ 346.1203; found 346.1201.

### (R)-12-(3-fluorobenzyl)-7-hydroxy-3,4,12,12a-tetrahydro-1H-[1,4]oxazino[3,4-c]pyrido[2,1-f][1,2,4]triazine-6,8-dione (Compound 9).

Yellow solid, m.p.120-123 °C, yield 45.6%.

¹H NMR (400 MHz, DMSO-*d*₆) δ 7.37 (q, *J* = 7.4 Hz, 1H, Ph-H), 7.17 (t, *J* = 8.5 Hz, 3H, 3 × Ph-H), 7.10 (d, *J* = 7.6 Hz, 2H, Ph-H, CH=CH), 5.77 (s, 1H, CH=CH), 5.02 (s, 1H, CH), 4.30 (s, 3H, 3 × CH), 3.95 - 3.79 (m, 2H, CH₂), 3.61 (s, 2H, 2 × CH), 3.17 (s, 1H, CH). ¹³C NMR (100 MHz, DMSO-*d*₆)δ 171.76, 164.23, 163.71, 161.29, 153.22, 138.54, 137.73, 131.07, 130.99, 126.41, 117.09, 116.88, 115.90, 115.69, 111.17, 65.96. HRMS (ESI): m/z calcd for C₁₇H₁₆FN₃O₄ [M+Na]⁺ 368.1023; found 368.1023.

### (R)-12-(4-chloro-3-fluorobenzyl)-7-hydroxy-3,4,12,12a-tetrahydro-1H-[1,4]oxazino[3,4-c]pyrido[2,1-f][1,2,4]triazine-6,8-dione (Compound 10).

Light orange solid, m.p. 241-244 °C, yield 49.7%.

¹H NMR (400 MHz, DMSO-*d*₆) δ 11.75 (s, 1H, OH), 7.55 (t, *J* = 8.0 Hz, 1H, Ph-H), 7.43 (d, *J* = 10.1 Hz, 1H, CH=CH), 7.16 (t, *J* = 7.7 Hz, 2H, 2 × Ph-H), 5.83 (d, *J* = 7.5 Hz, 1H, CH=CH), 5.06 (s, 1H, CH), 4.38 - 4.20 (m, 3H, 3 × CH), 3.88 (dd, *J* = 41.8, 12.0 Hz, 2H, CH₂), 3.63 (t, *J* = 10.5 Hz, 1H, CH), 3.49 (d, *J* = 12.2 Hz, 1H, CH), 3.26 (d, *J* = 12.4 Hz, 1H, CH). ¹³C NMR (100 MHz, DMSO-*d*₆) δ 171.53, 158.74, 156.29, 153.39, 138.44, 137.24, 131.14, 127.57, 119.93, 119.76, 118.74, 118.52, 116.22, 111.82, 65.92. HRMS (ESI): m/z calcd for C₁₇H₁₅ClFN₃O₄ [M+H]⁺ 380.0813; found 380.0810.

### (R)-7-hydroxy-12-(naphthalen-1-ylmethyl)-3,4,12,12a-tetrahydro-1H-[1,4]oxazino[3,4-c]pyrido[2,1-f][1,2,4]triazine-6,8-dione (Compound 11).

Light orange solid, m.p. 279-282 °C, yield 45.2%.

¹H NMR (400 MHz, DMSO-*d*₆) δ 11.86 (s, 1H, OH), 8.26 (s, 1H, Ph-H), 7.95 (dd, *J* = 20.1, 8.2 Hz, 2H, 2 × Ph-H), 7.75 - 7.51 (m, 2H, 2 × Ph-H), 7.35 (t, *J* = 7.5 Hz, 1H, Ph-H), 7.26 - 7.11 (m, 1H, Ph-H), 6.40 (s, 1H, CH=CH), 5.52 (s, 1H, CH=CH), 5.29 (s, 1H, CH), 4.73 (s, 2H, CH₂), 4.38 (s, 1H, CH), 3.95 (d, *J* = 64.5 Hz, 2H, CH₂), 3.53 (d, *J* = 13.6 Hz, 2H, CH₂), 3.20 (d, *J* = 57.7 Hz, 1H, CH). ¹³C NMR (150 MHz, DMSO-d₆) δ 171.36, 153.49, 138.01, 133.79, 132.01, 130.32, 129.98, 129.80, 129.08, 127.21, 126.55, 125.73, 124.62, 115.97, 111.07, 66.02, 55.33, 53.88, 42.14. HRMS (ESI): m/z calcd for C₂₁H₁₉N₃O₄ [M+H]⁺ 378.1454; found 378.1455.

### (R)-12-(3-chloro-4-fluorobenzyl)-7-hydroxy-3,4,12,12a-tetrahydro-1H-[1,4]oxazino[3,4-c]pyrido[2,1-f][1,2,4]triazine-6,8-dione (Compound 12)

Light orange solid, m.p. 267-270 °C, yield 41.9%.

¹H NMR (400 MHz, DMSO-*d*₆) δ 11.67 (s, 1H, OH), 7.56 (d, *J* = 7.0 Hz, 1H, Ph-H), 7.37 (t, *J* = 8.9 Hz, 1H, Ph-H), 7.29 (t, *J* = 6.6 Hz, 1H, Ph-H), 7.11 (d, *J* = 7.7 Hz, 1H, CH=CH), 5.82 (d, *J* = 7.5 Hz, 1H, CH=CH), 5.05 (s, 1H, CH), 4.30 (s, 3H, 3 × CH), 4.00 - 3.76 (m, 2H, CH₂), 3.63 (t, *J* = 10.2 Hz, 1H, CH), 3.49 (d, *J* = 11.0 Hz, 1H, CH), 3.23 (d, *J* = 12.4 Hz, 1H, CH). ¹³C NMR (100 MHz, DMSO-*d*₆) δ 171.53, 158.78, 156.33, 153.39, 138.45, 133.35, 132.52, 131.19, 131.12, 120.05, 119.87, 117.48, 117.27, 116.23, 111.72, 65.94. ESI-MS: m/z 380.02 [M+1]⁺. C₁₇H₁₅ClFN₃O₄ (379.07).

### (R)-7-hydroxy-12-(thiophen-2-ylmethyl)-3,4,12,12a-tetrahydro-1H-[1,4]oxazino[3,4-c]pyrido[2,1-f][1,2,4]triazine-6,8-dione (Compound 13)

**Intermediate 13b** was subjected to benzyl deprotection with LiCl in DMF solvent, and the target product **Compound 13** was obtained by HPLC separation. Yellow solid, m.p. 213-215°C, yield 25.6%. ¹H NMR (400 MHz, DMSO-*d*₆) δ 7.53 (dd, *J* = 4.9, 2.9 Hz, 1H, Ph-H), 7.35 (d, *J* = 2.9 Hz, 1H, Ph-H), 7.15 - 703 (m, 2H, Ph-H, CH=CH), 5.82 (d, *J* = 7.6 Hz, 1H, CH=CH), 5.13 - 4.99 (m, 1H, CH), 4.31 (t, *J* = 10.1 Hz, 3H, 3 × CH ), 3.98 - 3.78 (m, 2H, 2 × CH ), 3.65 (t, *J* = 10.6 Hz, 1H, CH), 3.52 (d, *J* = 11.4 Hz, 1H, CH), 3.29 - 3.20 (m, 1H, CH).¹³C NMR (100 MHz, DMSO) δ 171.47, 161.98, 153.27, 138.25, 135.63, 129.08, 127.45, 126.98, 116.19, 111.67, 72.03, 67.64, 65.99, 49.09. ESI-MS: m/z 334.27 [M+1]⁺. C₁₅H₁₅N₃O₄S(333.07)

### (R)-4-((7-hydroxy-6,8-dioxo-1,3,4,6,8,12a-hexahydro-12H-[1,4]oxazino[3,4-c]pyrido[2,1-f][1,2,4]triazin-12-yl)methyl)benzamide (Compound 14)

Yellow solid, m.p. 284-286°C, yield 45.3%.

¹H NMR (400 MHz, DMSO-*d*₆) δ 8.00 (s, 1H, Ph-H), 7.84 (d, *J* = 7.8 Hz, 2H, CONH₂), 7.48 - 7.27 (m, 3H, 3 × Ph-H), 7.09 (d, *J* = 7.7 Hz, 1H, CH=CH), 5.79 (d, *J* = 7.6 Hz, 1H, CH=CH), 5.15 - 4.97 (m, 1H, CH), 4.33 (q, *J* = 14.1 Hz, 3H, 3 × CH ), 3.94 (d, *J* = 10.8 Hz, 1H, CH), 3.84 (d, *J* = 11.5 Hz, 1H, CH), 3.66 (t, *J* = 10.6 Hz, 1H, CH), 3.52 (t, *J* = 11.3 Hz, 1H, CH), 3.25 (t, *J* = 13.1 Hz, 1H, CH).¹³C NMR (100 MHz, DMSO-*d*₆) δ 171.51, 167.88, 153.37, 138.40, 134.48, 130.14, 129.84, 128.63, 128.16, 116.22, 111.76, 65.96, 49.08. ESI-MS: m/z 371.40 [M+1]⁺. C₁₈H₁₈N₄O₅(370.13).

### EXAMPLE 7: Synthesis of Compounds 15 to 49.

The **Intermediates 15c-49c** were weighed and placed in 25 mL nightshade bottles respectively and dissolved with 10 mL *N*,*N*-dimethylformamide (DMF). Then lithium chloride (10 eq.) was added at room temperature, and the reaction was heated to 95 °C for 12 h. After the reaction was over, the solvent DMF was removed under pressure, 10 mL of water was added to the concentrated residue, and the solid was then precipitated, and filtered. The filter cake was dissolved by methanol chromatography and purified by HPLC to obtain the corresponding target **Compounds 15-49.**

### (R)-7-hydroxy-12-(4-(pyridin-4-yl)benzyl)-3,4,12,12a-tetrahydro-1H-[1,4]oxazino[3,4-c]pyrido[2,1-f][1,2,4]triazine-6,8-dione (Compound 15).

Light orange solid, m.p. 260-263 °C, yield 49.7%.

¹H NMR (400 MHz, DMSO-*d*₆) δ 11.75 (s, 1H, OH), 8.64 (d, *J* = 5.0 Hz, 2H, 2 × Ph-H), 7.80 (d, *J* = 7.9 Hz, 2H, 2 × Ph-H), 7.73 (d, *J* = 5.0 Hz, 2H, 2 × Ph-H), 7.44 (d, *J* = 7.8 Hz, 2H, 2 × Ph-H), 7.13 (d, *J* = 7.6 Hz, 1H, CH=CH), 5.80 (d, *J* = 7.6 Hz, 1H, CH=CH), 5.07 (d, *J* = 10.1 Hz, 1H, CH), 4.44 - 4.24 (m, 3H, 3 × CH), 3.89 (dd, *J* = 41.2, 10.7 Hz, 2H, CH₂), 3.67 (t, *J* = 9.7 Hz, 1H, CH), 3.50 (d, *J* = 11.4 Hz, 1H, CH), 3.26 (t, *J* = 12.6 Hz, 1H, CH). ¹³C NMR (100 MHz, DMSO-*d*₆) δ 171.53, 153.41, 150.76, 146.72, 138.41, 137.36, 136.47, 131.15, 127.33, 121.55, 116.24, 111.80, 65.97. HRMS (ESI): m/z calcd for C₂₂H₂₀N₄O₄ [M+H]⁺ 405.1563; found 405.1560.

### (R)-7-hydroxy-12-(4-(pyrimidin-5-yl)benzyl)-3,4,12,12a-tetrahydro-1H-[1,4]oxazino[3,4-c]pyrido[2,1-f][1,2,4]triazine-6,8-dione (Compound 16).

Light orange solid, m.p. 242-244 °C, yield 37%.

¹H NMR (400 MHz, DMSO-*d*₆) δ 11.75 (s, 1H, OH), 9.18 (d, *J* = 10.2 Hz, 3H, 3 × Ph-H), 7.93 - 7.72 (m, 2H, 2 × Ph-H), 7.47 (d, *J* = 7.1 Hz, 2H, 2 × Ph-H), 7.15 (d, *J* = 7.6 Hz, 1H, CH=CH), 5.86 - 5.74 (m, 1H, CH=CH), 5.06 (s, 1H, CH), 4.34 (d, *J* = 18.4 Hz, 3H, 3 × CH), 4.03 - 3.76 (m, 2H, CH₂), 3.68 (d, *J* = 9.5 Hz, 1H, CH), 3.58 - 3.44 (m, 1H, CH), 3.27 (d, *J* = 13.3 Hz, 1H, CH). ¹³C NMR (100 MHz, DMSO-*d*₆) δ 157.85, 155.13, 138.37, 136.18, 134.07, 133.02, 131.20, 127.40, 116.20, 111.79, 65.98, 55.39. HRMS (ESI): m/z calcd for C₂₁H₁₉N₅O₄ [M+H]⁺ 406.1515; found 406.1512.

### (R)-12-(4-(1H-pyrazol-4-yl)benzyl)-7-hydroxy-3,4,12,12a-tetrahydro-1H-[1,4]oxazino[3,4-c]pyrido[2,1-f][1,2,4]triazine-6,8-dione (Compound 17).

Light orange solid, m.p. 291-294 °C, yield 52%.

¹H NMR (400 MHz, DMSO-*d*₆) δ 12.96 (s, 1H, NH), 11.77 (s, 1H, OH), 8.08 (s, 2H, 2CH=CH), 7.57 (d, *J* = 7.7 Hz, 2H, 2 × Ph-H), 7.21 (d, *J* = 7.8 Hz, 2H, 2 × Ph-H), 7.08 (d, *J* = 7.6 Hz, 1H, CH=CH), 5.78 (d, *J* = 7.7 Hz, 1H, CH=CH), 5.07 (s, 1H, CH), 4.26 (s, 3H, 3 × CH), 4.00 - 3.75 (m, 2H, CH₂), 3.66 (t, *J* = 9.8 Hz, 1H, CH), 3.49 (d, *J* = 12.8 Hz, 1H, CH), 3.26 (d, *J* = 10.3 Hz, 1H, CH). ¹³C NMR (100 MHz, DMSO-*d*₆) δ 171.49, 153.36, 138.52, 133.47, 132.31, 130.92, 125.50, 121.10, 116.22, 111.68, 65.98, 55.39. HRMS (ESI): m/z calcd for C₂₀H₁₉N₅O₄ [M+H]⁺ 394.1515; found 394.1515.

### (R)-12-(4-(furan-2-yl)benzyl)-7-hydroxy-3,4,12,12a-tetrahydro-1H-[1,4]oxazino[3,4-c]pyrido[2,1-f][1,2,4]triazine-6,8-dione (Compound 18).

Light orange solid, m.p. 225-228 °C, yield 45.8%.

¹H NMR (400 MHz, DMSO-*d*₆) δ 11.71 (s, 1H, OH), 7.76 (d, *J* = 1.8 Hz, 1H, CH=CH), 7.65 (d, *J* = 8.0 Hz, 2H, 2 × Ph-H), 7.30 (d, *J* = 8.0 Hz, 2H, 2 × Ph-H), 7.06 (d, *J* = 7.7 Hz, 1H, CH=CH), 6.98 (d, *J* = 3.3 Hz, 1H, CH=CH), 6.60 (dd, *J* = 3.5, 1.8 Hz, 1H, CH=CH), 5.77 (d, *J* = 7.3 Hz, 1H, CH=CH), 5.07 (d, *J* = 8.1 Hz, 1H, CH), 4.40 - 4.19 (m, 3H, 3 × CH), 3.89 (dd, *J* = 39.9, 9.4 Hz, 2H, CH₂), 3.68 (d, *J* = 11.7 Hz, 1H, CH), 3.51 (t, *J* = 18.1 Hz, 1H, CH), 3.24 (td, *J* = 12.9, 3.8 Hz, 1H, CH). ¹³C NMR (100 MHz, DMSO-*d*₆) δ 171.48, 153.37, 153.06, 143.59, 138.46, 134.13, 131.00, 130.74, 123.89, 116.21, 112.63, 111.67, 106.84, 65.98. HRMS (ESI): m/z calcd for C₂₁H₁₉N₃O₅ [M+H]⁺ 394.1403; found 394.1402.

### (R)-12-([1,1'-biphenyl]-4-yimethyl)-7-hydroxy-3,4,12,12a-tetrahydro-1H-[1,4]oxazino[3,4-c]pyrido[2,1-f][1,2,4]triazine-6,8-dione (Compound 19).

Light orange solid, m.p. 258-261 °C, yield 50%.

¹H NMR (400 MHz, DMSO-*d*₆) δ 11.64 (s, 1H, OH), 7.66 (dd, *J* = 13.1, 7.8 Hz, 4H, 4 × Ph-H), 7.47 (t, *J* = 7.5 Hz, 2H, 2 × Ph-H), 7.36 (d, *J* = 8.0 Hz, 3H, 3 × Ph-H), 7.12 (d, *J* = 7.6 Hz, 1H, CH=CH), 5.80 (d, *J* = 7.5 Hz, 1H, CH=CH), 5.06 (d, *J* = 10.3 Hz, 1H, CH), 4.32 (s, 3H, 3 × CH), 3.88 (dd, *J* = 40.4, 9.9 Hz, 2H, CH₂), 3.66 (t, *J* = 10.1 Hz, 1H, CH), 3.50 (d, *J* = 11.9 Hz, 1H, CH), 3.25 (t, *J* = 10.8 Hz, 1H, CH). ¹³C NMR (100 MHz, DMSO-*d*₆) δ 171.54, 153.44, 140.44, 139.89, 138.44, 134.36, 130.94, 129.45, 128.12, 127.15, 127.08, 116.22, 111.74, 65.98, 55.39. HRMS (ESI): m/z calcd for C₂₃H₂₁N₃O₄ [M+H]⁺ 404.1610; found 404.1609.

### (R)-12-(4-(benzo[d][1,3]dioxol-5-yl)benzyl)-7-hydroxy-3,4,12,12a-tetrahydro-1H-[1,4]oxazino[3,4-c]pyrido[2,1-f][1,2,4]triazine-6,8-dione (Compound 20).

Light orange solid, m.p. 239-242 °C, yield 47.5%.

¹H NMR (400 MHz, DMSO-*d*₆) δ11.83 (s, 1H, OH), 7.58 (d, *J* = 7.9 Hz, 2H, 2 × Ph-H), 7.35 - 7.23 (m, 3H, 3 × Ph-H), 7.20 - 7.08 (m, 2H, 2 × Ph-H), 6.99 (d, *J* = 8.1 Hz, 1H, CH=CH), 6.06 (s, 2H, CH2), 5.80 (d, *J* = 7.6 Hz, 1H, CH=CH), 5.05 (s, 1H, CH), 4.31 (d, *J* = 12.3 Hz, 3H, 3 × CH), 3.98 - 3.79 (m, 2H, CH₂), 3.65 (d, *J* = 10.4 Hz, 1H, CH), 3.51 (t, *J* = 12.8 Hz, 1H, CH), 3.24 (td, *J* = 12.5, 3.6 Hz, 1H, CH). ¹³C NMR (100 MHz, DMSO-*d*₆) δ 171.53, 153.43, 148.49, 147.45, 140.16, 138.43, 134.16, 133.84, 130.83, 126.85, 120.72, 116.19, 111.77, 109.15, 107.47, 101.65, 65.98. HRMS (ESI): m/z calcd for C₂₄H₂₁N₃O₆ [M+H]⁺ 448.1508; found 448.1507.

### (R)-7-hydroxy-12-((2'-hydroxy-[1,1'-biphenyl]-4-yl)methyl)-3,4,12,12a-tetrahydro-1H-[1,4]oxazino[3,4-c]pyrido[2,1-f][1,2,4]triazine-6,8-dione (Compound 21).

Light orange solid, m.p. 289-291 °C, yield 42.6%.

¹H NMR (600 MHz, DMSO-*d*₆) δ 11.84 (s, 1H, OH), 9.56 (s, 1H, OH), 7.52 (d, *J* = 8.0 Hz, 2H, 2 × Ph-H), 7.30 (d, *J* = 7.8 Hz, 2H, 2 × Ph-H), 7.25 (dd, *J* = 7.6, 1.7 Hz, 1H, Ph-H), 7.16 (td, *J* = 7.7, 1.8 Hz, 2H, 2 × Ph-H), 6.94 (d, *J* = 8.1 Hz, 1H, CH=CH), 6.87 (t, *J* = 7.4 Hz, 1H, Ph-H), 5.83 (d, *J* = 7.6 Hz, 1H, CH=CH), 5.03 (s, 1H, CH), 4.29 (s, 3H, 3 × CH), 3.97 - 3.78 (m, 2H, CH₂), 3.67 (d, *J* = 10.7 Hz, 1H, CH), 3.52 (d, *J* = 12.1 Hz, 1H, CH), 3.29 - 3.22 (m, 1H, CH). ¹³C NMR (100 MHz, DMSO-*d*₆) δ 171.53, 154.78, 153.41, 139.04, 138.45, 133.30, 130.77, 129.83, 129.59, 129.13, 127.48, 119.93, 116.56, 116.21, 111.91, 65.99. HRMS (ESI): m/z calcd for C₂₃H₂₁N₃O₅ [M+H]⁺ 420.1559; found 420.1562.

### (R)-7-hydroxy-12-((3'-hydroxy-[1,1-biphenyl]-4-yl)methyl)-3,4,12,12a-tetrahydro-1H-[1,4]oxazino[3,4-c]pyrido[2,1-f][1,2,4]triazine-6,8-dione (Compound 22).

Light orange solid, m.p. 271-274 °C, yield 41%.

¹H NMR (400 MHz, DMSO-*d*₆) δ 11.66 (s, 1H, OH), 9.53 (s, 1H, OH), 7.56 (d, *J* = 7.8 Hz, 2H, 2 × Ph-H), 7.33 (d, *J* = 7.8 Hz, 2H, 2 × Ph-H), 7.25 (t, *J* = 7.9 Hz, 1H, Ph-H), 7.09 (dd, *J* = 15.6, 7.7 Hz, 2H, 2 × Ph-H), 7.03 (s, 1H, Ph-H), 6.77 (d, *J* = 8.0 Hz, 1H, CH=CH), 5.80 (d, *J* = 7.6 Hz, 1H, CH=CH), 5.06 (d, *J* = 9.8 Hz, 1H, CH), 4.30 (d, *J* = 10.3 Hz, 3H, 3 × CH), 3.89 (dd, *J* = 39.6, 11.3 Hz, 2H, CH₂), 3.66 (t, *J* = 10.7 Hz, 1H, CH), 3.51 (t, *J* = 12.0 Hz, 1H, CH), 3.31 - 3.22 (m, 1H, CH). ¹³C NMR (101 MHz, DMSO-*d*₆) δ 171.52, 158.31, 153.40, 141.35, 140.63, 138.44, 134.27, 130.87, 130.44, 127.06, 117.84, 116.22, 115.11, 113.88, 111.77, 65.98. HRMS (ESI): m/z calcd for C₂₃H₂₁N₃O₅ [M+H]⁺ 420.1559; found 420.1559.

### (R)-7-hydroxy-12-((4'-hydroxy-[1,1]-biphenyl]-4-yl)methyl)-3,4,12,12a-tetrahydro-1H-[1,4]oxazino[3,4-c]pyrido[2,1-f][1,2,4]triazine-6,8-dione (Compound 23).

Light orange solid, m.p. 280-283 °C, yield 51.8%.

¹H NMR (600 MHz, DMSO-*d*₆) δ 11.71 (s, 1H, OH), 9.61 (s, 1H, OH), 7.55 (d, *J* = 7.8 Hz, 2H, 2 × Ph-H), 7.50 (d, *J* = 8.2 Hz, 2H, 2 × Ph-H), 7.28 (d, *J* = 7.8 Hz, 2H, 2 × Ph-H), 7.14 - 7.06 (m, 1H, CH=CH), 6.84 (d, *J* = 8.3 Hz, 2H, 2 × Ph-H), 5.79 (d, *J* = 7.6 Hz, 1H, CH=CH), 5.04 (s, 1H, CH), 4.29 (s, 3H, 3 × CH), 3.88 (d, *J* = 57.5 Hz, 2H, CH₂), 3.66 (s, 1H, CH), 3.50 (s, 1H, CH), 3.28 - 3.20 (m, 1H, CH). ¹³C NMR (100 MHz, DMSO-*d*₆) δ 171.50, 157.77, 152.15, 140.48, 138.41, 133.09, 130.85, 130.56, 128.15, 126.31, 116.22, 111.61, 65.99, 55.48. HRMS (ESI): m/z calcd for C₂₃H₂₁N₃O₅ [M+H]⁺ 420.1559; found 420.1556.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carbonitrile (Compound 24).

Light orange solid, m.p. 217-220 °C, yield 43%.

¹H NMR (400 MHz, DMSO-*d*₆) δ 11.73 (s, 1H, OH), 8.18 (s, 1H, Ph-H), 8.05 (d, *J* = 8.7 Hz, 1H, Ph-H), 7.84 (d, *J* = 7.6 Hz, 1H, Ph-H), 7.75 (d, *J* = 7.9 Hz, 2H, 2 × Ph-H), 7.67 (t, *J* = 7.8 Hz, 1H, Ph-H), 7.41 (d, *J* = 7.9 Hz, 2H, 2 × Ph-H), 7.13 (d, *J* = 7.6 Hz, 1H, CH=CH), 5.81 (d, *J* = 7.7 Hz, 1H, CH=CH), 5.07 (s, 1H, CH), 4.33 (t, *J* = 14.6 Hz, 3H, 3 × CH), 3.89 (dd, *J* = 41.1, 10.1 Hz, 2H, CH₂), 3.66 (d, *J* = 10.0 Hz, 1H, CH), 3.51 (d, *J* = 11.6 Hz, 1H, CH), 3.25 (td, *J* = 12.5, 3.7 Hz, 1H, CH). ¹³C NMR (100 MHz, DMSO-*d*₆) δ 171.52, 153.42, 140.98, 138.40, 138.23, 135.51, 131.86, 131.70, 131.07, 130.65, 127.42, 119.24, 116.22, 112.62, 111.81, 65.98. HRMS (ESI): m/z calcd for C₂₄H₂₀N₄O₄ [M+H]⁺ 429.1563; found 429.1563.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carbonitrile (Compound 25).

Light orange solid, m.p. 237-240 °C, yield 51%.

¹H NMR (400 MHz, DMSO-*d*₆) δ 11.75 (s, 1H, OH), 7.97 - 7.88 (m, 4H, 4 × Ph-H), 7.74 (d, *J* = 7.8 Hz, 2H, 2 × Ph-H), 7.42 (d, *J* = 7.7 Hz, 2H, 2 × Ph-H), 7.13 (d, *J* = 7.6 Hz, 1H, CH=CH), 5.80 (d, *J* = 7.6 Hz, 1H, CH=CH), 5.06 (d, *J* = 8.0 Hz, 1H, CH), 4.31 (d, *J* = 29.2 Hz, 3H, 3 × CH), 3.89 (dd, *J* = 39.9, 10.3 Hz, 2H, CH₂), 3.67 (t, *J* = 10.3 Hz, 1H, CH), 3.51 (d, *J* = 100 Hz, 1H, CH), 3.25 (t, *J* = 10.8 Hz, 1H, CH). ¹³C NMR (100 MHz, DMSO-*d*₆) δ 171.52, 153.41, 144.35, 138.46, 135.93, 133.36, 131.11, 127.96, 127.59, 119.31, 116.23, 111.82, 110.65, 65.97. HRMS (ESI): m/z calcd for C₂₄H₂₀N₄O₄ [M+H]⁺ 429.1563; found 429.1562.

### (R)-12-((2'-amino-[1,1'-biphenyl]-4-yl)methyl)-7-hydroxy-3,4,12,12a-tetrahydro-1H-[1,4]oxazino[3,4-c]pyrido[2,1-f][1,2,4]triazine-6,8-dione (Compound 26).

Light orange solid, m.p. 214-217 °C, yield 39.9%.

¹H NMR (400 MHz, DMSO-*d*₆) δ 11.82 (s, 1H, OH), 7.33 (q, *J* = 7.9 Hz, 4H, 4 × Ph-H), 7.16 (d, *J* = 7.3 Hz, 1H, Ph-H), 7.05 (t, *J* = 7.7 Hz, 1H, Ph-H), 6.96 (d, J = 7.5 Hz, 1H, CH=CH), 6.76 (d, J = 8.0 Hz, 1H, Ph-H), 6.63 (t, J = 7.4 Hz, 1H, Ph-H), 5.81 (d, J = 7.6 Hz, 1H, CH=CH), 5.08 (d, J = 7.4 Hz, 1H, CH), 4.73 (s, 2H, NH₂), 4.31 (s, 3H, 3 × CH), 3.89 (dd, J = 39.6, 10.2 Hz, 2H, CH₂), 3.68 (d, J = 10.7 Hz, 1H, CH), 3.51 (t, J = 9.9 Hz, 1H, CH), 3.25 (t, J = 12.7 Hz, 1H, CH). ¹³C NMR (100 MHz, DMSO-d6) δ 171.50, 153.30, 145.40, 140.19, 138.53, 133.44, 130.78, 130.37, 129.21, 128.80, 125.69, 125.66, 117.19, 116.33, 115.69, 111.77, 65.99, 55.39. HRMS (ESI): m/z calcd for C₂₃H₂₂N₄O₄ [M+H]⁺ 419.1719; found 419.1716.

### (R)-12-((3'-amino-[1,1'-biphenyl]-4-yl)methyl)-7-hydroxy-3,4,12,12a-tetrahydro-1H-[1,4]oxazino[3,4-c]pyrido[2,1-f][1,2,4]triazine-6,8-dione (Compound 27).

Yellow solid, m.p. 180-182°C, yield 34.2%.

¹H NMR (400 MHz, DMSO-*d*₆) δ 7.52 (d, *J* = 7.8 Hz, 2H, 2× Ph-H), 7.30 (d, *J* = 7.8 Hz, 2H, 2×Ph-H), 7.09 (t, *J* = 7.5 Hz, 2H, Ph-H, CH=CH), 6.85 (s, 1H, Ph-H), 6.78 (d, *J* = 7.7 Hz, 1H, Ph-H), 6.57 (d, *J* = 8.0 Hz, 1H, Ph-H), 5.80 (d, *J* = 7.6 Hz, 1H, CH=CH), 5.20 (s, 2H, NH₂), 5.07 (s, 1H, CH), 4.31 (s, 3H, 3×CH), 3.96 - 3.81 (m, 2H, CH₂), 3.67 (s, 2H, 2× CH), 3.29 (s, 1H, CH). ¹³C NMR (100 MHz, DMSO-*d*₆) δ 171.51, 153.39, 149.62, 141.39, 140.62, 138.46, 133.88, 130.80, 129.92, 126.90, 114.69, 113.79. HRMS (ESI): m/z calcd for C₂₃H₂₂N₄O₄ [M+H]⁺ 419.1719; found 419.1719.

### (R)-12-((4'-amino-[1,1'-biphenyl]-4-yl)methyl)-7-hydroxy-3,4,12,12a-tetrahydro-1H-[1,4]oxazino[3,4-c]pyrido[2,1-f][1,2,4]triazine-6,8-dione (Compound 28).

Yellow solid, m.p. 278-280 °C, yield 33.5%.

¹H NMR (400 MHz, DMSO-*d*₆) δ 7.51 (s, 2H, 2× Ph-H), 7.37 (s, 2H, 2× Ph-H), 7.23 (s, 2H, 2× Ph-H), 7.08 (s, 1H, CH=CH), 6.63 (d, *J* = 8.1 Hz, 2H, 2× Ph-H), 5.77 (s, 1H, CH=CH), 5.28 (s, 2H, NH₂), 5.03 (s, 1H, CH), 4.27 (s, 3H, 3× CH), 3.97 - 3.73 (m, 3H, 3× CH), 3.71 - 3.63 (m, 1H, CH), 3.17 (s, 1H, CH). ¹³C NMR (100 MHz, DMSO-*d*₆) δ 171.55, 152.91, 148.98, 139.87, 132.30, 130.70, 127.57, 127.07, 125.66, 114.71, 66.05. HRMS (ESI): m/z calcd for C₂₃H₂₂N₄O₄ [M+H]⁺ 419.1719; found 419.1720.

### (R)-12-((4'-fluoro-[1,1'-biphenyl]-4-yl)methyl)-7-hydroxy-3,4,12,12a-tefrahydro-1H-[1,4]oxazino[3,4-c]pyrido[2,1-f][1,2,4]triazine-6,8-dione (Compound 29).

Light orange solid, m.p. 267-270 °C, yield 51%.

¹H NMR (400 MHz, DMSO-*d*₆) δ 11.77 (s, 1H, OH), 7.72 (dd, *J* = 8.4, 5.5 Hz, 2H, 2 × Ph-H), 7.63 (d, *J* = 7.8 Hz, 2H, 2 × Ph-H), 7.46 - 7.25 (m, 4H, 4 × Ph-H), 7.12 (d, *J* = 7.7 Hz, 1H, CH=CH), 5.80 (d, *J* = 7.6 Hz, 1H, CH=CH), 5.06 (d, *J* = 9.0 Hz, 1H, CH), 4.32 (s, 3H, 3 × CH), 3.89 (dd, *J* = 38.3, 10.2 Hz, 2H, CH₂), 3.67 (t, *J* = 10.1 Hz, 1H, CH), 3.51 (t, *J* = 8.7 Hz, 1H, CH), 3.32 - 3.20 (m, 1H, CH). ¹³C NMR (100 MHz, DMSO-*d*₆) δ 171.50, 163.66, 161.22, 153.40, 139.42, 138.41, 136.40, 136.37, 134.37, 130.95, 129.13, 129.05, 127.09, 116.32, 116.22, 116.11, 111.79, 65.98. HRMS (ESI): m/z calcd for C₂₃H₂₀FN₃O₄ [M+H]⁺ 422.1516; found 422.1513.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-2-carbonitrile (Compound 30).

Light orange solid, m.p. 249-252 °C, yield 56%.

¹H NMR (400 MHz, DMSO-*d*₆) δ 11.71 (s, 1H, OH), 7.95 (d, *J* = 7.6 Hz, 1H, Ph-H), 7.80 (t, *J* = 7.6 Hz, 1H, Ph-H), 7.64 - 7.53 (m, 4H, 4 × Ph-H), 7.46 (d, *J* = 7.8 Hz, 2H, 2 × Ph-H), 7.14 (s, 1H, CH=CH), 5.82 (d, *J =* 7.6 Hz, 1H, CH=CH), 5.07 (d, *J* = 7.5 Hz, 1H, CH), 4.38 (s, 3H, 3 × CH), 3.90 (dd, *J* = 44.4, 10.9 Hz, 2H, CH₂), 3.67 (t, *J* = 10.6 Hz, 1H, CH), 3.51 (d, *J* = 8.8 Hz, 1H, CH), 3.31 - 3.21 (m, 1H, CH). ¹³C NMR (100 MHz, DMSO-*d*₆) δ 171.52, 153.41, 144.53, 138.29, 135.98, 134.32, 134.01, 130.56, 130.54, 129.33, 128.82, 118.91, 116.25, 111.85, 110.72, 65.98. HRMS (ESI): m/z calcd for C₂₄H₂₀N₄O₄ [M+H]⁺ 429.1563; found 429.1562.

### (R)-7-hydroxy-12-((4-methoxy-[1,1'-biphenyl]-4-yl)methyl)-3,4,12,12a-tetrahydro-1H-[1,4]oxazino[3,4-c]pyrido[2,1-f][1,2,4]triazine-6,8-dione (Compound 31).

Light orange solid, m.p. 198-201 °C, yield 53.1%.

¹H NMR (400 MHz, DMSO-*d*₆) δ 11.81 (s, 1H, OH), 7.61 (dd, *J* = 12.3, 8.3 Hz, 4H, 4 × Ph -H), 7.31 (d, *J* = 8.0 Hz, 2H, 2 × Ph -H), 7.12 (d, *J* = 7.6 Hz, 1H, CH=CH), 7.02 (d, *J* = 8.7 Hz, 2H, 2 × Ph -H), 5.80 (d, *J* = 7.6 Hz, 1H, CH=CH), 5.05 (d, *J* = 9.4 Hz, 1H, CH), 4.30 (s, 3H, 3 × CH), 3.98 - 3.82 (m, 2H, CH₂), 3.80 (s, 3H, CH₃), 3.66 (t, *J* = 10.2 Hz, 1H, CH), 3.51 (t, *J* = 12.5 Hz, 1H, CH), 3.25 (td, *J* = 13.0, 12.2, 3.6 Hz, 1H, CH). ¹³C NMR (100 MHz, DMSO-*d*₆) δ 171.51, 159.51, 153.41, 140.11, 138.45, 133.50, 132.21, 130.90, 128.18, 126.56, 116.20, 114.85, 111.76, 65.98, 55.65, 55.39. HRMS (ESI): m/z calcd for C₂₄H₂₃N₃O₅ [M+H]⁺ 434.1716; found 434.1711.

### (R)-7-hydroxy-12-((4'-(trifluoromethoxy)-[1,1'-biphenyl]-4-yl)methyl)-3,4,12,12a-tetrahydro-1H-[1,4]oxazino[3,4-c]pyrido[2,1-f][1,2,4]triazine-6,8-dione (Compound 32).

Light orange solid, m.p. 219-221 °C, yield 39%.

¹H NMR (400 MHz, DMSO-*d*₆) δ 11.79 (s, 1H, OH), 7.81 (d, *J* = 7.9 Hz, 2H, 2 × Ph -H), 7.67 (d, *J* = 7.5 Hz, 2H, 2 × Ph-H), 7.45 (d, *J* = 8.2 Hz, 2H, 2 × Ph-H), 7.38 (d, *J* = 7.7 Hz, 2H, 2 × Ph-H), 7.13 (d, *J* = 7.0 Hz, 1H, CH=CH), 5.80 (d, *J* = 7.2 Hz, 1H, CH=CH), 5.07 (s, 1H, CH), 4.33 (s, 3H, 3 × CH), 3.89 (dd, *J* = 40.4, 9.9 Hz, 2H, CH₂), 3.68 (d, *J* = 10.2 Hz, 1H, CH), 3.52 (t, *J* = 9.4 Hz, 1H, CH), 3.26 (t, *J* = 10.8 Hz, 1H, CH). ¹³C NMR (100 MHz, DMSO-*d*₆) δ 171.51, 153.40, 148.42, 139.23, 138.98, 138.41, 134.90, 131.01, 129.00, 127.30, 121.94, 121.86, 119.31, 116.22, 111.78, 65.98. HRMS (ESI): m/z calcd for C₂₄H₂₀F₃N₃O₅ [M+H]⁺ 488.1433; found 488.1431.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carboxylic acid (Compound 33).

White solid, m.p. 283-285°C, yield 85.6%.

¹H NMR (400 MHz, DMSO-*d*₆) δ 8.02 (d, *J* = 8.1 Hz, 2H, 2×Ph -H), 7.82 (d, *J* = 8.1 Hz, 2H, 2×Ph -H), 7.73 (d, *J* = 7.9 Hz, 2H, 2×Ph -H), 7.40 (d, *J* = 7.9 Hz, 2H, 2×Ph -H), 7.13 (d, *J* = 7.7 Hz, 1H, CH=CH), 5.81 (d, *J* = 7.6 Hz, 1H, CH=CH), 5.07 (s, 1H, CH), 4.41 - 4.19 (m, 3H, 3× CH), 3.99 - 3.78 (m, 2H, 2× CH), 3.68 (s, 2H, 2× CH), 3.17 (s, 1H, CH). ¹³C NMR (100 MHz, DMSO-*d*₆) δ 171.53, 167.59, 153.39, 144.00, 139.29, 138.44, 135.32, 131.05, 130.45, 130.30, 127.46, 127.20, 116.24, 111.79, 65.97. HRMS (ESI): m/z calcd for C₂₄H₂₁N₃O₆ [M+H]⁺ 448.1508; found 448.1508.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carboxamide (Compound 34).

Light orange solid, m.p. 236-239 °C, yield 54.4%.

¹H NMR (400 MHz, DMSO-*d*₆) δ 11.81 (s, 1H, OH), 8.03 (s, 1H, Ph-H), 7.97 (d, *J* = 8.0 Hz, 2H, 2 × Ph-H), 7.77 (d, *J* = 8.0 Hz, 2H, 2 × Ph-H), 7.72 (d, *J* = 7.8 Hz, 2H, NH₂), 7.39 (d, *J* = 7.1 Hz, 3H, 3 × Ph-H), 7.13 (d, *J* = 7.0 Hz, 1H, CH=CH), 5.81 (d, *J* = 7.5 Hz, 1H, CH=CH), 5.07 (s, 1H, CH), 4.42 - 4.21 (m, 3H, 3 × CH), 3.89 (dd, *J* = 40.0, 10.0 Hz, 2H, CH₂), 3.68 (d, *J* = 10.5 Hz, 1H, CH), 3.58 - 3.46 (m, 1H, CH), 3.25 (t, *J* = 10.9 Hz, 1H, CH). ¹³C NMR (100 MHz, DMSO-*d*₆) δ 171.55, 167.96, 153.45, 142.45, 139.46, 138.43, 135.03, 133.69, 131.00, 128.67, 127.35, 126.83, 116.22, 111.78, 65.98, 60.23, 21.23, 14.55. HRMS (ESI): m/z calcd for C₂₄H₂₂N₄O₅ [M+H]⁺ 447.1668; found 447.1669.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-N-methyl-[1,1'-biphenyl]-4-carboxamide (Compound 35).

Red brown solid, m.p.242-245 °C, yield 68.9%.

¹H NMR (400 MHz, DMSO-*d*₆) δ 8.57 - 8.49 (m, 1H, CONH), 7.93 (q, *J* = 8.6 Hz, 3H, 3 × Ph-H), 7.78 (d, *J* = 8.2 Hz, 2H, 2 × Ph-H), 7.72 (d, *J* = 8.2 Hz, 2H, 2 × Ph-H), 7.38 (d, *J* = 7.7 Hz, 2H, Ph-H, CH=CH), 5.77 (s, 1H, CH=CH), 5.04 (s, 1H, CH), 4.34 (s, 3H, 3 × CH), 3.99 - 3.78 (m, 2H, CH₂), 3.67 (s, 2H, 2 × CH), 3.17 (s, 1H, CH), 2.80 (d, *J* = 4.3 Hz, 3H, CONCH₃). ¹³C NMR (100MHz, DMSO-*d*₆) δ 166.69, 142.29, 139.36, 134.26, 133.90, 130.89, 128.25, 128.22, 127.32, 127.16, 126.89, 66.00, 26.74. HRMS (ESI): m/z calcd for C₂₅H₂₄N₄O₅ [M+H]⁺ 461.1825; found 461.1828.

### (R)-7-hydroxy-12-((4'-(methylsulphonyl)-[1,1-biphenyl]-4-yl)methyl)-3,4,12,12a-tetrahydro-1H-[1,4]oxazino[3,4-c]pyrido[2,1-f][1,2,4]triazine-6,8-dione (Compound 36).

Light orange solid, m.p. 215-218 °C, yield 43%.

¹H NMR (400 MHz, DMSO-*d*₆) δ 11.75 (s, 1H, OH), 8.02 - 7.93 (m, 4H, 4 × Ph -H), 7.74 (d, *J* = 7.8 Hz, 2H, 2 × Ph-H), 7.43 (d, *J* = 7.9 Hz, 2H, 2 × Ph-H), 7.14 (d, *J* = 7.6 Hz, 1H, CH=CH), 5.81 (d, *J* = 7.6 Hz, 1H, CH=CH), 5.07 (d, *J* = 8.3 Hz, 1H, CH), 4.43 - 4.24 (m, 3H, 3 × CH), 3.89 (dd, *J* = 41.8, 11.1 Hz, 2H, CH₂), 3.67 (t, *J* = 10.5 Hz, 1H, CH), 3.52 (t, *J* = 10.5 Hz, 1H, CH), 3.29 (d, *J* = 3.7 Hz, 1H, CH), 3.25 (s, 3H, CH₃). ¹³C NMR (100 MHz, DMSO-*d*₆) δ 171.52, 153.40, 144.83, 140.17, 138.64, 138.41, 135.81, 131.12, 128.13, 127.99, 127.70, 116.24, 111.81, 65.98, 55.40, 44.05. HRMS (ESI): m/z calcd for C₂₄H₂₃N₃O₆S [M+H]⁺ 482.1386; found 482.1386.

### (R)-N-cyclopentyl-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carboxamide (Compound 37).

Yellow solid, m.p. 165-167°C, yield 54.3%.

¹H NMR (400 MHz, DMSO-*d*₆) δ 8.36 (d, *J* = 7.3 Hz, 1H, CONH), 7.94 (d, *J* = 8.1 Hz, 2H, 2 × Ph-H), 7.77 (d, *J* = 8.1 Hz, 2H, 2 × Ph-H), 7.72 (d, *J* = 8.0 Hz, 2H, 2 × Ph-H), 7.39 (d, *J* = 7.9 Hz, 2H, 2 × Ph-H), 7.13 (d, *J* = 7.6 Hz, 1H, CH=CH), 5.81 (d, *J* = 7.6 Hz, 1H, CH=CH), 5.07 (s, 1H, CH), 4.41 - 4.27 (m, 3H, 3 × CH), 4.28 - 4.22 (m, 1H, CH), 3.97 - 3.81 (m, 2H, CH₂), 3.68 (s, 1H, CH), 3.52 (s, 1H, CH), 3.27 - 3.21 (m, 1H, CH), 1.94 - 1.86 (m, 2H, CH₂), 1.74 - 1.67 (m, 2H, CH₂), 1.58 - 1.51 (m, 4H, 2 × CH₂). ¹³C NMR (100 MHz, DMSO-*d*₆) δ 171.52, 165.92, 153.41, 142.15, 139.46, 138.47, 134.99, 134.17, 131.03, 128.48, 127.32, 126.73, 116.23, 111.77, 65.98, 51.42, 32.60, 24.14. HRMS (ESI): m/z calcd for C₂₉H₃₀N₄O₅ [M+H]⁺ 515.2294; found 515.2297.

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carboxamide (Compound 38)

Light orange solid, m.p. 201-204 °C, yield 48%.

¹H NMR (400 MHz, DMSO-*d*₆) δ 11.57 (s, 1H, OH), 8.16 (d, *J =* 19.7 Hz, 2H, 2 × Ph-H), 7.85 (dd, *J =* 14.2, 7.7 Hz, 2H, 2 × Ph-H), 7.72 (d, *J* = 7.7 Hz, 2H, NH2), 7.55 (t, *J* = 7.8 Hz, 1H, Ph-H), 7.48 - 7.35 (m, 3H, 3 × Ph -H), 7.12 (d, *J* = 7.6 Hz, 1H, CH=CH), 5.80 (d, *J* = 7.3 Hz, 1H, CH=CH), 5.07 (s, 1H, CH), 4.33 (s, 3H, 3 × CH), 3.89 (dd, *J* = 41.2, 9.7 Hz, 2H, CH₂), 3.68 (d, *J* = 10.4 Hz, 1H, CH), 3.50 (d, *J* = 11.4 Hz, 1H, CH), 3.24 (d, *J* = 13.0 Hz, 1H, CH). ¹³C NMR (150 MHz, DMSO-*d*₆) δ 171.54, 168.19, 162.76, 153.42, 139.84, 138.41, 135.42, 134.68, 130.96, 130.10, 129.77, 129.42, 127.28, 127.24, 126.02, 116.20, 111.74, 65.96, 55.35, 36.23. ESI-MS: m/z 447.36 [M+1]⁺. C₂₄H₂₂N₄O₅ (446.16).

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carboxylic acid (Compound 39)

Light orange solid, m.p. 290-293 °C, yield 52.4%.

¹H NMR (400 MHz, DMSO-*d*₆) δ 12.82 (s, 1H, COOH), 12.12 (s, 1H, OH), 8.19 (s, 1H, Ph-H), 7.94 (t, *J* = 8.7 Hz, 2H, 2 × Ph-H), 7.68 (d, *J* = 7.8 Hz, 2H, 2 × Ph-H), 7.60 (t, *J* = 7.7 Hz, 1H, Ph-H), 7.39 (d, *J* = 7.7 Hz, 2H, 2 × Ph -H), 7.13 (d, *J* = 7.6 Hz, 1H, CH=CH), 5.81 (d, *J* = 7.6 Hz, 1H, CH=CH), 5.08 (d, *J* = 7.0 Hz, 1H, CH), 4.36 (d, *J* = 12.8 Hz, 3H, 3 × CH), 3.90 (dd, *J* = 42.4, 11.1 Hz, 2H, CH₂), 3.69 (d, *J* = 10.3 Hz, 1H, CH), 3.58 - 3.46 (m, 1H, CH), 3.27 (d, *J* = 10.4 Hz, 1H, CH). ¹³C NMR (100 MHz, DMSO-*d*₆) δ 171.52, 167.70, 153.42, 140.28, 139.61, 138.43, 134.88, 132.20, 131.43, 131.08, 129.81, 128.90, 127.74, 127.30, 116.22, 111.77, 65.98. ESI-MS: m/z 448.36 [M+1]⁺. C₂₄H₂₁N₃O₆ (447.14).

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-sulphonamide (Compound 40)

Light orange solid, m.p. 278-281 °C, yield 48%.

¹H NMR (400 MHz, DMSO-*d*₆) δ 11.75 (s, 1H, OH), 8.13 (s, 1H, Ph-H), 7.91 (d, *J* = 7.9 Hz, 1H, NH), 7.82 (d, *J* = 7.8 Hz, 1H, NH), 7.68 (t, *J* = 7.9 Hz, 3H, 3 × Ph-H), 7.42 (d, *J* = 7.9 Hz, 4H, 4 × Ph-H), 7.12 (d, *J =* 7.3 Hz, 1H, CH=CH), 5.80 (d, *J* = 7.6 Hz, 1H, CH=CH), 5.09 (s, 1H, CH), 4.33 (d, *J* = 21.2 Hz, 3H, 3 × CH), 3.90 (dd, *J* = 42.5, 9.8 Hz, 2H, CH₂), 3.69 (d, *J* = 10.7 Hz, 1H, CH), 3.54 (d, *J* = 12.2 Hz, 1H, CH), 3.27 (d, *J* = 12.7 Hz, 1H, CH). ¹³C NMR (100 MHz, DMSO-*d*₆) δ 171.52, 153.39, 145.35, 140.60, 139.02, 138.45, 135.24, 131.20, 130.34, 130.19, 127.26, 125.05, 124.14, 116.25, 111.75, 65.98. ESI-MS: m/z 483.28 [M+1]⁺. C₂₃H₂₂N₄O₆S (482.13).

### (R)-7-hydroxy-12-((2'-nitro-[1,1'-biphenyl]-4-yl)methyl)-3,4,12,12a-tetrahydro-1H-[1,4]oxazino[3,4-c]pyrido[2,1-f][1,2,4]triazine-6,8-dione (Compound 41)

Yellow solid, m.p. 211-214 °C, yield 47.8%.

¹H NMR (400 MHz, DMSO-*d*₆) δ 11.61 (s, 1H, OH), 7.99 (d, *J* = 8.1 Hz, 1H, Ph-H), 7.78 (t, *J* = 7.6 Hz, 1H, Ph-H), 7.64 (t, *J* = 7.8 Hz, 1H, Ph-H), 7.54 (d, *J* = 6.3 Hz, 1H, Ph-H), 7.41 - 7.27 (m, 4H, 4 × Ph-H), 7.06 (d, *J* = 7.7 Hz, 1H, CH=CH), 5.79 (d, *J* = 7.7 Hz, 1H, CH=CH), 5.06 (d, *J* = 9.8 Hz, 1H, CH), 4.32 (d, *J =* 19.8 Hz, 3H, 3 × CH), 3.89 (dd, *J* = 39.0, 90 Hz, 2H, CH₂), 3.66 (t, *J* = 10.6 Hz, 1H, CH), 3.51 (d, *J* = 11.9 Hz, 1H, CH), 3.30 - 3.21 (m, 1H, CH). ¹³C NMR (100 MHz, DMSO-*d*₆) δ 171.49, 153.40, 149.34, 138.26, 137.53, 135.36, 135.16, 133.43, 132.26, 130.64, 129.47, 128.43, 124.60, 116.21, 111.72, 65.98. ESI-MS: m/z 449.36 [M+1]⁺. C₂₃H₂₀N₄O₆ (448.14).

### (R)-7-hydroxy-12-((3'-(methylsulphonyl)-[1,1-biphenyl]-4-yl)methyl)-3,4,12,12a-tetrahydro-1H-[1,4]oxazino[3,4-c]pyrido[2,1-f][1,2,4]triazine-6,8-dione (Compound 42)

Light orange solid, m.p. 234-237 °C, yield 46%.

¹H NMR (400 MHz, DMSO-*d*₆) δ 11.77 (s, 1H, OH), 8.17 (s, 1H, Ph-H), 8.05 (d, *J* = 7.8 Hz, 1H, Ph-H), 7.93 (d, *J =* 7.7 Hz, 1H, Ph-H), 7.76 (d, *J =* 7.9 Hz, 3H, 3 × Ph-H), 7.42 (d, *J =* 7.9 Hz, 2H, 2 × Ph-H), 7.13 (d, *J* = 7.6 Hz, 1H, CH=CH), 5.80 (d, *J* = 7.7 Hz, 1H, CH=CH), 5.07 (d, *J* = 11.9 Hz, 1H, CH), 4.36 (t, *J* = 14.1 Hz, 3H, 3 × CH), 3.90 (dd, *J* = 42.2, 10.1 Hz, 2H, CH₂), 3.69 (d, *J* = 10.7 Hz, 1H, CH), 3.51 (d, *J* = 11.7 Hz, 1H, CH), 3.30 (s, 3H, CH₃), 3.29 - 3.21 (m, 1H, CH). ¹³C NMR (100 MHz, DMSO-*d*₆) δ 171.52, 153.40, 142.15, 141.08, 138.69, 138.43, 135.42, 132.13, 131.15, 130.62, 127.52, 126.38, 125.36, 116.24, 111.77, 65.98, 43.91. ESI-MS: m/z 482.30 [M+1]⁺. C₂₄H₂₃N₃O₆S (481.13).

### (R)-3-fluoro-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carbaldehyde (Compound 43)

Light orange solid, m.p. 259-262 °C, yield 50.8%.

¹H NMR (400 MHz, DMSO-*d*₆) δ 11.81 (s, 1H, OH), 10.25 (s, 1H, CHO), 7.92 (t, *J* = 7.7 Hz, 1H, Ph-H), 7.84 - 7.75 (m, 4H, 4 × Ph-H), 7.43 (d, *J* = 7.9 Hz, 2H, 2 × Ph-H), 7.13 (d, *J* = 7.6 Hz, 1H, CH=CH), 5.81 (d, *J* = 7.6 Hz, 1H, CH=CH), 5.06 (d, *J* = 8.0 Hz, 1H, CH), 4.35 (t, *J* = 18.6 Hz, 3H, 3 × CH), 3.89 (dd, *J =* 41.5, 9.8 Hz, 2H, CH₂), 3.68 (d, *J* = 9.9 Hz, 1H, CH), 3.52 (s, 1H, CH), 3.24 (d, *J* = 12.1 Hz, 1H, CH). ¹³C NMR (100 MHz, DMSO-*d*₆) δ 187.97, 187.93, 171.53, 165.52, 162.97, 153.41, 148.08, 147.99, 138.40, 137.66, 136.48, 131.11, 130.49, 130.46, 127.72, 123.56, 123.53, 123.11, 123.03, 116.24, 115.04, 114.82, 111.82, 65.97. ESI-MS: m/z 450.29 [M+1]⁺. C₂₄H₂₀FN₃O₅ (449.14).

### (R)-12-((3'-fluoro-[1,1-biphenyl]-4-yl)methyl)-7-hydroxy-3,4,12,12a-tetrahydro-1H-[1,4]oxazino[3,4-c]pyrido[2,1-f][1,2,4]triazine-6,8-dione (Compound 44)

Light orange solid, m.p. 240-243 °C, yield 56%.

¹H NMR (400 MHz, DMSO-*d*₆) δ 11.75 (s, 1H, OH), 7.69 (d, *J* = 7.8 Hz, 2H, 2 × Ph-H), 7.57 - 7.49 (m, 3H, 3 × Ph-H), 7.38 (d, *J* = 7.9 Hz, 2H, 2 × Ph-H), 7.20 (t, *J* = 8.2 Hz, 1H, Ph-H), 7.13 (d, *J* = 7.5 Hz, 1H, CH=CH), 5.81 (d, *J* = 7.6 Hz, 1H, CH=CH), 5.06 (d, *J* = 6.7 Hz, 1H, CH), 4.35 (d, *J* = 13.0 Hz, 3H, 3 × CH), 3.89 (dd, *J* = 40.3, 9.0 Hz, 2H, CH₂), 3.67 (t, *J* = 10.4 Hz, 1H, CH), 3.53 (d, *J* = 10.3 Hz, 1H, CH), 3.30 - 3.20 (m, 1H, CH). ¹³C NMR (150 MHz, DMSO-*d*₆) δ 171.49, 163.98, 162.37, 153.38, 142.37, 142.32, 138.99, 138.38, 135.08, 131.96, 131.90, 131.37, 131.31, 130.94, 129.24, 129.16, 127.27, 123.12, 123.10, 116.20, 114.85, 114.71, 113.82, 113.67, 111.78, 65.95, 55.24. ESI-MS: m/z 422.27 [M+1]⁺. C₂₃H₂₀FN₃O₄ (421.14).

### (R)-7-hydroxy-12-((3'-methoxy-[1,1'-biphenyl]-4-yl)methyl)-3,4,12,12a-tetrahydro-1H-[1,4]oxazino[3,4-c]pyrido[2,1-f][1,2,4]triazine-6,8-dione (Compound 45)

Light orange solid, m.p. 231-234 °C, yield 49%.

¹H NMR (400 MHz, DMSO-*d*₆) δ 11.48 (s, 1H, OH), 7.65 (d, *J* = 7.9 Hz, 2H, 2 × Ph-H), 7.36 (dd, *J* = 7.9, 5.1 Hz, 3H, 3 × Ph-H), 7.27 - 7.17 (m, 2H, 2 × Ph-H), 7.13 (d, *J* = 7.6 Hz, 1H, Ph-H), 6.94 (dd, *J* = 8.2, 2.5 Hz, 1H, CH=CH), 5.81 (d, *J* = 7.6 Hz, 1H, CH=CH), 5.05 (d, *J* = 9.6 Hz, 1H, CH), 4.32 (s, 3H, 3 × CH), 3.93 (d, *J* = 9.2 Hz, 2H, CH₂), 3.82 (s, 3H, CH₃), 3.68 (d, *J* = 10.5 Hz, 1H, CH), 3.50 (d, *J* = 11.8 Hz, 1H, CH), 3.31 - 3.20 (m, 1H, CH). ¹³C NMR (100 MHz, DMSO-*d*₆) δ 171.52, 160.24, 153.42, 141.40, 140.32, 138.45, 134.52, 130.87, 130.50, 127.26, 119.41, 116.22, 113.74, 112.52, 111.81, 65.99, 55.61. ESI-MS: m/z 434.01 [M+1]⁺. C₂₄H₂₃N₃O₅ (433.16).

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-3-carbaldehyde (Compound 46)

Light orange solid, m.p. 221-224 °C, yield 47%.

¹H NMR (400 MHz, DMSO-*d*₆) δ 11.83 (s, 1H, OH), 10.11 (s, 1H, CHO), 8.22 (s, 1H, Ph-H), 8.04 (d, *J =* 7.8 Hz, 1H, Ph-H), 7.91 (d, *J* = 7.5 Hz, 1H, Ph-H), 7.72 (dd, *J* = 19.5, 7.7 Hz, 3H, 3 × Ph-H), 7.41 (d, *J =* 7.8 Hz, 2H, 2 × Ph-H), 7.13 (d, *J* = 7.6 Hz, 1H, CH=CH), 5.81 (d, *J* = 7.5 Hz, 1H, CH=CH), 5.08 (d, *J* = 8.1 Hz, 1H, CH), 4.37 (d, *J* = 12.7 Hz, 3H, 3 × CH), 4.01 - 3.80 (m, 2H, CH₂), 3.68 (t, *J* = 10.4 Hz, 1H, CH), 3.58 - 3.47 (m, 1H, CH), 3.30 - 3.21 (m, 1H, CH). ¹³C NMR (100 MHz, DMSO-*d*₆) δ 193.71, 171.52, 153.42, 140.74, 139.14, 138.43, 137.33, 135.11, 133.02, 131.12, 130.37, 128.60, 128.49, 127.32, 116.23, 111.77, 65.98. ESI-MS: m/z 431.98 [M+1]⁺. C₂₄H₂₁N₃O₅ (431.15).

### (R)-3-fluoro-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-4-carbonitrile (Compound 47)

Light orange solid, m.p. 236-239 °C, yield 49%.

¹H NMR (400 MHz, DMSO-*d*₆) δ 11.61 (s, 1H, OH), 8.00 (t, *J* = 7.5 Hz, 1H, Ph-H), 7.92 (d, *J* = 11.1 Hz, 1H, Ph-H), 7.80 (d, *J* = 8.3 Hz, 3H, 3 × Ph-H), 7.44 (d, *J* = 7.7 Hz, 2H, 2 × Ph-H), 7.13 (d, *J* = 7.6 Hz, 1H, CH=CH), 5.81 (d, *J* = 7.5 Hz, 1H, CH=CH), 5.07 (s, 1H, CH), 4.44 - 4.22 (m, 3H, 3 × CH), 3.89 (dd, *J =* 40.5, 11.2 Hz, 2H, CH₂), 3.67 (t, *J* = 10.5 Hz, 1H, CH), 3.54 (d, *J* = 11.8 Hz, 1H, CH), 3.27 (d, *J* = 10.7 Hz, 1H, CH). ¹³C NMR (100 MHz, DMSO-*d*₆) δ 171.52, 164.70, 162.16, 153.41, 147.45, 147.37, 138.38, 137.18, 136.71, 134.79, 131.12, 127.77, 123.97, 123.94, 116.22, 114.86, 114.65, 114.60, 111.83, 99.31, 99.16, 65.96. ESI-MS: m/z 447.03 [M+1]⁺. C₂₄H₁₉FN₄O₄ (446.14).

### (R)-4'-((7-hydroxy-6,8-dioxo-1,3,4,6,8,12a-hexahydro-12H-[1,4]oxazino[3,4-c]pyrido[2,1-f][1,2,4]triazin-12-yl)methyl)-[1,1'-biphenyl]-2-carbaldehyde (Compound 48)

Light orange solid, m.p. 234-237 °C, yield 45.8%.

¹H NMR (400 MHz, DMSO-*d*₆) δ 11.75 (s, 1H, OH), 9.85 (s, 1H, CHO), 7.93 (d, *J =* 7.8 Hz, 1H, Ph-H), 7.76 (t, *J* = 6.8 Hz, 1H, Ph-H), 7.60 (t, *J* = 7.5 Hz, 1H, Ph-H), 7.50 (d, *J* = 8.1 Hz, 1H, Ph-H), 7.40 (s, 4H, Ph-H), 7.15 (d, *J* = 7.6 Hz, 1H, CH=CH), 5.81 (d, *J* = 7.6 Hz, 1H, CH=CH), 5.11 (d, *J* = 8.1 Hz, 1H, CH), 4.45 - 4.26 (m, 3H, 3 × CH), 3.99 - 3.81 (m, 2H, CH₂), 3.66 (d, *J* = 15.7 Hz, 1H, CH), 3.54 (d, *J* = 16.1 Hz, 1H, CH), 3.29 - 3.20 (m, 1H, CH). ¹³C NMR (100 MHz, DMSO-*d*₆) δ 192.17, 171.52, 153.34, 145.09, 138.44, 137.83, 135.10, 134.42, 133.65, 131.32, 130.59, 130.50, 128.60, 127.85, 116.34, 111.71, 65.98. ESI-MS: m/z 432.10 [M+1]⁺. C₂₄H₂₁N₃O₆ (431.15).

### (R)-12-((3',4'-dimethoxy-[1,1'-biphenyl]-4-yl)methyl)-7-hydroxy-3,4,12,12a-tetrahydro-1H-[1,4]oxazino[3,4-c]pyrido[2,1-f][1,2,4]triazine-6,8-dione (Compound 49)

Light orange solid, m.p. 213-216 °C, yield 43.9%.

¹H NMR (400 MHz, DMSO-*d*₆) δ 11.79 (s, 1H, OH), 7.63 (d, *J* = 7.9 Hz, 2H, 2 × Ph-H), 7.31 (d, *J* = 7.9 Hz, 2H, 2 × Ph-H), 7.22 (d, *J* = 8.4 Hz, 2H, 2 × Ph-H), 7.13 (d, *J* = 7.6 Hz, 1H, Ph-H), 7.03 (d, *J* = 8.1 Hz, 1H, CH=CH), 5.81 (d, *J* = 7.6 Hz, 1H, CH=CH), 5.05 (s, 1H, CH), 4.30 (s, 3H, 3 × CH), 3.98 - 3.85 (m, 2H, CH₂), 3.84 (s, 3H, CH₃), 3.79 (s, 3H, CH₃), 3.72 - 3.61 (m, 1H, CH), 3.57 - 3.47 (m, 1H, CH), 3.29 - 3.20 (m, 1H, CH). ¹³C NMR (100 MHz, DMSO-*d*₆) δ 171.51, 153.41, 149.54, 149.13, 140.37, 138.47, 133.56, 132.55, 130.84, 126.76, 119.28, 116.20, 112.61, 111.78, 110.70, 65.99, 56.05, 56.03, 55.39. ESI-MS: m/z 463.99 [M+1]⁺. C₂₅H₂₅N₃O₆ (463.17).

### EXAMPLE 8: Synthesis of Intermediates 50c and Compounds 50 to 68. Compound 50

### (R)-7-(benzyloxy)-12-(4-(phenylthio)benzyl)-3,4,12,12a-tetrahydro-1H-[1,4]oxazino[3,4-c]pyrido[2,1-f][1,2,4]triazine-6,8-dione (Intermediate 50c)

**Intermediate 5b** (543 mg, 1 mmol, 1 eq), thiophenol (220 mg, 2 mmol, 2 eq), Cul (19 mg, 0.1 mmol, 0.1 eq), 1,10-phenanthroline (36 mg, 0.2 mmol, 0.2 eq), and K₂CO₃ (276 mg, 2 mmol, 2 eq) were charged into a 50 mL pear-shaped flask. 20 mL of 1,4-dioxane were added as the solvent. The Ullmann reaction was performed under a nitrogen atmosphere by refluxing at 100°C overnight. After 12 hours, the reaction completion was monitored by TLC. The reaction mixture was filtered, and concentrated under reduced pressure to remove 1,4-dioxane. The residue was diluted with water and extracted with ethyl acetate. The organic layer was washed with saturated NaCl solution, dried over anhydrous Na₂SO₄, and concentrated under reduced pressure. The crude product was purified by column chromatography using 200-300 mesh silica gel to obtain the pale yellow powdered **Intermediate 50c** with a yield of 23.4%. ESI-MS: m/z 526.24 [M+1]⁺. C₃₀H₂₇N₃O₄S (525.17).

### (R)-7-hydroxy-12-(4-(phenylthio)benzyl)-3,4,12,12a-tetrahydro-1H-[1,4]oxazino[3,4-c]pyrido[2,1-f][1,2,4]triazine-6,8-dione (Compound 50)

**Intermediate 50c** was subjected to benzyl deprotection with LiCI in DMF solvent, and the target product **Compound 50** was obtained by HPLC separation. Brown solid, m.p. 210-213°C, yield 37.8%.

¹H NMR (400 MHz, DMSO-*d*₆) δ 7.69 (d, *J =* 7.6 Hz, 3H, 3 × Ph-H), 7.45 - 7.20 (m, 4H, 4 × Ph-H), 7.08 (d, *J* = 7.8 Hz, 3H, 2 × Ph-H, CH=CH), 5.78 (s, 1H, CH=CH), 5.04 (s, 1H, CH), 4.26 (s, 3H, 3 × CH), 3.96 - 3.79 (m, 3H, 3 × CH), 3.64 (s, 1H, CH), 3.17 (s, 1H, CH). ESI-MS: m/z 434.26 [M-1]⁻. C₂₃H₂₁N₃O₄S (435.15).

### Compound 51

### (R)-7-(benzyloxy)-12-(4-((4-(trifluoromethyl)phenyl)thio)benzyl)-3,4,12,12a-tetrahydro-1H-[1.4]oxazino[3,4-c]pyrido[2,1-f][1,2,4]triazine-6,8-dione (Intermediate 51c)

**Intermediate 5b** (543 mg, 1 mmol, 1 eq), 4-(Trifluoromethyl)benzenethiol (354 mg, 2 mmol, 2 eq), Cul (19 mg, 0.1 mmol, 0.1 eq), 1,10-phenanthroline (36 mg, 0.2 mmol, 0.2 eq), and K₂CO₃ (276 mg, 2 mmol, 2 eq) were charged into a 50 mL pear-shaped flask. 20 mL of 1,4-dioxane were added as the solvent. The Ullmann reaction was performed under a nitrogen atmosphere by refluxing at 100°C overnight. The subsequent procedure was identical to that of **Intermediate 50c**, yielding a pale yellow solid **Intermediate 51c** with a yield of 24.5%. ESI-MS: m/z 594.28 [M+1]⁺. C₃₁H₂₆F₃N₃O₄S (593.62).

### (R)-7-hydroxy-12-(4-((4-(trifluoromethyl)phenyl)thio)benzyl)-3,4,12,12a-tetrahydro-1H-[1,4]oxazino[3,4-c]pyrido[2,1-f][1,2,4]triazine-6,8-dione (Compound 51)

**Intermediate 51c** was subjected to benzyl deprotection with LiCI in DMF solvent, and the target product **Compound 51** was obtained by HPLC separation. Yellow solid, m.p. 241-243°C, yield 42.5%.

¹H NMR (400 MHz, DMSO-*d*₆) δ 7.68 (d, *J* = 8.2 Hz, 2H, 2 × Ph-H), 7.46 (d, *J* = 7.9 Hz, 2H, 2 × Ph-H), 7.36 (d, *J* = 8.0 Hz, 2H, 2 × Ph-H), 7.29 (d, *J* = 8.1 Hz, 2H, 2 × Ph-H), 7.10 (s, 1H, CH=CH), 5.79 (s, 1H, CH=CH), 5.09 (s, 1H, CH), 4.34 (s, 3H, 3 × CH), 4.05 - 3.73 (m, 3H, 3 × CH), 3.64 (s, 1H, CH), 3.17 (s, 1H, CH). ESI-MS: m/z 526.20 [M+Na]⁺. C₂₄H₂₀F₃N₃O₄S (503.11)

### Compound 52

### (R)-12-(4-((2-aminophenyl)thio)benzyl)-7-(benzyloxy)-3,4,12,12a-tetrahydro-1H-[1,4]oxazino[3,4-c]pyrido[2,1-f][1,2,4]triazine-6,8-dione (Intermediate 52c)

**Intermediate 5b** (543 mg, 1 mmol, 1 eq), 2-aminobenzenethiol (250 mg, 2 mmol, 2 eq), Cul (19 mg, 0.1 mmol, 0.1 eq), 1,10-phenanthroline (36 mg, 0.2 mmol, 0.2 eq), and K₂CO₃ (276 mg, 2 mmol, 2 eq) were charged into a 50 mL pear-shaped flask. 20 mL of 1,4-dioxane as the solvent were added. The Ullmann reaction was performed under a nitrogen atmosphere by refluxing at 100°C overnight. The subsequent procedure was identical to that of **Intermediate 50c**, yielding a pale yellow solid **Intermediate 52c** with a yield of 25.8%. ESI-MS: m/z 541.64 [M+1]⁺. C₃₀H₂₈N₄O₄S (540.18).

### (R)-12-(4-((2-aminophenyl)thio)benzyl)-7-hydroxy-3,4,12,12a-tetrahydro-1H-[1,4]oxazino[3,4-c]pyrido[2,1-f][1,2,4]triazine-6,8-dione (Compound 52)

**Intermediate 52c** was subjected to benzyl deprotection with LiCI in DMF solvent, and the target product **Compound 52** was obtained by HPLC separation. Yellow solid, m.p. 242-243°C, yield 51.6%.

¹H NMR (400 MHz, DMSO-*d*₆) δ 7.29 (d, *J* = 7.7 Hz, 1H, Ph-H), 7.24 - 7.13 (m, 3H, 3 × Ph-H), 7.10 - 7.03 (m, 1H, CH= CH), 6.99 (d, *J* = 7.9 Hz, 2H, 2 × Ph-H), 6.81 (d, *J* = 8.1 Hz, 1H, Ph-H), 6.60 (t, *J* = 7.5 Hz, 1H, Ph-H), 5.78 (d, *J* = 11.3 Hz, 1H, CH= CH), 5.37 (s, 2H, NH₂), 4.98 (s, 1H, CH), 4.20 (s, 3H, 3 × CH), 3.87 (s, 2H, 2 × CH), 3.61 (s, 1H, CH), 3.45 (s, 2H, 2 × CH). ESI-MS: m/z 451.42 [M+1]⁺. C₂₃H₂₂N₄O₄S (450.51).

### Compound 53

### (R)-12-(4-((3-aminophenyl)thio)benzyl)-7-(benzyloxy)-3,4,12,12a-tetrahydro-1H-[1,4]oxazino[3,4-c]pyrido[2,1-f][1,2,4]triazine-6,8-dione (Intermediate 53c)

**Intermediate 5b** (543 mg, 1 mmol, 1 eq), 3-aminobenzenethiol (250 mg, 2 mmol, 2 eq), Cul (19 mg, 0.1 mmol, 0.1 eq), 1,10-phenanthroline (36 mg, 0.2 mmol, 0.2 eq), and K₂CO₃ (276 mg, 2 mmol, 2 eq) were charged into a 50 mL pear-shaped flask. 20 mL of 1,4-dioxane as the solvent were added. The Ullmann reaction was performed under a nitrogen atmosphere by refluxing at 100°C overnight. The subsequent procedure was identical to that of Intermediate 50c, yielding a pale yellow solid **Intermediate 53c** with a yield of 23.6%. ESI-MS: m/z 541.28 [M+1]+. C₃₀H₂₈N₄O₄S (540.18).

### (R)-12-(4-((3-aminophenyl)thio)benzyl)-7-hydroxy-3,4,12,12a-tetrahydro-1H-[1,4]oxazino[3,4-c]pyrido[2,1-f][1,2,4]triazine-6,8-dione (Compound 53)

**Intermediate 53c** was subjected to benzyl deprotection with LiCI in DMF solvent, and the target product **Compound 53** was obtained by HPLC separation. Yellow solid, m.p. 243-244°C, yield 46.7%.

¹H NMR (400 MHz, DMSO-*d*₆) δ 7.37 - 7.26 (m, 2H, 2× Ph-H), 7.22 (m, 3H, 3 × Ph-H), 7.09 (m, 1H, 3 × Ph-H ), 7.00 (q, *J* = 10.1 Hz, 1H, CH=CH), 6.50 (d, *J* = 8.3 Hz, 1H, Ph-H), 6.44 (d, *J* = 7.6 Hz, 1H, Ph-H), 5.78 (d, *J* = 8.0 Hz, 1H, CH=CH), 5.31 (s, 2H, NH₂), 5.06 (s, 1H, CH), 4.41 - 4.16 (m, 3H, 3 × CH), 3.97 - 3.79 (m, 2H, 2 × CH), 3.65 (s, 1H, CH), 3.50 (s, 1H, CH), 3.25 (m, 1H, CH). ESI-MS: m/z 451.46 [M+1]⁺. C₂₃H₂₂N₄O₄S (450.51).

### Compound 54

### (R)-12-(4-((4-aminophenyl)thio)benzyl)-7-(benzyloxy)-3,4,12,12a-tetrahydro-1H-[1.4]oxazino[3,4-c]pyrido[2,1-f][1,2,4]triazine-6,8-dione (Intermediate 54c)

**Intermediate 5b** (543 mg, 1 mmol, 1 eq), 4-aminobenzenethiol (250 mg, 2 mmol, 2 eq), Cul (19 mg, 0.1 mmol, 0.1 eq), 1,10-phenanthroline (36 mg, 0.2 mmol, 0.2 eq), and K₂CO₃ (276 mg, 2 mmol, 2 eq) were charged into a 50 mL pear-shaped flask. 20 mL of 1,4-dioxane as the solvent were added. The Ullmann reaction was performed under a nitrogen atmosphere by refluxing at 100°C overnight. The subsequent procedure was identical to that of **Intermediate 50c**, yielding a pale yellow solid **Intermediate 54c** with a yield of 26.9%. ESI-MS: m/z 541.47 [M+1]⁺. C₃₀H₂₈N₄O₄S (540.18).

### (R)-12-(4-((4-aminophenyl)thio)benzyl)-7-hydroxy-3,4,12,12a-tetrahydro-1H-[1,4]oxazino[3,4-c]pyrido[2,1-f][1,2,4]triazine-6,8-dione (Compound 54)

**Intermediate 54c** was subjected to benzyl deprotection with LiCI in DMF solvent, and the target product **Compound 54** was obtained by HPLC separation. Yellow solid, m.p. 246-247°C, yield 43.4%

¹H NMR (400 MHz, DMSO-*d*₆) δ 7.19 - 7.03 (m, 5H,4 × Ph-H, CH = CH), 6.95 (d, *J* = 7.9 Hz, 2H, 2 × Ph-H), 6.63 (d, *J* = 8.1 Hz, 2H, 2 × Ph-H), 5.81 (d, *J* = 7.6 Hz, 1H, CH=CH), 5.56 (s, 2H, NH₂), 5.12 - 4.88 (m, 1H, CH), 4.37 - 4.10 (m, 3H, 3 × CH), 3.92 - 3.80 (m, 2H, 2 × CH), 3.62 (t, *J* = 10.6 Hz, 1H, CH), 3.53 - 3.44 (m, 1H, CH), 3.27 - 3.18 (m, 1H, CH). ESI-MS: m/z 451.23 [M+1]⁺. C₂₃H₂₂N₄O₄S (450.51).

### Compound 55

### (R)-7-(benzyloxy)-12-(4-phenoxybenzyl)-3,4,12,12a-tetrahydro-1H-[1,4]oxazino[3,4-c]pyrido[2,1-f][1,2,4]triazine-6,8-dione (Intermediate 55b)

**Intermediate 1a** (283 mg, 0.86 mmol, 1 eq) and 1-(Bromomethyl)-4-phenoxybenzene (250 mg, 0.95 mmol, 1.1 eq) were combined with Cs₂CO₃ (560 mg, 1.72 mmol, 2 eq) in 20 mL of DMF solvent and stirred at room temperature for 12 hours. The progress of the reaction was monitored by TLC. The solvent was then removed by vacuum concentration. The residue was added to a saturated sodium chloride solution and extracted with ethyl acetate (3 × 20 mL). The combined organic phase was dried over anhydrous sodium sulphate, filtered, and concentrated. Finally, the concentrated residue was purified by flash column chromatography (DCM/MeOH = 20:1) to obtain **Intermediate 55b** as a white powder. The yield was 91.2%. ESI-MS: m/z 510.28 [M+1]⁺. C₃₀H₂₇N₃O₅ (509.56).

### (R)-7-hydroxy-12-(4-phenoxybenzyl)-3,4,12,12a-tetrahydro-1H-[1,4]oxazino[3,4-c]pyrido[2,1-f][1,2,4]triazine-6,8-dione (Compound 55)

**Intermediate 55b** was subjected to benzyl deprotection with LiCl in DMF solvent, and the target product **Compound 55** was obtained by HPLC separation. Yellow solid, m.p. 253-254°C, yield 45.1%.

¹H NMR (400 MHz, DMSO-*d*₆) δ 7.40 (t, *J* = 7.7 Hz, 2H, 2 × Ph-H), 7.29 - 7.11 (m, 3H, 3× Ph-H), 7.09 - 6.91 (m, 4H, 4 × Ph-H), 6.91 - 6.56 (m, 1H, CH=CH), 5.85 - 5.26 (m, 1H, CH=CH), 5.16 - 4.59 (m, 1H, CH), 4.47 - 4.01 (m, 3H, 3 × CH), 4.01 - 3.60 (m, 3H, 3 × CH), 3.51 (s, 1H, CH), 3.18 (s, 1H, CH). ESI-MS: m/z 418.29 [M-1]⁻. C₂₃H₂₁N₃O₅ (419.15)

### Compound 56

### (R)-12-(4-benzoylbenzyl)-7-(benzyloxy)-3,4,12,12a-tetrahydro-1H-[1,4]oxazino[3,4-c]pyrido[2,1-f][1,2,4]triazine-6,8-dione (Intermediate 56b)

**Intermediate 1a** (500 mg, 1.53 mmol, 1 eq) and 4-(Bromomethyl)benzophenone (505 mg, 1.84 mmol, 1.2 eq) were combined with Cs₂CO₃ (1 g, 3.06 mmol, 2 eq) in 20 mL of DMF solvent and stirred at room temperature for 12 hours. The reaction was monitored and the work-up procedure was identical to that of **Intermediate 2b**, yielding a yellow **Intermediate 56b** with a yield of 88.6%. ESI-MS: m/z 522.20 [M+1]⁺. C₃₁H₂₇N₃O₅ (521.57).

### (R)-12-(4-benzoylbenzyl)-7-hydroxy-3,4,12,12a-tetrahydro-1H-[1,4]oxazino[3,4-c]pyrido[2,1-f][1,2,4]triazine-6,8-dione (Compound 56)

**Intermediate 56b** was subjected to benzyl deprotection with LiCI in DMF solvent, and the target product **Compound 56** was obtained by HPLC separation. Yellow solid, m.p. 238-240°C, yield 45.8%.

¹H NMR (400 MHz, DMSO-*d*₆) δ 7.76 - 7.65 (m, 5H, 5 × Ph-H), 7.58 (t, *J* = 7.5 Hz, 2H, 2 × Ph-H), 7.49 (d, *J* = 7.9 Hz, 2H, 2 × Ph-H), 7.16 (d, *J* = 7.7 Hz, 1H, CH=CH), 5.83 (d, *J* = 7.6 Hz, 1H, CH=CH), 5.10 (d, *J* = 9.4 Hz, 1H, CH), 4.49 - 4.23 (m, 3H, 3 × CH), 3.97 (dd, *J* = 11.1, 3.6 Hz, 1H, CH), 3.85 (d, *J* = 11.5 Hz, 1H, CH), 3.67 (t, *J* = 10.6 Hz, 1H, CH), 3.53 (s, 1H, CH), 3.29 (m, , 1H, CH).¹³C NMR (100 MHz, DMSO-*d*₆) δ 196.01, 171.54, 153.35, 139.99, 138.38, 137.42, 137.30, 133.30, 130.40, 130.23, 130.09, 129.09, 116.30, 111.84, 65.95. ESI-MS: m/z 432.24 [M+1]⁺. C₂₄H₂₁N₃O₆ (431.15)

### Compound 57

### (R)-12-(4-benzylbenzyl)-7-(benzyloxy)-3,4,12,12a-tetrahydro-1H-[1,4]oxazino[3,4-c]pyrido[2,1-f][1,2,4]triazine-6,8-dione (Intermediate 57b)

**Intermediate 1a** (500 mg, 1.53 mmol, 1 eq) and 1-Benzyl-4-(chloromethyl)benzene (399 mg, 1.84 mmol, 1.2 eq) were combined with Cs₂CO₃ (1 g, 3.06 mmol, 2 eq) in 20 mL of DMF solvent and stirred at room temperature for 12 hours. The reaction was monitored and the work-up procedure was identical to that of **Intermediate 2b**, yielding a white **Intermediate 57b** with a yield of 89.4%. ESI-MS: m/z 508.65 [M+1]⁺. C₃₁H₂₉N₃O₄ (507.59).

### (R)-12-(4-benzylbenzyl)-7-hydroxy-3,4,12,12a-tetrahydro-1H-[1,4]oxazino[3,4-c]pyrido[2,1-f][1,2,4]triazine-6,8-dione (Compound 57)

**Intermediate 57b** was subjected to benzyl deprotection with LiCl in DMF solvent, and the target product **Compound 57** was obtained by HPLC separation. Yellow solid, m.p.170-172 °C, yield 42.5%.

¹H NMR (400 MHz, DMSO-*d*₆) δ 7.68 - 7.54 (m, 1H, Ph-H), 7.40 (dd, *J* = 13.5, 6.4 Hz, 1H, Ph-H), 7.28 (d, *J* = 7.4 Hz, 1H, Ph-H), 7.19 (d, *J* = 7.1 Hz, 6H, 6 × Ph-H), 7.08 - 6.96 (m, 1H,CH=CH), 5.79 - 5.74 (m, 1H, CH=CH), 5.12 - 4.99 (m, 2H, CH, 2 × CH), 4.34 - 4.17 (m, 3H, 3 × CH), 3.96 - 3.79 (m, 4H, 4 × CH), 3.64 (t, *J* = 10.6 Hz, 1H, CH), 3.23 - 3.17 (m, 1H, CH). ¹³C NMR (100 MHz, DMSO-*d*₆) δ 171.45, 153.32, 141.88, 141.57, 138.39, 132.67, 132.00, 131.88, 130.49, 129.43, 129.11, 128.90, 128.88, 128.03, 127.23, 127.14, 126.45, 116.21, 115.30, 111.67, 69.71, 65.97. ESI-MS: m/z 418.24 [M+1]⁺. C₂₄H₂₃N₃O₄(417.17)

### Compound 58

### (R)-7-(benzyloxy)-12-(3-phenylpropanoyl)-3,4,12,12a-tetrahydro-1H-[1,4Joxazinof3,4-c]pyrido[2,1-f][1,2,4]triazine-6,8-dione (Intermediate 58b)

**Intermediate 1a** (500 mg, 1.53 mmol, 1 eq.) was dissolved in 20 mL of dichloromethane. Triethylamine (232 mg, 2.3 mmol, 1.5 eq.) was added, and the mixture was stirred at room temperature for 10 minutes. 3-Phenylpropanoyl chloride (310 mg, 1.84 mmol, 1.2 eq.) was then added dropwise under an ice bath. The reaction was continued at room temperature for 3 hours, and the progress was monitored by TLC. The subsequent work-up and purification procedures were identical to those used for **Intermediate 1b**. The final product was obtained as a brown solid **Intermediate 58b** with a yield of 66.0%. ESI-MS: m/z 460.38 [M+1]⁺. C₂₆H₂₅N₃O₅ (459.50).

### (R)-7-hydroxy-12-(3-phenylpropanoyl)-3,4,12,12a-tetrahydro-1H-[1,4]oxazino[3,4-c]pyrido[2,1-f][1,2,4]triazine-6,8-dione (Compound 58)

**Intermediate 58b** was subjected to benzyl deprotection with LiCI in DMF solvent, and the target product **Compound 58** was obtained by HPLC separation. Yellow solid, m.p. 216-218°C, yield 56.4%.

¹H NMR (400 MHz, DMSO-*d*₆) δ 7.62 (d, *J* = 7.6 Hz, 1H, Ph-H), 7.33 - 7.16 (m, 5H, 5 × Ph-H, CH=CH), 6.16 (d, *J =* 7.7 Hz, 1H, CH=CH), 5.90 (dd, *J =* 10.2, 3.2 Hz, 1H, CH), 4.39 - 4.25 (m, 1H, CH), 4.00 (dd, *J* = 10.7, 3.3 Hz, 1H, CH), 3.79 - 3.69 (m, 2H, CH₂), 3.46 (d, *J* = 3.3 Hz, 2H), 3.06 - 2.96 (m, 1H), 2.87 (dq, *J* = 17.1, 8.9 Hz, 3H, 3× CH). ¹³C NMR (100 MHz, DMSO-*d*₆) δ 176.43, 172.09, 161.09, 152.29, 140.78, 140.54, 140.38, 128.87, 128.80, 128.75, 126.65, 117.68, 111.96, 67.55, 66.93, 65.73, 46.04, 33.91, 30.39. ESI-MS: m/z 370.43 [M+1]⁺. C₁₉H₁₉N₃O₅ (369.37).

### Compound 59

### (R)-12-([1,1'-biphenyl]-4-carbonyl)-7-(benzyloxy)-3,4,12,12a-tetrahydro-1H-[1,4]oxazino[3,4-c]pyrido[2,1-f][1,2,4]triazine-6,8-dione (Intermediate 59b)

**Intermediate 1a** (500 mg, 1.53 mmol, 1 eq.) was dissolved in 20 mL of dichloromethane. Triethylamine (232 mg, 2.3 mmol, 1.5 eq.) was added, and the mixture was stirred at room temperature for 10 minutes. 4-biphenylcarbonyl chloride (400 mg, 1.84 mmol, 1.2 eq.) was then added dropwise under an ice bath. The reaction was continued at room temperature for 3 hours, and the progress was monitored by TLC. The subsequent work-up and purification procedures were identical to those used for **Intermediate 1b.** The final product was obtained as a brown solid **Intermediate 59b** with a yield of 69.7%. ESI-MS: m/z 508.31 [M+1]⁺. C₃₀H₂₅N₃O₅(507.54)

### (R)-12-([1,1'-biphenyl]-4-carbonyl)-7-hydroxy-3,4,12,12a-tetrahydro-1H-[1,4]oxazino[3,4-c]pyrido[2,1-f][1,2,4]triazine-6,8-dione (Compound 59)

**Intermediate 59b** was subjected to benzyl deprotection with LiCI in DMF solvent, and the target product **Compound 59** was obtained by HPLC separation. Red brown solid, m.p.228-230 °C, yield 46.4%.

¹H NMR (400 MHz, DMSO-*d*₆) δ 7.95 - 7.88 (m, 4H, 4 × Ph-H), 7.80 (dt, *J* = 7.8, 2.2 Hz, 3H, 3 × Ph-H), 7.54 (dd, *J* = 8.4, 6.7 Hz, 2H, 2 × Ph-H), 7.50 - 7.44 (m, 1H, CH=CH), 6.24 (d, *J* = 7.7 Hz, 1H, CH=CH), 5.57 (dd, *J* = 10.3, 3.2 Hz, 1H, CH), 4.38 (dd, *J* = 11.0, 3.3 Hz, 1H, CH), 4.30 (dd, *J* = 13.5, 2.3 Hz, 1H, CH), 3.95 (t, *J* = 10.7 Hz, 1H, CH), 3.69 (dd, *J* = 11.6, 3.2 Hz, 1H, CH), 3.50 - 3.47 (m, 1H, CH), 3.27 - 3.24 (m, 1H, CH).¹³C NMR (100 MHz, DMSO-*d*₆) δ 173.41, 172.19, 160.78, 152.24, 145.34, 140.10, 139.06, 130.33, 129.67, 129.56, 129.09, 127.78, 127.51, 118.04, 112.88, 70.39, 67.29, 65.74, 45.87. ESI-MS: m/z 418.27 [M+1]⁺. C₂₃H₁₉N₃O₅(417.13)

### Compound 60

*methyl (R)-4-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)benzoate **(Intermediate 60b)***

**Intermediate 1a** (500 mg, 1.53 mmol, 1 eq) and methyl 4-(bromomethyl)benzoate (421 mg, 1.84 mmol, 1.2 eq) were combined with Cs₂CO₃ (1 g, 3.06 mmol, 2 eq) in 20 mL of DMF solvent and stirred at room temperature for 12 hours. The reaction was monitored and the work-up procedure was identical to that of **Intermediate 2b**, yielding a white **Intermediate 60b** with a yield of 90.2%. ESI-MS: m/z 476.52 [M+1]⁺. C₂₆H₂₅N₃O₅(475.50).

*methyl (R)-4-((7-hydroxy-6,8-dioxo-1,3,4,6,8,12a-hexahydro-12H-[1,4]oxazino[3,4-c]pyrido[2,1-f][1,2,4]triazin-12-yl)methyl)benzoate (***Compound 60***)*

**Intermediate 60b** was subjected to benzyl deprotection with LiCI in DMF solvent, and the target product **Compound 60** was obtained by HPLC separation. Yellow solid, m.p. 158-160°C, yield 42.5%.

¹H NMR (400 MHz, DMSO-*d*₆) δ7.91 (d, *J* = 7.8 Hz, 2H, 2× Ph-H), 7.44 (d, *J* = 7.9 Hz, 2H, 2× Ph-H), 7.05 (d, *J* = 7.6 Hz, 1H,CH=CH), 5.75 (d, *J* = 7.6 Hz, 1H, CH=CH), 5.07 (s, 1H, CH), 4.47 - 4.23 (m, 3H, 3× CH), 3.95 (d, *J* = 10.6 Hz, 2H, 2× CH), 3.86 (s, 3H, CH₃), 3.67 (s, 1H, CH), 3.51 (s, 1H, CH), 3.23 (s, 1H, CH).¹³C NMR (100 MHz, DMSO-d₆) δ 171.49, 166.42, 153.37, 140.74, 138.32, 130.67, 129.90, 129.79, 116.23, 111.71, 65.93, 52.69. ESI-MS: m/z 386.44 [M+1]⁺. C₁₉H₁₉N₃O₆(385.12)

### Compound 61

### (R)-4-((7-(benzyloxy)-6,8-dioxo-1,3,4,6,8,12a-hexahydro-12H-[1,4]oxazino[3,4-c]pyrido[2,1-f][1,2,4]triazin-12-yl)methyl)-N-phenylbenzamide (Intermediate 61b).

**Intermediate 60b** was dissolved in a 10% aqueous sodium hydroxide solution and methanol, and then refluxed at 65°C. The ester was hydrolysed to obtain product 60c. Subsequently, 60c was reacted with aniline in the presence of EDCI and HOBT in DMF solvent at room temperature to perform amide condensation, yielding a yellow **Intermediate 61b** with a yield of 65.2%. ESI-MS: m/z 537.45 [M+1]+. C₃₁H₂₈N₄O₅(536.58).

### (R)-4-((7-hydroxy-6,8-dioxo-1,3,4,6,8,12a-hexahydro-12H-[1,4]oxazino[3,4-c]pyrido[2,1-f][1,2,4]triazin-12-yl)methyl)-N-phenylbenzamide (Compound 61)

**Intermediate 61b** was subjected to benzyl deprotection with LiCl in DMF solvent, and the target product **Compound 61** was obtained by HPLC separation. Yellow solid, m.p. 286-288°C, yield 46.9%.

¹H NMR (400 MHz, DMSO-d₆) δ 10.28 (s, 1H, CONH), 8.08 - 7.66 (m, 4H, 4× Ph-H), 7.59 - 7.26 (m, 4H, 4× Ph-H), 7.22 - 6.93 (m, 2H, Ph-H, CH=CH), 5.81 (d, *J* = 7.7 Hz, 1H, CH=CH), 5.22 - 4.93 (m, 1H, CH), 4.66 - 4.12 (m, 3H, 3× CH), 4.06 - 3.77 (m, 2H, 2× CH), 3.76 - 3.34 (m, 3H, 3× CH).¹³C NMR (100 MHz, DMSO-d₆) δ 171.52, 165.42, 153.41, 139.53, 138.87, 138.37, 135.07, 130.28, 129.07, 128.36, 124.21, 120.94, 116.21, 111.80, 65.96, 55.39. ESI-MS: m/z 447.25 [M+1]⁺. C₁₉H₁₉N₃O₆(44616)

### Compound 62

### (R)-7-(benzyloxy)-12-(4-((4-fluorophenyl)thio)benzyl)-3,4,12,12a-tetrahydro-1H-[1,4][oxazino[3,4-c]pyrido[2,1-f][1,2,4]triazine-6,8-dione (Intermediate 62c)

**Intermediate 62c** was synthesized following the procedure used for the preparation of **Intermediate 50c,** with 4-fluorobenzenethiol substituted for benzenethiol. ESI-MS: m/z 544.61 [M+1]⁺. C₃₀H₂₆FN₃O₄S (543.16).

### (R)-12-(4-((4-fluorophenyl)thio)benzyl)-7-hydroxy-3,4,12,12a-tefrahydro-1H-[1,4]oxazino[3,4-c]pyrido[2,1-f][1,2,4]triazine-6,8-dione (Compound 62)

**Intermediate 62c** was subjected to benzyl deprotection with LiCl in DMF solvent, and the target product **Compound 62** was obtained by HPLC separation. Yellow solid, m.p. 262-263°C, yield 47.3%. ESI-MS: m/z 454.49 [M+1]⁺. C₂₃H₂₀FN₃O₄S (453.12).

### Compound 63

### (R)-7-(benzyloxy)-12-(4-((4-methoxyphenyl)thio)benzyl)-3,4,12,12a-tetrahydro-1H-[1,4]oxazino[3,4-c]pyrido[2,1-f][1,2,4]triazine-6,8-dione (Intermediate 63c)

**Intermediate 63c** was synthesized following the procedure used for the preparation of **Intermediate 50c,** with 4-methoxybenzenethiol substituted for benzenethiol. ESI-MS: m/z 556.30 [M+1]⁺. C₃₁H₂₉N₃O₅S (555.18).

### (R)-7-hydroxy-12-(4-((4-methoxyphenyl)thio)benzyl)-3,4,12,12a-tefrahydro-1H-[1,4]oxazino[3,4-c]pyrido[2,1-f][1,2,4]triazine-6,8-dione (Compound 63)

**Intermediate 63c** was subjected to benzyl deprotection with LiCl in DMF solvent, and the target product **Compound 63** was obtained by HPLC separation. Yellow solid, m.p. 220-221°C, yield 47.3%. ESI-MS: m/z 466.52 [M+1]⁺. C₂₄H₂₃N₃O₅S (465.14).

### Compound 64

### (R)-7-(benzyloxy)-12-(4-((4-nitrophenyl)thio)benzyl)-3,4,12,12a-tefrahydro-1H-[1,4]oxazino[3,4-c]pyrido[2,1-f][1,2,4]triazine-6,8-dione (Intermediate 64c)

**Intermediate 5b** (500 mg, 1 mmol, 1 eq), 4-nitrobenzenethiol (474 mg, 2 mmol, 2 eq), Cul (29 mg, 0.1 mmol, 0.1 eq), 1,10-phenanthroline (55 mg, 0.2 mmol, 0.2 eq), and K₂CO₃ (422 mg, 2 mmol, 2 eq) were charged into a 50 mL pear-shaped flask. 20 mL of 1,4-dioxane as the solvent were added. The Ullmann reaction was performed under a nitrogen atmosphere by refluxing at 100°C overnight. The subsequent procedure was identical to that of **Intermediate 50c,** yielding a light yellow solid **Intermediate 64c** with a yield of 24.5%. ESI-MS: m/z 571.16 [M+1]⁺. C₃₀H₂₆N₄O₆S (570.62).

### (R)-7-hydroxy-12-(4-((4-nifrophenyl)thio)benzyl)-3,4,12,12a-tetrahydro-1H-[1,4][oxazino[3,4-c]pyrido[2,1-f][1,2,4]triazine-6,8-dione (Compound 64)

**Intermediate 64c** was subjected to benzyl deprotection with LiCl in DMF solvent, and the target product **Compound 64** was obtained by HPLC separation. Light yellow solid, yield 32.5%.

¹H NMR (400 MHz, DMSO-*d*₆) δ 8.22 (d, J = 7.5 Hz, 2H, 2 × Ph-H), 7.55 (d, J = 7.4 Hz, 2H, 2 × Ph-H), 7.29 - 7.25 (m, 4H, 4 × Ph-H), 6.60 (s, 1H, CH=CH), 6.13 (s, 1H, CH=CH), 4.70 (s, 1H), 4.45 (s, 3H, 3 × CH), 3.99 - 3.90 (m, 3H), 3.79 (s, 1H, CH), 3.67 (s, 1H, CH). ESI-MS: m/z 481.30 [M+1]+. C₂₃H₂₀N₄O₆S (480.50)

### Compound 65

### (R)-7-(benzyloxy)-12-(4-((4-(methylthio)phenyl)thio)benzyl)-3,4,12,12a-tetrahydro-1H-[1,4]oxazino[3,4-c]pyrido[2,1-f][1,2,4]triazine-6,8-dione (Intermediate 65c)

**Intermediate 65c** was synthesized following the procedure used for the preparation of **Intermediate 50c,** with 4-methoxybenzenethiol substituted for benzenethiol. ESI-MS: m/z 572.71 [M+1]⁺. C₃₁H₂₉N₃O₄S₂ (571.16).

### (R)-7-hydroxy-12-(4-((4-(methyithio)phenyl)thio)benzyl)-3,4,12,12a-tetrahydro-1H-[1,4]oxazino[3,4-c]pyrido[2,1-1][1,2,4]triazine-6,8-dione (Compound 65)

**Intermediate 65c** was subjected to benzyl deprotection with LiCl in DMF solvent, and the target product **Compound 65** was obtained by HPLC separation. Yellow solid, m.p. 244-245°C, yield 41.1%. ESI-MS: m/z 466.52 [M+1]⁺. C₂₄H₂₃N₃O₄S₂ (465.14).

### Compound 66

### (R)-7-(benzyloxy)-12-(4-(pyridin-4-yithio)benzyl)-3,4,12,12a-tetrahydro-1H-[1,4]oxazino[3,4-c]pyrido[2,1-f][1,2,4]triazine-6,8-dione (Intermediate 66c)

**Intermediate 66c** was synthesized following the procedure used for the preparation of **Intermediate 50c,** with pyridine-4-thiol substituted for benzenethiol. ESI-MS: m/z 527.61 [M+1]⁺. C₂₉H₂₆N₄O₄S (526.17).

### (R)-7-hydroxy-12-(4-(pyridin-4-ylthio)benzyl)-3,4,12,12a-tetrahydro-1H-[1,4]oxazino[3,4-c]pyrido[2,1-f][1,2,4]triazine-6,8-dione (Compound 66)

**Intermediate 66c** was subjected to benzyl deprotection with LiCl in DMF solvent, and the target product **Compound 66** was obtained by HPLC separation. Yellow solid, m.p. 283-284°C, yield 53.4%. ESI-MS: m/z 437.49 [M+1]⁺. C₂₂H₂₀N₄O₄S₂ (436.12).

### Compound 67

### (R)-7-(benzyloxy)-12-(4-((5-bromopyridin-2-yl)thio)benzyl)-3,4,12,12a-tetrahydro-1H-[1,4]oxazino[3,4-c]pyrido[2,1-f][1,2,4]triazine-6,8-dione (Intermediate 67c)

**Intermediate 67c** was synthesized following the procedure used for the preparation of **Intermediate 50c,** with 5-bromopyridine-2-thiol substituted for benzenethiol. ESI-MS: m/z 605.51 [M+1]⁺. C₂₉H₂₅BrN₄O₄S (604.08).

**Intermediate 67c** was subjected to benzyl deprotection with LiCl in DMF solvent, and the target product **Compound 67** was obtained by HPLC separation. Yellow solid, m.p. 279-280 °C, yield 29.7%. ESI-MS: m/z 515.38 [M+1]⁺. C₂₂H₂₀N₄O₄S₂ (514.03).

### Compound 68

### (R)-7-(benzyloxy)-12-(4-((5-chloropyridin-2-yl)thio)benzyl)-3,4,12,12a-tetrahydro-1H-[1.4]oxazino[3,4-c]pyrido[2,1-f][1,2,4]triazine-6,8-dione (Intermediate 68c)

5b (500 mg, 1 mmol, 1 eq), 5-chloropyridine-2-thiol (445 mg, 2 mmol, 2 eq), Cul (29 mg, 0.1 mmol, 0.1 eq), 1,10-phenanthroline (55 mg, 0.2 mmol, 0.2 eq), and K₂CO₃ (276 mg, 2 mmol, 2 eq) were charged into a 50 mL pear-shaped flask. 20 mL of 1,4-dioxane as the solvent were added. The Ullmann reaction was performed under a nitrogen atmosphere by refluxing at 100°C overnight. The subsequent procedure was identical to that of **Intermediate 50c,** yielding a light yellow solid **Intermediate 64c** with a yield of 21.5%. ESI-MS: m/z 562.31 [M+1]⁺. C₂₉H₂₅ClN₄O₄S (561.05).

### (R)-12-(4-((5-chloropyridin-2-yl)thio)benzyl)-7-hydroxy-3,4,12,12a-tetrahydro-1H-[1,4]oxazino[3,4-c]pyrido[2,1-f][1,2,4]triazine-6,8-dione (Compound 68)

**Intermediate 68c** was subjected to benzyl deprotection with LiCl in DMF solvent, and the target product **Compound 68** was obtained by HPLC separation. Light yellow solid, yield 33.1%.

¹H NMR (400 MHz, DMSO-d₆) δ 8.31 (d, J = 1.9 Hz, 1H, Pyridine-H), 7.61 (d, J = 8.0 Hz, 2H, 2 × Ph-H), 7.44 (dd, J = 7.8, 1.9 Hz, 1H, Pyridine-H),7.27 (d, J = 8.0 Hz, 2H, 2 × Ph-H), 7.11 (d, J = 7.9 Hz, 1H, Pyridine-H), 6.63 (s, 1H, CH=CH), 6.33 (s, 1H, CH=CH), 4.70 (s, 1H, CH), 4.42 (s, 3H, 3 × CH), 4.12 - 3.94 (m, 3H, 3 × CH), 3.79(s, 1H, CH), 3.21 (s, 1H, CH). ESI-MS: m/z 471.79[M+1]+. C₂₂H₁₉ClN₄O₄S (470.93)

### EXAMPLE 9: HSV-1 pUL15C nuclease activity inhibition

The nuclease inhibitory activity of the synthesized compounds was evaluated in enzymatic assays using DNA/DNA hybrids or a hairpin oligonucleotide (containing a double-stranded DNA structure) as substrates of the reaction. One of the substrates contained a 33-nucleotide DNA labelled at its 5' end with ³²P, annealed to a 33-nucleotide complementary DNA strand, and the second one was a 42-nucleotide DNA substrate containing a hairpin structure [Masaoka et al., 2016, Biochemistry, 55(5), 809-819*].* The assays were performed with HSV-1 pUL15C expressed in *E*. *coli* BL21(DE3) cells and purified [*S*elvarajan Sigamani et al., 2013, J Virol., 87(12):7140-8*].* Substrates used were either a double-stranded DNA (dsDNA) template containing a ³²P-labeled oligonucleotide (5'-GATGAGACCACCAATAAAAAGGCTCAATTAATG-3') annealed to a 33mer complementary DNA strand (5'-CATTAATTGAGCCTTTTTATTGGTGGTCTCATC-3'), or a ³²P-labeled DNA oligonucleotide of 42 bases with a hairpin structure (5'-TATGTATTTAGGATTGGGATTATACCCAATCCTAAATACATA-3').

. DNA cleavage kinetics were determined at 37°C in 20 mM Tris-HCl (pH 7.0), containing 1 mM MnCl₂, 10 mM NaCl and 5% DMSO. The concentration of labelled substrate was 100 nM, and assays were carried out with an enzyme concentration of 400-600 nM. Nuclease cleavage was triggered by adding the dsDNA to a solution containing the enzyme and the inhibitor (at the appropriate concentration). Reaction aliquots were withdrawn after 15 min and immediately terminated by adding an equal volume of loading buffer [10 mM EDTA in 90% formamide containing xylene cyanol FF (3 mg/mL) and bromophenol blue (3 mg/mL)]. Hydrolysis products were fractionated by 20% denaturing urea polyacrylamide electrophoresis [19:1 acrylamide:bisacrylamide] and quantitatively analysed by phosphorimaging using a BAS1500 scanner (Fuji) or an Amersham Typhoon biomolecular imager (Cytiva), and the Tina version 2.09 software (Raytest Isotopenmessgerate Gmbh, Staubenhardt, Germany).

IC₅₀ values were determined from dose-response curves after calculating the inhibitory activity (i.e. percentage inhibition), in the presence of the tested compounds at concentrations in the range 0.25 - 50 µM. Reference values for maximum activity were obtained in the absence of inhibitors, in a buffer containing 5% DMSO. Data analyses were performed using the GraphPad Prism version 6.00 software (GraphPad Software, La Jolla, CA, USA).

As reference compounds, in **Table 3** the IC₅₀ of HSV-1 pUL15C nuclease activity inhibition (obtained with the DNA/DNA hybrid) of comparative compounds Baloxavir acid and β-thujaplicinol are indicated:

**Table 3**

| **Comparative antiviral compound** | **Structure** | **HSV-1 pUL15C (IC₅₀, µM)** |
|---|---|---|
| Baloxavir acid | 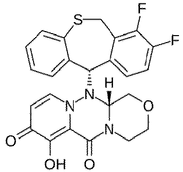 | **11.5 ± 0.4** |
| β-thujaplicinol | 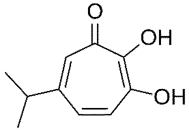 | **0.67** ± **0.1** |

The results obtained for the compounds according to the present invention, as defined above in **Table 1,** are shown in **Table 4** (dsDNA substrate) and **Table 5** (42-nt hairpin DNA substrate):

**Table 4**

| **Compound N°** | **HSV-1 pUL15C (IC₅₀, µM)** | **Compound N°** | **HSV-1 pUL15C (IC₅₀, µM)** |
|---|---|---|---|
| **1** | 3.28 ± 0.22 | **32** | 25.9 ± 8.50 |
| **2** | 2.51 ± 0.22 | **33** | 1.10 ± 0.10 |
| **3** | 2.20 ± 0.18 | **34** | 1.11 ± 0.11 |
| **4** | 8.79 ± 0.61 | **35** | 4.68 ± 0.37 |
| **5** | 4.87 ± 0.49 | **36** | 5.15 ± 0.62 |
| **6** | 6.69 ± 0.53 | **37** | 8.22 ± 0.73 |
| **7** | 4.49 ± 0.55 | **38** | 2.30 ± 0.37 |
| **8** | 5.67 ± 0.53 | **39** | 0.88 ± 0.11 |
| **9** | 2.07 ± 0.28 | **40** | 17.1 ± 0.9 |
| **10** | 2.47 ± 0.17 | **41** | 24.7 ± 3.1 |
| **11** | 2.56 ± 0.53 | **42** | 21.4 ± 1.4 |
| **12** | 28.8 ± 1.8 | **43** | 1.17 ± 0.12 |
| **13** | 2.92 ± 0.24 | **44** | 14.4 ± 1.8 |
| **14** | 5.07 ± 0.47 | **45** | 2.13 ± 0.52 |
| **15** | 2.66 ± 0.34 | **46** | 2.08 ± 0.33 |
| **16** | 1.74 ± 0.10 | **47** | 19.2 ± 1.5 |
| **17** | 6.35 ± 0.59 | **48** | 12.9 ± 1.6 |
| **18** | 10.6 ± 1.4 | **49** | 2.35 ± 0.25 |
| **19** | 1.56 ± 0.16 | **50** | 11.1 ± 1.4 |
| **20** | 1.69 ± 0.29 | **51** | 16.0 ± 0.6 |
| **21** | 6.47 ± 0.60 | **52** | 12.6 ± 1.8 |
| **22** | 12.9 ± 1.90 | **53** | 4.88 ± 0.27 |
| **23** | 6.05 ± 0.38 | **54** | 6.17 ± 0.34 |
| **24** | 15.1 ± 1.90 | **55** | 4.73 ± 0.29 |
| **25** | 1.76 ± 0.32 | **56** | 14.2 ± 2.6 |
| **26** | 2.80 ± 0.61 | **57** | 15.5 ± 2.7 |
| **27** | 1.70 ± 0.24 | **58** | 1.51 ± 0.20 |
| **28** | 1.20 ± 0.28 | **59** | 11.6 ± 1.7 |
| **29** | 5.40 ± 0.65 | **60** | 20.8 ± 1.8 |
| **30** | 3.31 ± 0.45 | **61** | 18.5 ± 3.6 |
| **31** | 19.4 ± 1.30 | | |

**Table 5**

| **Compound** | **HSV-1 pUL15C (IC₅₀, µM)** |
|---|---|
| **β-thujaplicinol** | 0.72 ± 0.07 |
| **19** | 2.42 ± 0.25 |
| **50** | 5.16 ± 0.28 |
| **62** | 28.0 ± 5.1 |
| **63** | 15.4 ± 1.4 |
| **64** | 3.54 ± 0.38 |
| **65** | 4.33 ± 0.62 |
| **66** | 14.7 ± 1.2 |
| **67** | 3.19 ± 0.34 |
| **68** | 1.00 ± 0.15 |

### EXAMPLE 10: Antiviral assays in Vero cells

The cellular antiviral activities of compounds according to the invention, as well as for the know antiviral compounds acyclovir (ACV), β-thujaplicinol, Baloxavir acid and Baloxavir marboxil, for comparison, were determined in Vero cells, a cell line derived from the kidney tissue of an adult African green monkey (ATCC CCL-81). The virus used was a wild type HSV-1 (F strain) (GenBank accession number GU734771) construct obtained by fusing GFP to the HSV-1 capsid protein VP26 *[*Desai and Person, 1998, J Virol., 72(9):7563-8]. Cells were cultured in low-glucose Dulbecco's modified Eagle medium (DMEM) supplemented with 5% fetal bovine serum (FBS), penicillin (50 U/mL) and streptomycin (50 µg/mL) at 37°C in a humidified atmosphere of 5% CO₂.

3.5 x 10⁴ cells/well were seeded in 48-well plates and cultured in DMEM media containing 5% FBS to form a monolayer of cells. Compounds were diluted in DMEM with 5% FBS and added to cell monolayers in triplicate for 1 h at different concentrations (2, 5, 10, 20 µM), at 37°C. Two controls were added in this experiment:
(i) mock cells, which were subjected to the same conditions as the rest of the samples, but were not infected, and
(ii) non-treated cells, which were infected with the virus but not treated with any compound.

HSV-1 K26-GFP was diluted in DMEM medium without FBS at a multiplicity of infection (m.o.i.) of 0.1. Cells were then incubated at 37°C for 1 h with the mix virus-compound. After 1 h of viral adsorption, the virus-containing inoculum was removed, wells were washed once in PBS and maintained in DMEM with 5% FBS containing the compounds (at appropriate concentrations). Cells were incubated at 37°C for an additional 24 h, and plates were then inspected by phase-contrast for toxicity. Cells in wells showing no obvious toxicity were processed for flow cytometry. Cells were dissociated by incubation for 3 min with 0.05% trypsin/0.1% EDTA at room temperature, washed and fixed in PBS with 1% bovine serum albumin, 1% FBS, 2% paraformaldehyde) for 15 min. Then, cells were rinsed, resuspended in PBS and analysed using a FACSCalibur Flow Cytometer (BD Biosciences). Data were processed with FlowJo software (BD, version 10.6.2). The 50% effective concentration (EC₅₀), which indicates the concentration of the compound that leads to a 50% reduction in viral infection was determined from dose-response curves as determined above.

The results obtained for a series of compounds according to the invention, and comparative antiviral compounds acyclovir (ACV), β-thujaplicinol, Baloxavir acid and Baloxavir marboxil are shown in following **Table 6.** Numbers between parentheses indicate percentage inhibition at the highest concentration tested (20 µM).

**Table 6**

| **Compound** | **EC₅₀ (µM)** |
|---|---|
| *Compound 7* | 3.4 ± 2.7 |
| *Compound 10* | 10.3 ± 0.9 |
| *Compound 18* | >20 (48.5 ± 2.0) |
| *Compound 19* | 2.8 ± 1.1 |
| *Compound 29* | 4.0 ± 1.3 |
| *Compound 32* | 8.9 ± 1.0 |
| *Compound 64* | 1.9 ± 0.4 |
| *Acyclovir (ACV)* | 0.68 ± 0.11 |
| *β-thujaplicinol* | 3.9 ± 0.9 |
| *Baloxavir acid* | >20 (40.7 ± 1.7) |
| *Baloxavir marboxil* | >20 (13.7 ± 4.4) |

### EXAMPLE 11: Antiviral activity of Compound 19 against HSV-2

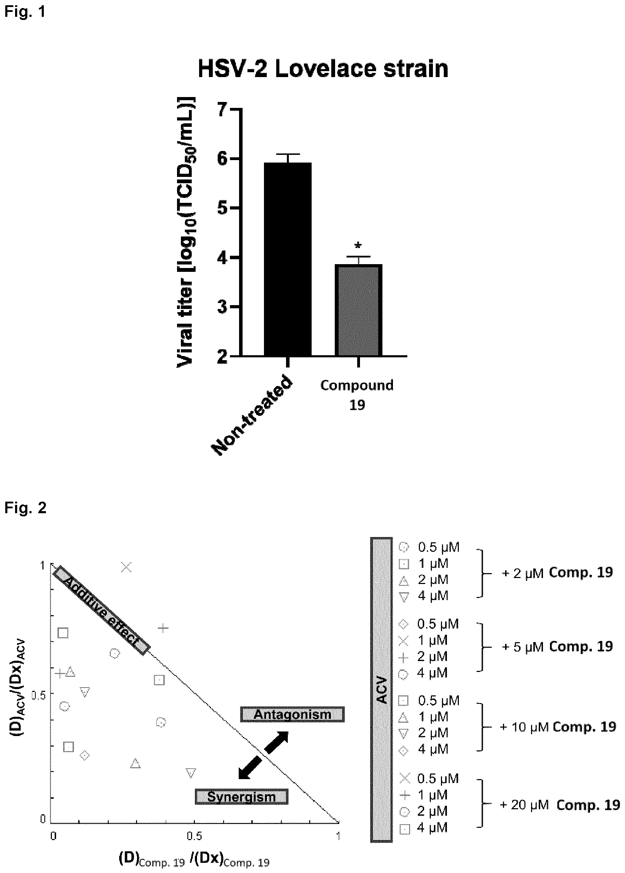

The antiviral activity of **Compound 19** was also tested against HSV-2 (Lovelace strain) in Vero cells. Cells were treated with **Compound 19** (at 10 µM concentration) for one hour before infection with the progeny virus at an m.o.i. of 0.1. Cells were incubated with the virus at 37°C for 1 h, and after removing the virus-containing inoculum, cultures were maintained at 37°C for an additional 24 h in the presence of 10 µM of **Compound 19.** After the incubation, cells were treated as above and tissue culture infective doses per mL (TCID₅₀/mL) were determined using an end-point dilution assay [Reed and Muench, 1938, Am. J. Epidemiol., 27(3), 493-497]. Non-treated Vero cells were used as a control for comparison. The results are shown in **Fig. 1****.**

### EXAMPLE 12: Combination experiments of acyclovir with Compound 19

The antiviral effect produced by the combination of acyclovir with **Compound 19** was studied in Vero cells with HSV-1 K26-GFP. Vero cells were infected with HSV-1 K26-GFP (m.o.i. = 0.1) in the presence of **Compound 19** at concentrations of 2, 5, 10 and 20 µM, each one in combination with four doses of acyclovir (0.5, 1, 2 and 4 µM). The antiviral effects were determined 24 h after the infection. Results were analysed using the method of Chou and Talalay [Chou, 2010, Cancer Res. 70(2), 440-446] to determine if the combination produced synergistic, additive, or antagonistic effects. The resulting data were analysed using the CompuSyn software (ComboSyn, Paramus, NJ). The assignment of synergy or antagonism was based on the theoretical values obtained for the combination index (Cl)-isobologram equation that facilitates a quantitative determination of drug interactions. The estimated CI value indicates whether the combined effect is synergistic (CI <1), additive (CI =1) or antagonistic (CI >1). More specifically, robust and moderate synergism are characterized by CI values in the range of 0.3 - 0.7 and 0.7 - 0.95, respectively. Fa values (0 to 1) correspond to 0 to 100% of viral infection inhibition. Results are shown below in **Table 7** and in **Fig. 2****;** as may be observed, most obtained CI values are in the synergistic range.

**Table 7**

| **Dose (µM) Compound 19** | **Dose (µM) Acyclovir** | **Effect (Fa)** | **CI value** | **Interaction** |
|---|---|---|---|---|
| 2 | 0.5 | 0.561 | 0.87875 | Moderate synergism |
| | 1 | 0.601 | 0.93184 | Moderate synergism |
| | 2 | 0.781 | 0.53090 | Robust synergism |
| | 4 | 0.8115 | 0.67871 | Robust synergism |
| 5 | 0.5 | 0.58 | 1.71754 | Antagonism |
| | 1 | 0.678 | 1.25220 | Antagonism |
| | 2 | 0.7645 | 1.14393 | Antagonism |
| | 4 | 0.84198 | 0.77526 | Moderate synergism |
| 10 | 0.5 | 0.85 | 0.77783 | Moderate synergism |
| | 1 | 0.8767 | 0.65412 | Robust synergism |
| | 2 | 0.8916 | 0.62477 | Robust synergism |
| | 4 | 0.9402 | 0.38283 | Robust synergism |
| 20 | 0.5 | 0.90177 | 0.93006 | Moderate synergism |
| | 1 | 1.9346 | 0.61266 | Robust synergism |
| | 2 | 0.94848 | 0.4999 | Robust synergism |
| | 4 | 0.96565 | 0.35772 | Robust synergism |

### EXAMPLE 13: Pharmacokinetics studies in rats.

Six male Sprague-Dawley rats (180-200 g) were randomly divided into two groups to receive oral administrations of **Compound 19** at 2 mg·Kg⁻¹ (intravenous administration, *iv)* or 10 mg·Kg⁻¹ (oral administration, *po).* **Compound 19** was dissolved in DMSO, followed by the addition of solutol HS-15 and water to prepare the formulation at a final concentration of 1 mg/mL containing 3% DMSO, 10% solutol HS-15, and 87% double-distilled water for dosing. Rats were purchased from Beijing Vital River Laboratory Animal Technology Co., Ltd. (Qualification No. SCXK(SH) 2021-0011). Animals were subjected to twelve-hour alternating light and dark cycles, *ad libitum* access to drinking water and food, and fasting for 12 hours prior to the initiation of treatments. For the oral dose at 10 mg·Kg⁻¹, blood samples were collected at 0.083, 0.25, 0.5, 1, 2, 4, 6, 8 and 24 h post-administration. For the intravenous dose (at 2 mg·Kg⁻¹), blood samples were collected at 0.083, 0.25, 0.5, 1, 2, 4, 6, 8 and 24 h post-administration. Twenty µL of blood were mixed with a 200-µL solution of MeOH:acetonitrile (1:1), vortexed for 5 min, and centrifuged (4000 rpm, 4°C, 10 min) to obtain plasma within 30 minutes of sampling. The supernatant was analysed by LC-MS/MS.

Chromatographic separation was performed using an ACQUITY UPLC HSS T3 column (2.1 × 50 mm, 1.8 µm). The mobile phase was: (A) Water with 0.025% formic acid; (B) acetonitrile. Gradient elution was carried out at a flow rate of 0.8 mL/min under the following conditions: the initial proportion of mobile phase B was 20%, increased to 95% B over 1.6 min, and maintained at 95% for 0.3 min, before being reduced back to 20% within 0.01 min and maintained at 20% for 0.29 min. Mass spectrometric detection was performed with an electrospray ionization (ESI) source operating in positive ion mode. Quantification was achieved using multiple reaction monitoring (MRM) with the following transitions: m/z 403.15.

Pharmacokinetic parameters such as AUC, *T*_{1/2} and Cₘₐₓ were calculated using non-atrial PK analysis with WinNonlin. The absolute oral bioavailability in rats was calculated based on the ratio of AUC after oral and intravenous administration, using the following equation: $F \left(%\right) = \frac{AUC \left(p.o.\right) \times Dose \left(i.v.\right)}{AUC \left(i.v.\right) \times Dose \left(p.o.\right)} \times 100 %$ where F represents the absolute bioavailability; AUC represents the area under the concentration-time curve; and Dose refers to the administered dose. The phamacokinetics analysis showed that the *T*ₘₐₓ of Compound 19 was 2 h *(po),* indicating a mild onset of action. In addition, it showed a low intrinsic clearance (CL = 1647 ± 230 mL/h/kg, *iv),* a favourable half-life (*T*_{1/2} = 5.54 ± 0.676 h *po* and 6.24 ± 1.24 h *iv)* and an optimal volume of distribution (AUC = 2980 ± 763 h*ng/mL *po* and 1231 ± 182 h*ng/mL *iv).* Furthermore, **Compound 19** exhibited good oral bioavailability (*F* = 48.4 ± 0.124%).

### EXAMPLE 14: Toxicity assays in mice.

BALB/c mice were purchased from Charles River Laboratories Spain and maintained at the Animal Facility of the *Centro de Biologia Molecular Severo Ochoa (CBM, Madrid, Spain).* The toxicity of the test compound **Compound 19** was investigated in BALB/c mice dosed as a solution at 25, 50 and 75 mg·Kg⁻¹. The compound was formulated in 3% DMSO + 10% solutol HS-15 + 87% double-distilled water (20 mg/mL). Animals had free access to food and water through each study. Thirty BALB/c mice were distributed in five different groups (n = 6), three of them receiving the specific doses of **Compound 19,** one receiving acyclovir (50 mg·Kg⁻¹), and the fifth one inoculated with phosphate buffered saline (PBS). Compounds were orally administered for five consecutive days. On day 12 mice were sacrificed and whole blood was obtained by cardiac puncture. Several parameters were analysed to monitor renal, hepatic, and immunological basic profiles. Body weight was also monitored to exclude weight loss or lack of weight gain that would be indicative of toxicity.

The mice did not show any toxic symptoms or mortality immediately or during the post-treatment period of 12 days. No abnormal behaviours or significant changes in the body weight were observed during the execution of the experiment. White blood cell counts and hematocrit values were similar in all groups of mice, and liver function and renal markers were not affected by the administration of different compounds.

### EXAMPLE 15: HSV-1 infection model in BALB/c mice.

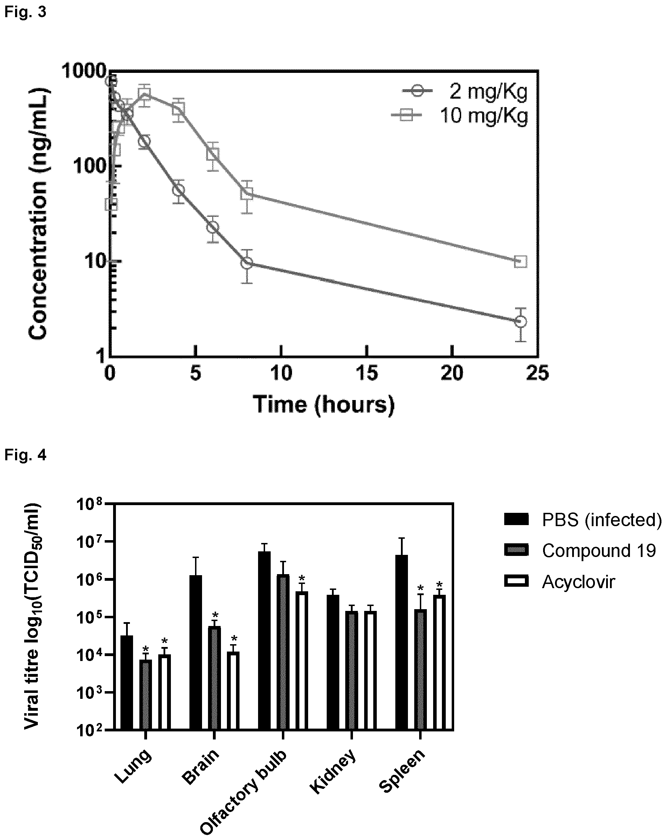

BALB/c mice were used to assess the activity of **Compound 19,** after viral titration in specific organs. Mice were maintained in the Biosafety level 2 Animal Facility of the CBM and separated into four groups of six individuals each, and after acclimatization, all of them were infected intranasally with HSV-1 (10⁵ PFU, 30 µL/mice). Compounds were orally administered in PBS, **Compound 19** (75 mg·Kg⁻¹) or acyclovir (50 mg·Kg⁻¹) at days -1, 0, 1, 2 and 3 post-infection (d.p.i.). At day 8 post-infection, animals were euthanized and organs (brain, lungs, olfactory bulbs, kidney and spleen) were extracted for virus quantitation. Organs were homogenized using TissueLyzer, subjected to three rounds of freezing/thawing, and stored at -80°C before titration by end-point dilution and determination of the tissue culture infective dose per mL (TCID₅₀/mL). Mean (n = 6) ± S.E.M. are shown, and statistical comparisons were performed using the Gehan-Breslow-Wilcoxon test; *p < 0.05. The results are shown in **Fig. 4****.**

Although the efficacy of **Compound 19** was slightly lower than in the case of acyclovir, its good pharmacokinetic and toxicity profiles make it an interesting alternative or supplement. Furthermore, drug combination experiments suggest a benefit of combining acyclovir with **Compound 19.**

At day 8 p.i., mice were euthanized and relevant organs and tissues were extracted. Progeny virus was titrated in Vero cells to determine the 50% tissue culture infective dose (TCID₅₀)/mL. Mean (n = 6) ± S.E.M. are shown, and statistical comparisons were performed using the Gehan-Breslow-Wilcoxon test; *p < 0.05.

## Claims

1. Compound having the formula (I): 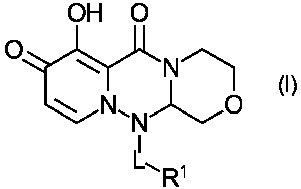 wherein
L is a linker, which is selected from -CH₂- and -(C=O)-;
R¹ is selected from hydrogen, linear or branched C₁-C₆ alkyl, naphthyl, or 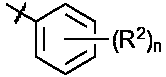 wherein
n is an integer selected from 0, 1 or 2,
if n is 2, the R² groups may be the same or different one from another,
R² is selected from halogen, C₁-C₃ haloalkyl, C₂-C₁₀ heteroaryl, benzodioxol or 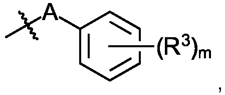 wherein
A is a bond or is selected from -CH₂-, -(C=O) -, O or S,
m is an integer selected from 0, 1 or 2,
if m is 2, the R³ groups may be the same or different one from another, R³ is selected from halogen, -CN, -NH₂, -CHO, -COOH, -SO₂CH₃, -SO₂NH₂, -CONHR⁴ or -OR⁵,
wherein
R⁴ is hydrogen, C₁-C₆ alkyl or C₅-C₆ cycloalkyl,
R⁵ is hydrogen, C₁-C₆ alkyl, C₁-C₃ haloalkyl,
and stereoisomers thereof and pharmaceutically acceptable salts thereof.

2. Compound according to claim 1, wherein linker L is -CH₂-.

3. Compound according to any one of claims 1 or 2, wherein R¹ is selected from hydrogen, methyl, naphthyl, or

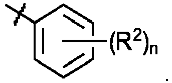

4. Compound according to any one of claims 1 to 3, wherein R' is 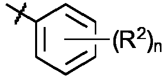 and R² is selected from fluorine, iodine, chlorine, trifluoromethyl, pyridine, pyrimidine, pyrazole, furane, indole, benzodioxol, or

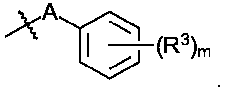

5. Compound according to any one of claims 1 to 4, wherein m and n are independently 1 or 2, preferably n and m are both 1.

6. Compound according to any one of claims 1 to 5, wherein R² is

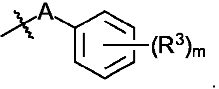

7. Compound according to any one of claims 1 to 6, wherein
R¹ is 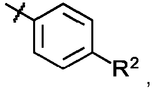
R² is 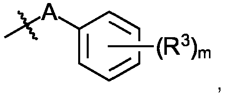 and
R³ is selected from chlorine, fluorine, -CN, -NH₂, -CHO, -COOH, -SO₂CH₃, -SO₂NH₂, -CONH₂ -CONHCH₃, -CONH(cyclopentyl), -OH or -OCH₃.

8. Compound according to any one of claims 1 to 7, wherein the compound is of formula (II) or (III): 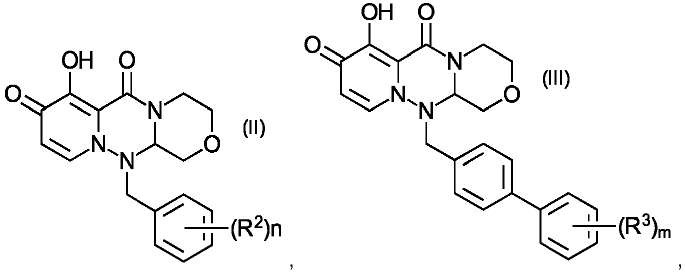 wherein R² and R³ are as defined above.

9. Compound of formula (I) according to any one of claims 1 to 8, wherein the compound is selected from:
| **Compound N°** | **Structure** | **Compound N°** | **Structure** |
|---|---|---|---|
| **1** | 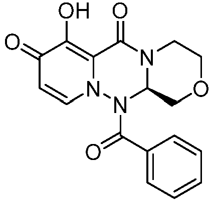 | **2** | 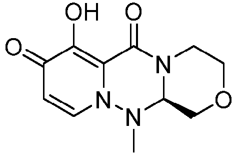 |
| **3** | 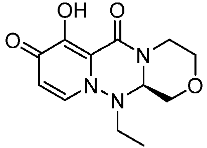 | **4** | 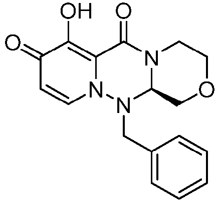 |
| **5** | 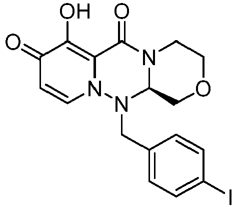 | **6** | 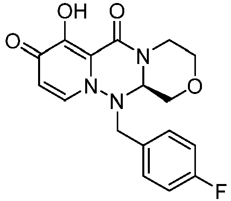 |
| **7** | 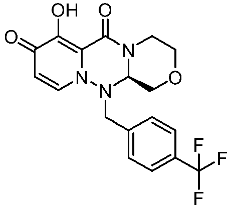 | **8** | 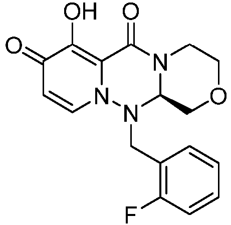 |
| **9** | 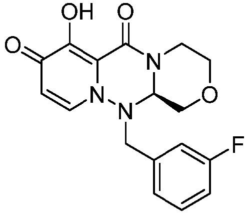 | **10** | 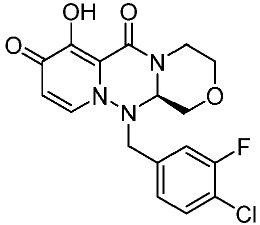 |
| **11** | 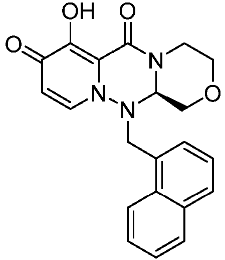 | **12** | 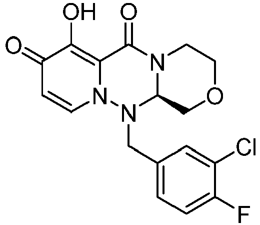 |
| **13** | 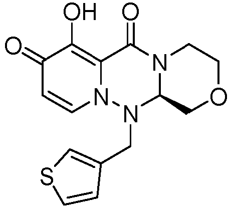 | **14** | 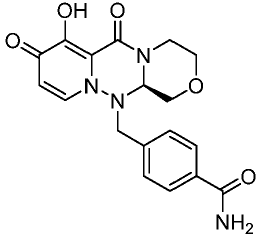 |
| **15** | 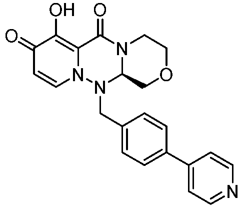 | **16** | 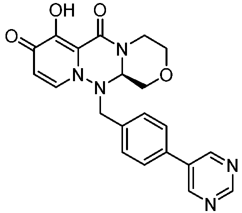 |
| **17** | 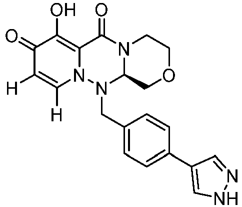 | **18** | 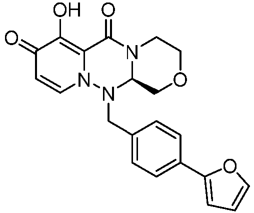 |
| **19** | 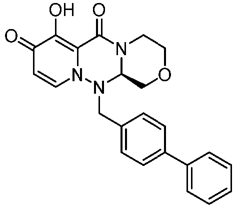 | **20** | 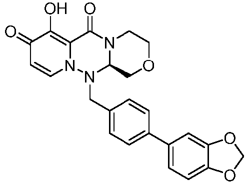 |
| **21** | 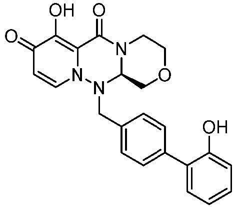 | **22** | 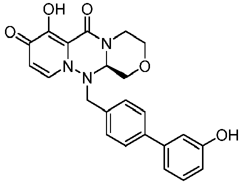 |
| **23** | 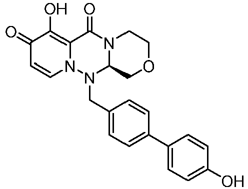 | **24** | 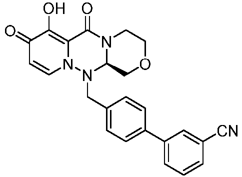 |
| **25** | 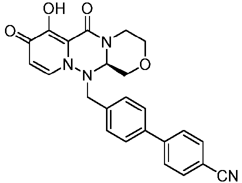 | **26** | 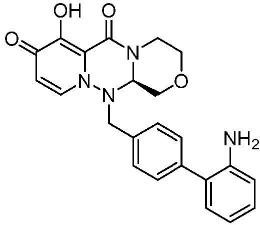 |
| **27** | 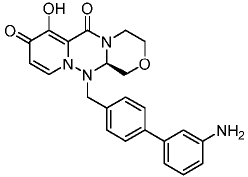 | **28** | 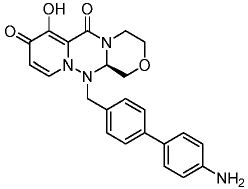 |
| **29** | 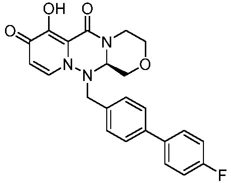 | **30** | 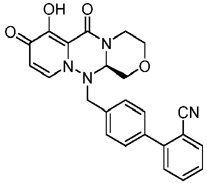 |
| **31** | 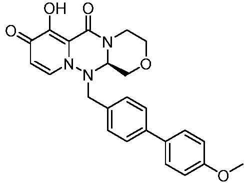 | **32** | 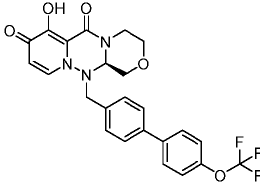 |
| **33** | 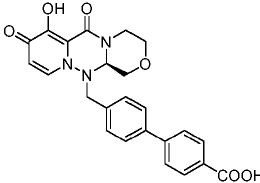 | **34** | 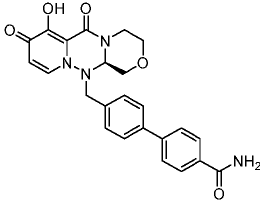 |
| **35** | 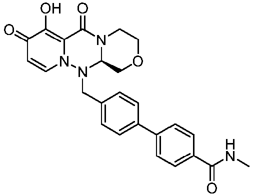 | **36** | 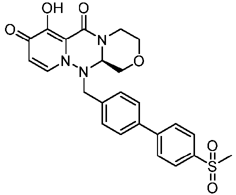 |
| **37** | 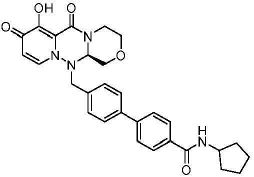 | **38** | 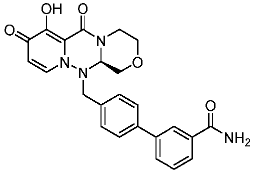 |
| **39** | 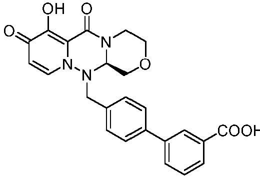 | **40** | 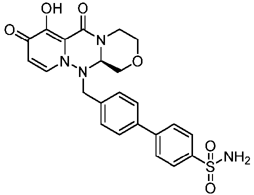 |
| **41** | 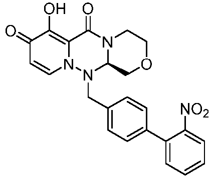 | **42** | 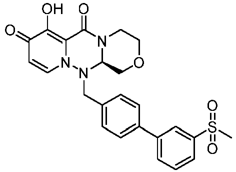 |
| **43** | 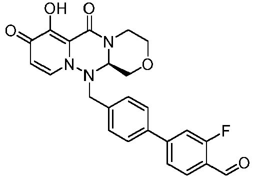 | **44** | 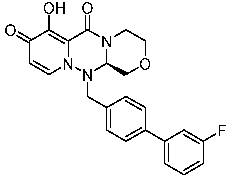 |
| **45** | 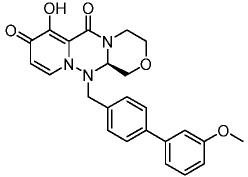 | **46** | 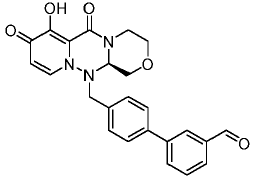 |
| **47** | 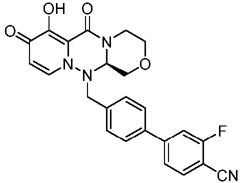 | **48** | 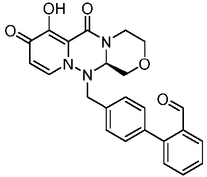 |
| **49** | 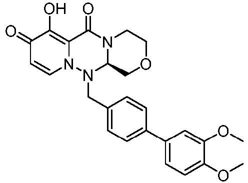 | **50** | 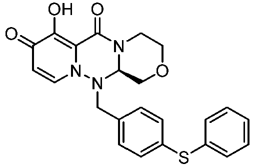 |
| **51** | 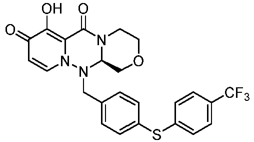 | **52** | 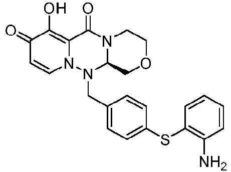 |
| **53** | 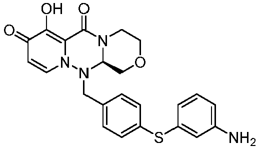 | **54** | 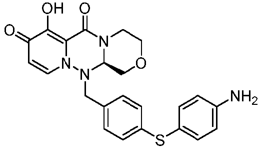 |
| **55** | 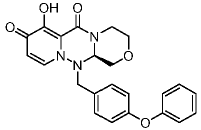 | **56** | 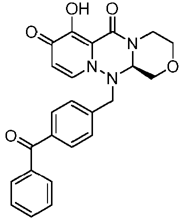 |
| **57** | 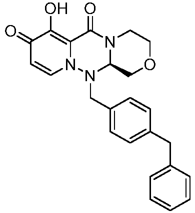 | **58** | 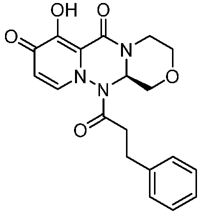 |
| **59** | 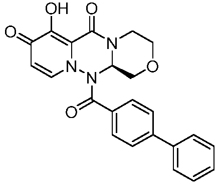 | **60** | 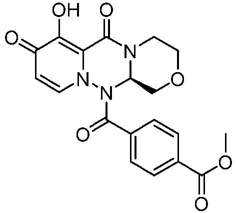 |
| **61** | 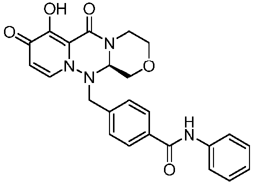 | **62** | 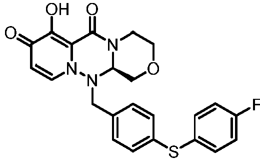 |
| **63** | 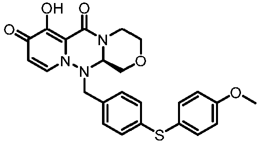 | **64** | 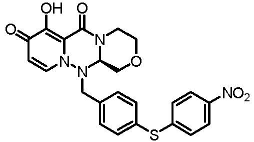 |
| **65** | 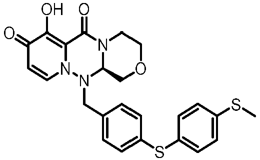 | **66** | 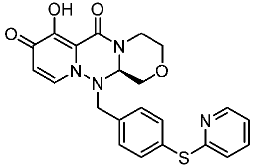 |
| **67** | 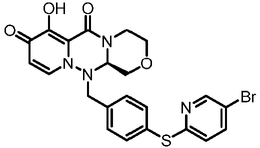 | **68** | 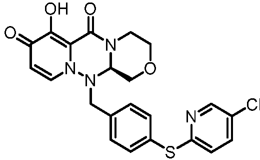 |

10. A compound of formula (I) as defined in any one of claims 1 to 9, which is:

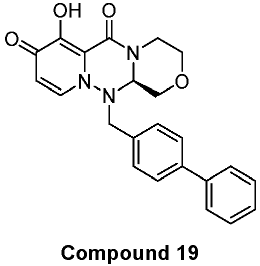

11. A compound of formula (I) as defined in any one of claims 1 to 10, for use as a medicament.

12. A compound of formula (I) as defined in any one of claims 1 to 10, for use as a medicament for the treatment or prophylaxis of a viral infection.

13. A compound for use according to claim 12, wherein the viral infection is caused by Herpes Simplex Virus.

14. A pharmaceutical composition comprising a compound of formula (I) as defined in any one of claims 1 to 10, and at least one pharmaceutically acceptable excipient.

15. A pharmaceutical composition according to claim 14, further comprising at least one further pharmaceutically active compound, particularly a compound for the prophylaxis or treatment of a viral infection, more particularly a viral infection caused by Herpes Simplex Virus, even further particularly acyclovir.
